# FASCICULE DE BREVET EUROPEEN

(11) **EP 3 322 814 B1**
(45) Date de publication et mention de la délivrance du brevet: **12.06.2024**
(21) Numéro de dépôt: 16750966.0
(22) Date de dépôt: 15.07.2016
(51) Int. Cl.: C12Q 1/18

(54) **PROCEDE DE DETERMINATION DU DEGRE DE SENSIBILITE D'UNE SOUCHE DE CHAMPIGNON VIS-A-VIS D'UN ANTIFONGIQUE**
VERFAHREN ZUR BESTIMMUNG DES EMPFINDLICHKEITSGRADES EINES PILZSTAMMES GEGEN EIN ANTIMYKOTIKUM
METHOD FOR DETERMINING THE DEGREE OF SENSITIVITY OF A STRAIN OF FUNGUS TO AN ANTIFUNGAL AGENT

(30) Priorité: 15.07.2015 FR 1501489
(43) Date de publication de la demande: 23.05.2018
(73) Titulaire: Université Grenoble Alpes, 38400 Saint Martin d'Hères (FR); Centre Hospitalier Universitaire de Grenoble, 38043 Grenoble Cedex 9 (FR)
(72) Inventeur: MORIN-ALDEBERT, Delphine, Paule, Renée, 38100 Grenoble (FR); CORNET-GIGOU, Murielle, Monique, Jacqueline, 38330 Saint-ismier (FR)
(74) Mandataire: Germain Maureau
(86) Numéro de dépôt international: PCT/FR2016/051832
(87) Numéro de publication internationale: WO 2017/009585

(56) Documents cités:
- WO-A1-2013/130875
- WO-A2-95/06132
- FR-A1- 2 863 273
- RONCERO C ET AL: "ISOLATION AND CHARACTERIZATION OF SACCHAROMYCES CEREVISIAE MUTANTS RESISTANT TO CALCOFLUOR WHITE", JOURNAL OF APPLIED BACTERIOLOGY, BLACKWELL PUBLISHING LTD., OXFORD, GB, vol. 170, no. 4, 1 avril 1988 (1988-04-01) , pages 1950-1954, XP008011949, ISSN: 0021-8847
- JUCHIMIUK MATEUSZ ET AL: "Candida albicans cis-prenyltransferase Rer2 is required for protein glycosylation, cell wall integrity and hypha formation", FUNGAL GENETICS AND BIOLOGY, SAN DIEGO, CA, US, vol. 69, 27 mai 2014 (2014-05-27), pages 1-12, XP029010265, ISSN: 1087-1845, DOI: 10.1016/J.FGB.2014.05.004

## Description

L'invention concerne un procédé de détermination du degré de sensibilité d'une souche de champignon vis-à-vis d'un antifongique tel que défini dans les revendications attachées.

Depuis les années 1980, les champignons sont considérés comme une source majeure de pathologies chez l'homme.

La candidose invasive, causée par différentes espèces de levures, représente la quatrième cause d'infections nosocomiales du sang aux Etats-Unis, et est associée à une mortalité d'environ 40%. Ces septicémies sont principalement liées à des immunodépressions, des actes chirurgicaux ou invasifs.

L'augmentation de l'apparition de souches résistances aux molécules antifongiques notamment chez les levures *Candida* spp., entraine des échecs cliniques croissants. La caractérisation précise et rapide des isolats résistants est devenue l'une des grandes préoccupations afin d'adapter le traitement en fonction du phénotype de la souche le plus rapidement possible après le début de l'infection.

Les tests de sensibilité aux antifongiques qui sont actuellement disponibles sont basés sur l'évaluation de l'inhibition de la croissance des champignons en présence d'un antifongique et permettent de déterminer la concentration minimale inhibitrice (CMI).

Cette valeur peut être ensuite interprétée par rapport aux seuils cliniques (clinical breakpoints, CBP) définis par l'Institut des standards cliniques et de laboratoire (CLSI) et le comité Européen des tests de sensibilité antimicrobien (EUCAST). Ces seuils cliniques permettent d'établir le phénotype sensible, résistant ou intermédiaire de la souche fongique, et ainsi de prédire la réponse des patients au traitement antifongique afin d'adapter celui-ci selon le phénotype de la souche infectante.

Les méthodes de référence CLSI ou EUCAST de détermination de la CMI sont basées sur des microdilutions et se trouvent de ce fait inadaptées à la pratique clinique en routine.

D'autres tests plus faciles à mettre en oeuvre ont été développés et commercialisés pour une utilisation médicale.

Fungitest ^{™} (Biorad) est une méthode de détermination de la sensibilité de souches de levure réalisée à partir d'une suspension cellulaire, en présence de six agents antifongiques (amphotéricine B, flucytosine, fluconazole, itraconazole, kétoconazole et miconazole) à deux concentrations différentes. Elle se présente sous la forme de microplaques comprenant des cupules avec lesdits antifongiques. Elle permet la détermination de la sensibilité de la souche par lecture colorimétrique, après une incubation entre 48 et 72h des microplaques et la mise en présence d'un indicateur redox.

Sensititre Yeast One ^{®} (Trek Diagnostics Systems) est une méthode de diagnostic *in vitro* de la sensibilité des souches de levures incluant également les espèces Candida, Cryptococcus et Aspergillus et développée pour le voriconazole, la caspofungine, l'anidulafungine et la micafungine. Cette méthode se présente sous la forme de microplaques comprenant des cupules et permet la détermination de la CMI à partir de microdilutions et à l'aide d'un indicateur colorimétrique après une incubation de la microplaque entre 24 et 72h selon les espèces.

Etest ^{®} (Biomérieux) est une méthode de détermination de la CMI de microorganismes (bactéries, champignons) développée pour plus de 100 molécules de références comprenant des agents antibiotiques, antifongiques, antimycobactériens, antimicrobiens). Elle se présente sous la forme de bandelettes comportant un gradient exponentiel prédéfini de 15 concentrations d'antifongique ou d'antibiotique qui est appliqué sur une boite de gélose ensemencée. Après incubation de 24h environ, la valeur de la CMI se détermine visuellement par la lecture de la valeur indiquée sur la bandelette et correspondant au bord de l'ellipse d'inhibition. Cette intersection entre l'ellipse d'inhibition et la bandelette indique la concentration pour laquelle la molécule testée inhibe la croissance du microorganisme. La méthode Etest^{®} est la plus utilisée en routine en raison de sa simplicité de mise en oeuvre.

Vitek 2 (Biomérieux) est un système d'identification de bactéries ou de levures et d'antibiogramme automatisé à partir d'un inoculum en milieu liquide et utilisant des plaques de cupules réactionnelles comportant différents tests colorimétriques. Il permet à la fois d'identifier le microorganisme et d'en déterminer la CMI par rapport à un ou plusieurs antifongiques ou antibiotiques dans un délai de 3 à 7 heures. *(*Ling et al., 2003, Evaluation of the VITEK 2 System for Rapid Direct Identification and Susceptibility Testing of Gram-Negative Bacillifrom Positive Blood Cultures*).*

Des méthodes utilisant la cytométrie en flux et des colorants de la viabilité cellules ont également été développées principalement pour *Candida* spp.. Celles-ci ont montré une bonne corrélation avec les méthodes de référence CLSI et EUCAST mais seulement pour quelques couples spécifiques antifongiques-espèces.

Des tests chimiques ont été créés avec par exemple la quantification de la synthèse d'ergostérol.

La plupart des tests de sensibilité commercialisés repose sur des tests colorimétriques ou des tests de croissance pour la détermination de la CMI de la souche donnée.

L'interprétation visuelle des résultats peut être une source d'erreur, sachant que l'effet de traîne de la croissance en présence d'azoles peut également fausser l'interprétation du test et correspond à une croissance à minima dans la zone d'inhibition.

Tous les tests existants présentent des inconvénients, le majeur étant le délai de réponse du test (au moins 24h) et la subjectivité de la lecture du résultat.

La mise au point de nouveaux tests plus rapides et plus fiables permettant une détermination du phénotype d'une souche de champignon vis-à-vis d'un antifongique est donc nécessaire en raison du développement accru de résistances des différentes espèces champignons, afin d'adapter le traitement le plus rapidement possible après le début de l'infection.

Parmi les antifongiques couramment utilisés pour constituer la base des traitements, on distingue notamment la classe des échinocandines comprenant la micafungine, la caspofungine et l'anidulafungine. Cette classe d'antifongiques agit directement sur la paroi des champignons. Ils inhibent la synthèse de B 1,3 glucane de la paroi cellulaire en se fixant à la β-1,3 glucane synthase, une enzyme liée à la membrane et composée de sous-unités régulatrices (Rho) et catalytiques (Fks), dont l'inhibition entraine ainsi l'inhibition de la formation de la paroi.

La principale alternative aux échinocandines sont les antifongiques à action inhibitrice de la synthèse des stérols présents dans la membrane cellulaire. Parmi eux, on distingue la classe des azoles, ou antifongiques azolés, qui comprend notamment le fluconazole, le posaconazole, le voriconazole et l'itraconazole qui sont les plus couramment utilisés. Les antifongiques azolés ciblent le cytochrome P450-Erg11p également appelé Cyp51p intervenant dans la voie de biosynthèse de l'ergostérol, qui est un composant majeur de la membrane fongique. Le blocage de la voie de synthèse de l'ergostérol entraine la production de stérols méthylés toxiques et des dommages dans la membrane cellulaire. Les azolés ont montré qu'ils entraînaient une augmentation de la production des protéines de la paroi en tant que phénomène compensatoire, ce qui suggère que les azolés ne sont pas seulement des inhibiteurs de la membrane mais aussi de la paroi cellulaire.

Les polyènes, essentiellement représentés par l'amphotéricine B présentent une forte toxicité, Les pyrimidines sont aussi utilisées comme antifongiques mais le plus souvent en combinaison avec un antifongique d'une autre classe en raison du développement de résistances aux analogues de pyrimidines.

Le mécanisme d'action des azolés et des échinocandines fait donc intervenir la membrane et / ou la paroi cellulaire. Certaines voies de signalisation de réponses aux stress telles que celles de la PKC (protéine kinase C), la voie HOG (haute osmolarité glycérol), la voie MAPK (MAP kinase) et la voie de la Calcuneurine sont activées en réponse à un stress pariétal et coordonnent une augmentation de la synthèse de chitine afin de compenser les dommages causés à la paroi et/ou à la membrane cellulaire.

Dans la publication de Costa de Oliveira et al. (2013), les auteurs ont constaté que certaines espèces de levures qui présentent la capacité de croître à de fortes concentrations en caspofungine (supérieure à la CMI), présentent également une quantité élevée de chitine dans leur paroi cellulaire.

Walker et al. (2013) ont décrit un effet de croissance paradoxale de champignon en présence d'échinocandines qui survient le plus fréquemment lors de traitement avec de la caspofungine. Ils ont ainsi montré que la croissance de C. *albicans* est inhibée à de faibles concentrations et à de très fortes concentrations, mais une croissance est observée à des concentrations intermédiaires.

Roncero et al. (1988) ont décrit une méthode comprenant la détermination du taux de chitine dans une population decellules d'une souche de champignon Saccharomyces cerevisiae, ledit taux étant associé à la résistance de ladit souche vis-à-vis d'un antifongique (calcifluor). Contrairement à la présente invention, cette méthode ne permet pas une appréhension fine de lasensibilité des différentes souches de champignons et ignore les champignons pluricellulaires.

La demande de brevet WO9506132 décrit différentes souches de levures Candida et leur résistance vis-à-vis d'un antifongique. Cette demande reste muette quant aux autres souches de champignons.

La demande de brevet WO2013130875 décrit des procédés de détermination de la susceptibilité de la sensibilité de micro-organismes envers des antibiotiques et antifongiques basé sur la détection de la croissance des cellules.

Mateusz et al. (2004) décrit l'analyse de la croissance des souches de champignons Candida en présence de différents antifongiques dont la caspofungine.

Ces documents de l'état de la technique ignorent tout un pan de la présente invention et ne permettent pas d'obtenir la finesse de diagnostic proposée ici.

L'un des aspects de l'invention est de fournir un test fiable de détermination du degré de sensibilité d'une souche de champignon.

Un autre aspect de l'invention est de fournir un test fiable de détermination du degré de sensibilité d'une souche de champignon dont le délai de réponse est inférieur à 48h, voire 24h, et plus particulièrement 6,5h.

Un autre aspect de l'invention est de fournir un test fiable de détermination du degré de sensibilité d'une souche de champignon dont l'interprétation est objective, non soumise à l'effet de traîne.

Un autre aspect de l'invention est de fournir un test fiable de détermination du degré de sensibilité d'une souche de champignon utilisable en clinique.

La présente invention a pour objet l'utilisation de la variation éventuelle du taux de chitine dans une population de cellules d'une souche de champignon pour déterminer le degré de sensibilité de ladite souche de champignon vis-à-vis d'un antifongique, ladite variation étant déterminée par rapport au taux de chitine d'une population de cellules de ladite souche de champignon en absence d'antifongique.

Les Inventeurs ont trouvé que la variation éventuelle du taux de chitine permettait de déterminer le degré de sensibilité d'une souche de champignon par rapport à un antifongique.

La variation éventuelle du taux de chitine utilisée pour la détermination du degré de sensibilité signifie que le taux de chitine peut varier, c'est-à-dire augmenter ou diminuer, mais cette variation est éventuelle ce qui signifie qu'il peut y avoir, selon les conditions, une variation du taux de chitine, ou une absence de variation.

Ils ont ainsi mis en évidence qu'une variation ou une absence de variation du taux de chitine dans une population de cellules d'une souche de champignon en présence d'un antifongique par rapport à une même population de cellules en absence d'antifongique, étaient significatives pour la détermination du degré de sensibilité de cette souche de champignon par rapport à cet antifongique.

Le degré de sensibilité d'une souche de champignon vis-à-vis d'un antifongique correspond à la capacité de cet antifongique à tuer ou à inhiber suffisamment la croissance de cette souche.

Par le terme « antifongique », on entend toute molécule qui permet de lutter contre les champignons. Les antifongiques peuvent également être désignés par le terme « antimycosique ».

L'invention concerne la détermination du degré de sensibilité d'une souche de champignon vis-à-vis d'un antifongique, ce qui signifie que la sensibilité de ladite souche est déterminée vis-à-vis d'un seul antifongique déterminé et non pas vis-à-vis d'un mélange d'antifongiques.

Chez les champignons, la chitine est un constituant essentiel de la paroi qui entoure et protège les cellules fongiques vis-à-vis de l'environnement. La chitine participerait également à la rigidité de la paroi cellulaire chez les champignons. La chitine est un polysaccharide azoté de formule (C₈H₁₃NO₅)ₙ, de la famille des glucides, issue de la polymérisation de N-acétylglucosamine liés entre eux par une liaison osidique du type β-1,4, et synthétisé par plusieurs enzymes : les chitine synthases (CHS).

Le terme de « champignons » désigne des organismes eucaryotes pluricellulaires ou unicellulaires, immobiles et qui se nourrissent par l'absorption des molécules organiques directement dans le milieu. Les cellules de champignons présentent comme particularités la présence d'une paroi refermant de la chitine, et l'absence de chlorophylles et/ou de plastes car ces organismes sont hétérotrophes vis-à-vis du carbone. Le terme « champignon » désigne le règne des *Fungi.*

L'expression « population de cellules d'une souche de champignon» détermine un ensemble homogène de cellules c'est-à-dire présentant une homogénéité au niveau génotypique et phénotypique.

L'expression « absence d'antifongique » signifie que la population de cellules de la souche de champignon se trouve en absence de l'antifongique testé dans le cadre de la détermination du degré de sensibilité de ladite souche de champignon, mais également en absence totale de toute autre molécule de la classe des antifongiques.

Par « taux de chitine dans une population de cellules de champignon », on entend la quantité moyenne estimée par la méthode expliquée ci-après, dans une population de cellules de champignon déterminée.

Le degré de sensibilité évalue l'efficacité de l'antifongique c'est-à-dire la capacité de l'antifongique à inhiber la multiplication du champignon ou à le tuer dans des conditions de laboratoire mimant les conditions *in vivo.*

Trois catégories ont été retenues pour l'interprétation du degré de sensibilité *in vitro :* Sensible, Résistant et Intermédiaire.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le degré de sensibilité correspond soit à un phénotype sensible, soit à un phénotype résistant, soit à un phénotype intermédiaire de ladite souche de champignon vis-à-vis d'un antifongique.
- Les souches catégorisées comme sensibles sont celles pour lesquelles la probabilité de succès thérapeutique est forte (90 %) dans le cas d'un traitement par voie systémique avec la posologie recommandée dans le résumé des caractéristiques du produit (RCP), rédigé par l'Agence Française de Sécurité Sanitaire des Produits de Santé (AFSSAPS).
- Les souches catégorisées comme résistantes sont celles pour lesquelles il existe une forte probabilité (40 %) d'échec thérapeutique quel que soit le type de traitement et la dose d'antifongique utilisé.
- Les souches catégorisées comme intermédiaires sont celles pour lesquelles le succès thérapeutique est imprévisible. Ces souches forment un ensemble hétérogène pour lequel les résultats obtenus *in vitro* ne sont pas prédictifs d'un succès thérapeutique. En effet, ces souches :
   - peuvent présenter un mécanisme de résistance dont l'expression *in vitro* est faible, avec pour conséquence leur classement dans la catégorie sensible. Cependant, *in vivo,* une partie de ces souches apparaît résistante au traitement et conduit un échec thérapeutique ;
   - peuvent présenter un mécanisme de résistance dont l'expression n'est pas suffisante pour justifier un classement dans la catégorie résistante, mais suffisamment faible pour espérer un effet thérapeutique dans certaines conditions (fortes concentrations locales ou posologies accrues).

Les champignons sont des organismes qui peuvent être pluricellulaires ou unicellulaires.

Dans un cas particulier, les champignons peuvent être unicellulaires, leur appareil végétatif est alors constitué d'une seule cellule.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est un champignon unicellulaire.

D'autres champignons sont pluricellulaires et leur appareil végétatif pluricellulaire s'apparente alors à des filaments, appelés hyphes, d'où l'appellation de champignons filamenteux pour désigner les champignons pluricellulaires. Ces hyphes peuvent être plus ou moins ramifiés et enchevêtrés entre eux. L'ensemble des hyphes forme le mycélium.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est un champignon pluricellulaire.

Pour déterminer le degré de sensibilité d'une souche de champignon vis-à-vis d'un antifongique, le paramètre de croissance de ladite souche de champignon peut être pris en compte en plus de la variation éventuelle du taux de chitine.

Dans le cas de la détermination du degré de sensibilité d'une souche de champignon unicellulaire, des cellules de ladite souche constituent le matériel vivant à partir duquel le degré de sensibilité sera déterminé.

Ainsi dans le cas d'une souche de champignon unicellulaire, la croissance de ladite souche est évaluée par quantification du nombre de cellules de ladite souche de champignon dans le milieu.

Selon un mode de réalisation particulier, la présente invention a pour objet l'utilisation de la variation éventuelle du taux de chitine dans une population de cellules d'une souche de champignon, ledit champignon étant un champignon unicellulaire, et de la variation éventuelle du nombre de cellules d'une population de cellules de ladite souche de champignon, pour déterminer le degré de sensibilité de ladite souche de champignon vis-à-vis d'un antifongique, ladite variation du nombre de cellules étant déterminée par rapport au nombre de cellules d'une population de cellules de ladite souche de champignon en absence d'antifongique.

Dans le cas de la détermination du degré de sensibilité d'une souche de champignon pluricellulaire, les conidies de ladite souche constituent le matériel vivant à partir duquel le degré de sensibilité sera déterminé.

Ainsi, dans le cas d'une souche de champignon pluricellulaire, la croissance de ladite souche est évaluée par quantification de la longueur de l'hyphe végétatif de germination émis par les cellules de ladite souche de champignon.

Dans le cas d'une souche de champignon pluricellulaire, la croissance de ladite souche est évaluée par quantification de la longueur de l'hyphe végétatif de germination émis par les conidies.

Selon un mode de réalisation particulier, la présente invention a pour objet l'utilisation de la variation éventuelle du taux de chitine dans une population de cellules d'une souche de champignon, ledit champignon étant un champignon pluricellulaire, et de la variation éventuelle de la longueur de l'hyphe végétatif de germination dans ladite population de cellules de ladite souche de champignon, pour déterminer le degré de sensibilité de ladite souche de champignon vis-à-vis d'un antifongique, ladite variation de la longueur de l'hyphe végétatif de germination étant déterminée par rapport à la longueur de l'hyphe végétatif de germination d'une population de cellules de ladite souche de champignon en absence d'antifongique.

Par « hyphe végétatif de germination » on entend, le premier hyphe végétatif émis par une spore lors de la germination de cette dernière.

Les champignons se reproduisent et se disséminent sous la forme de cellules appelées conidies. Le terme « conidies » désigne une spore assurant la multiplication asexuée des champignons et non capable de mobilité autonome.

Dans le cas d'une souche de champignon unicellulaire ou pluricellulaire, la seule utilisation de la variation éventuelle du taux de chitine suffit pour la détermination du phénotype résistant de la souche vis-à-vis d'un antifongique dans le cas d'une absence d'augmentation du taux de chitine ou d'une augmentation du taux de chitine inférieure à 10 % dans une population de cellules d'une souche de champignon en présence dudit antifongique par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, dans laquelle le phénotype résistant de ladite souche de champignon vis-à-vis d'un antifongique est déterminé par une absence d'augmentation du taux de chitine ou une augmentation du taux de chitine inférieure à 10 %, par rapport au taux de chitine d'une population de cellules de ladite souche de champignon en absence d'antifongique. L'expression « augmentation du taux de chitine inférieure à 10% » signifie qu'une augmentation du taux de chitine est observée dans une population de cellules d'une souche de champignon en présence d'antifongique par rapport à une population de cellules de ladite souche de champignon en absence d'antifongique et que cette augmentation peut prendre toute valeur supérieure à 0 et strictement inférieure à 10%.

L'expression « absence d'augmentation du taux de chitine » signifie que le taux de chitine dans une population de cellules d'une souche de champignon en présence d'un antifongique n'augmente pas par rapport à une population de cellules de ladite souche de champignon en absence d'antifongique, c'est-à-dire qu'il peut soit être stationnaire, soit diminuer.

Selon un mode de réalisation particulier, la présente invention a pour objet l'utilisation de la variation éventuelle du taux de chitine dans une population de cellules d'une souche de champignon, dans laquelle le phénotype résistant de ladite souche de champignon vis-à-vis d'un antifongique est déterminé par une augmentation du taux de chitine inférieure à 10 % ou par une diminution du taux de chitine ou par un taux de chitine stationnaire, par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, dans laquelle le phénotype résistant de ladite souche de champignon vis-à-vis d'un antifongique est déterminé par une augmentation du taux de chitine inférieure à 10 % ou par une diminution du taux de chitine inférieure à 20% ou par un taux de chitine stationnaire, par rapport au taux de chitine d'une population de cellules de ladite souche de champignon en absence d'antifongique.

Dans le cas d'une souche de champignon unicellulaire ou pluricellulaire, la seule utilisation de la variation éventuelle du taux de chitine suffit pour la détermination du phénotype intermédiaire de la souche vis-à-vis d'un antifongique dans le cas d'une augmentation du taux de chitine de 10% à une valeur inférieure à 20% dans une population de cellules d'une souche de champignon en présence dudit antifongique par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, dans laquelle le phénotype intermédiaire de ladite souche de champignon vis-à-vis d'un antifongique est déterminé par une augmentation du taux de chitine de 10% à une valeur inférieure à 20%, par rapport au taux de chitine d'une population de cellules de ladite souche de champignon en absence d'antifongique.

L'expression « augmentation du taux de chitine de 10% à une valeur inférieure à 20% » signifie qu'une augmentation du taux de chitine est observée et que cette augmentation peut prendre la valeur de 10% ou toute autre valeur supérieure à 10% et strictement inférieure à 20%.

Dans le cas d'une souche de champignon unicellulaire ou pluricellulaire, la seule utilisation de la variation éventuelle du taux de chitine suffit pour déterminer que le degré de sensibilité de la souche ne correspond pas à un phénotype résistant mais à un phénotype sensible ou intermédiaire vis-à-vis d'un antifongique dans le cas d'une augmentation du taux de chitine supérieure ou égale à 20% dans une population de cellules d'une souche de champignon en présence dudit antifongique par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, dans laquelle le phénotype sensible ou intermédiaire de ladite souche de champignon vis-à-vis d'un antifongique est déterminé par une augmentation du taux de chitine supérieure ou égale à 20%, par rapport au taux de chitine d'une population de cellules de ladite souche de champignon en absence d'antifongique.

L'expression « augmentation du taux de chitine supérieure ou égale à 20% » signifie qu'une augmentation du taux de chitine est observée dans une population de cellules d'une souche de champignon par rapport à une population de cellules de ladite souche de champignon en absence d'antifongique et que cette augmentation peut prendre la valeur de 20% ou toute autre valeur supérieure à 20%.

Cependant, la seule utilisation de la variation éventuelle du taux de chitine ne suffit pas pour déterminer si le degré de sensibilité de ladite souche vis-à-vis d'un antifongique correspond à un phénotype sensible ou intermédiaire dans le cas d'une augmentation du taux de chitine supérieure ou égale à 20% dans une population de cellules d'une souche de champignon en présence d'antifongique par rapport au taux de chitine d'une population de cellules de ladite souche de champignon en absence d'antifongique.

Dans le cas d'une souche de champignon unicellulaire, l'utilisation de la variation éventuelle du taux de chitine associée à la variation éventuelle du nombre de cellules, dans une population de cellules d'une souche de champignon en présence d'antifongique par rapport à une population de cellules de ladite souche de champignon en absence d'antifongique, permet la détermination du phénotype intermédiaire ou sensible de la souche vis-à-vis dudit antifongique, dans le cas d'une augmentation du taux de chitine supérieure ou égale à 20%.

Plus particulièrement, dans le cas d'une souche de champignon unicellulaire, l'utilisation de la variation éventuelle du taux de chitine associée à la variation du nombre de cellules, dans une population de cellules d'une souche de champignon en présence d'antifongique par rapport à une population de cellules de ladite souche de champignon en absence d'antifongique, permet la détermination du phénotype sensible vis-à-vis dudit antifongique dans le cas d'une augmentation du taux de chitine supérieure ou égale à 20% et d'une diminution du nombre de cellules d'au moins 0,3 log.

Selon un mode de réalisation particulier, la présente invention a pour objet l'utilisation de la variation éventuelle du taux de chitine dans une population de cellules d'une souche de champignon, ledit champignon étant un champignon unicellulaire, dans laquelle le phénotype sensible de ladite souche de champignon vis-à-vis d'un antifongique est déterminé par une augmentation du taux de chitine supérieure ou égale à 20% par rapport au taux de chitine d'une population de cellules de ladite souche de champignon en absence d'antifongique, et par la détermination de la variation du nombre de cellules dans ladite population de cellules de ladite souche de champignon en présence d'antifongique, ladite variation étant une diminution du nombre de cellules d'au moins 0,3 log par rapport au nombre de cellules d'une population de cellules de ladite souche de champignon en absence d'antifongique.

L'expression « diminution du nombre de cellules d'au moins 0,3 log » signifie qu'une diminution du nombre de cellules est observée dans une population de cellules d'une souche de champignons en présence d'antifongique par rapport à une population de cellules de ladite souche champignon en absence dudit antifongique et que cette diminution peut prendre la valeur de 0,3 log ou toute valeur supérieure à 0,3 log.

Dans le cas d'une souche de champignon unicellulaire, l'utilisation de la variation éventuelle du taux de chitine associée à la variation éventuelle du nombre de cellules, dans une population de cellules d'une souche de champignon en présence d'antifongique par rapport à une population de cellules de ladite souche de champignon en absence d'antifongique, permet la détermination du phénotype intermédiaire vis-à-vis dudit antifongique dans le cas d'une augmentation du taux de chitine supérieure ou égale à 20% et d'une diminution du nombre de cellules de moins de 0,3 log ou d'un nombre de cellules stationnaire. La variation éventuelle du nombre de cellules, dans une population de cellules d'une souche de champignon en présence d'antifongique par rapport à une population de cellules de ladite souche de champignon en absence d'antifongique, signifie que le nombre de cellules peut varier, c'est-à-dire augmenter ou diminuer. Cette variation est éventuelle ce qui signifie qu'il peut y avoir une variation du nombre de cellules, c'est-à-dire augmentation ou diminution, ou une absence de variation, le nombre de cellules étant alors stationnaire.

L'expression « diminution du nombre de cellules de moins de 0,3 log » signifie qu'une diminution du nombre de cellules est observée dans une population de cellules d'une souche de champignons en présence d'antifongique par rapport à une population de cellules de ladite souche champignon en absence dudit antifongique et que cette diminution peut prendre des valeurs positives strictement inférieures à 0,3 log.

Selon un mode de réalisation particulier, la présente invention a pour objet l'utilisation de la variation éventuelle du taux de chitine dans une population de cellules d'une souche de champignon, ledit champignon étant un champignon unicellulaire, dans laquelle le phénotype intermédiaire de ladite souche de champignon vis-à-vis d'un antifongique est déterminé par une augmentation du taux de chitine supérieure ou égale à 20% par rapport au taux de chitine d'une population de cellules de ladite souche de champignon en absence d'antifongique, et par la variation éventuelle du nombre de cellules dans ladite population de cellules par rapport au nombre de cellules dans une population de cellules de ladite souche de champignon en absence d'antifongique, ladite variation éventuelle étant une diminution du nombre de cellules de moins de 0,3 log ou un nombre de cellules stationnaire, par rapport au nombre de cellules d'une population de cellules de ladite souche de champignon en absence d'antifongique.

Dans le cas d'une souche de champignon pluricellulaire, la seule utilisation de la variation éventuelle du taux de chitine suffit pour la détermination du phénotype résistant de la souche vis-à-vis dudit antifongique dans le cas d'une augmentation du taux de chitine inférieure à 10% ou d'une absence d'augmentation du taux de chitine dans une population de cellules d'une souche de champignon en présence d'antifongique par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, ledit champignon étant un champignon pluricellulaire, dans laquelle le phénotype résistant de ladite souche de champignon vis-à-vis d'un antifongique est déterminé par une augmentation du taux de chitine inférieure à 10 % ou une absence d'augmentation du taux de chitine, par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, ledit champignon étant un champignon pluricellulaire, dans laquelle le phénotype résistant de ladite souche de champignon vis-à-vis dudit antifongique est déterminé par une augmentation du taux de chitine inférieure à 10 % ou par une diminution du taux de chitine ou par un taux de chitine stationnaire, par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, ledit champignon étant un champignon pluricellulaire, dans laquelle le phénotype résistant de ladite souche de champignon vis-à-vis dudit antifongique est déterminé par une augmentation du taux de chitine inférieure à 10 % ou par une diminution du taux de chitine inférieure à 20% ou par un taux de chitine stationnaire, par rapport au taux de chitine d'une population de cellules de ladite souche de champignon en absence d'antifongique.

Dans le cas d'une souche de champignon pluricellulaire, la seule utilisation de la variation éventuelle du taux de chitine suffit pour déterminer que le degré de sensibilité de la souche vis-à-vis d'un antifongique correspond à un phénotype intermédiaire, dans le cas d'une augmentation du taux de chitine de 10% à une valeur inférieure à 20% dans une population de cellules d'une souche de champignon en présence dudit antifongique par rapport au taux de chitine d'une population de cellules de ladite souche de champignon en absence d'antifongique.

Selon un mode de réalisation particulier, la présente invention a pour objet l'utilisation de la variation éventuelle du taux de chitine dans une population de cellules d'une souche de champignon, ledit champignon étant un champignon pluricellulaire, dans laquelle le phénotype intermédiaire de ladite souche de champignon vis-à-vis d'un antifongique est déterminé par une augmentation du taux de chitine de 10% à une valeur inférieure à 20%, par rapport au taux de chitine d'une population de cellules de ladite souche de champignon en absence d'antifongique.

Dans le cas d'une souche de champignon pluricellulaire, l'utilisation de la variation éventuelle du taux de chitine associée à la variation éventuelle de la longueur de l'hyphe végétatif de germination émis par les cellules de ladite souche de champignon, dans une population de cellules d'une souche de champignon en présence d'antifongique par rapport à une population de cellules de ladite souche de champignon en absence d'antifongique, permet de la détermination du phénotype intermédiaire ou sensible de la souche vis-à-vis dudit antifongique, dans le cas d'une augmentation du taux de chitine supérieure ou égale à 20% et d'une diminution de la longueur dudit l'hyphe ou d'une longueur stationnaire dudit hyphe.

Selon un mode de réalisation particulier, la présente invention a pour objet l'utilisation de la variation éventuelle du taux de chitine dans une population de cellules d'une souche de champignon, ledit champignon étant un champignon pluricellulaire, dans laquelle le phénotype sensible ou intermédiaire de ladite souche de champignon vis-à-vis d'un antifongique est déterminé par une augmentation du taux de chitine supérieure ou égale à 20%, par rapport au taux de chitine d'une population de cellules de ladite souche de champignon en absence d'antifongique, et par la variation éventuelle de la longueur de l'hyphe végétatif de germination dans ladite population de cellules par rapport la longueur dudit hyphe dans une population de cellules de ladite souche de champignon en absence d'antifongique, ladite variation éventuelle étant une diminution de la longueur dudit hyphe ou une longueur stationnaire dudit hyphe, par rapport à la longueur de l'hyphe végétatif de germination d'une population de cellules de ladite souche de champignon en absence d'antifongique.

Chez les champignons unicellulaires et pluricellulaires, la chitine est un constituant de la paroi cellulaire. Ainsi, le taux de chitine est déterminé dans la paroi des cellules de champignon par la technique ci-après.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la variation éventuelle du taux de chitine est déterminée dans la paroi desdites cellules de champignon.

Le seuil minimal de cellules dans ladite population de cellules de ladite souche de champignon présentant une augmentation du taux de chitine est d'au moins 10%.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, ledit champignon étant un champignon unicellulaire, dans laquelle le seuil minimal de cellules dans ladite population de cellules de ladite souche de champignon présentant une augmentation du taux de chitine est d'au moins 10%.

L'expression « augmentation du taux de chitine d'au moins 10% » signifie qu'une augmentation du taux de chitine est observée dans une population de cellules d'une souche de champignon par rapport à une population de cellules de ladite souche de champignon en absence d'antifongique et que cette augmentation peut prendre la valeur de 10% ou toute autre valeur supérieure à 10%.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100%.

Selon un mode de réalisation particulier, la présente invention a pour objet l'utilisation de la variation éventuelle du nombre de cellules dans une population de cellules d'une souche de champignon pour déterminer le degré de sensibilité de ladite souche de champignon vis-à-vis d'un antifongique, dans laquelle le seuil minimal de diminution du nombre de cellules dans ladite population de cellules de ladite souche de champignon est d'au moins 0.3 log, notamment 0,4 ; 0,5 ; 0,6 ; 0,7 ; 0,8 ; 0,9 ; 1 ; 1,1 ; 1,2 ; 1,3 ; 1,4 ; 1,5 ; 1,6 ; 1,7 ; 1,8 ; 1,9 ; 2 ; 2,1 ; 2,2 ; 2,3 ; 2,4 ; 2,5 ; 2,6 ; 2,7 ; 2,8 ; 2,9 ; 3 .

Le degré de sensibilité de la souche de champignon vis-à-vis d'un antifongique est déterminé via la variation éventuelle du taux de chitine de cellules de ladite souche de champignon cultivées en présence d'un gradient de concentrations en antifongique dans le milieu de culture liquide, par rapport à ladite souche de champignon en absence d'antifongique dans leur milieu de culture.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle ladite variation éventuelle du taux de chitine est déterminée en présence d'un gradient de concentrations d'un antifongique.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le gradient de concentrations d'un antifongique est de 0,0009 à 130 µg/ml.

Le taux de chitine dans les cellules d'une souche de champignon peut être mesuré par différentes techniques telles que le dosage chimique ou la mesure de la fluorescence.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la variation éventuelle du taux de chitine est déterminée par fluorescence.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la variation éventuelle du taux de chitine est déterminée par une méthode de microscopie à fluorescence.

Le taux de chitine dans les cellules d'une souche de champignon unicellulaire ou pluricellulaire est mesuré par une méthode de microscopie à fluorescence, telles qu'une méthode de microscopie à fluorescence mesurant la quantité globale de fluorescence ou une méthode de microscopie automatisée à haut contenu d'images (HCA), qui mesure a la quantité globale de fluorescence par cellule.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle ladite méthode de microscopie à fluorescence est la microscopie automatisée à haut contenu d'images (HCA).

Chez les champignons unicellulaires ou pluricellulaires, la mesure de la chitine est réalisée dans les parois des cellules de champignons unicellulaires ou pluricellulaires par une méthode de microscopie à fluorescence, à l'aide d'un marqueur fluorescent.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la variation éventuelle du taux de chitine est déterminée à l'aide d'un marqueur fluorescent.

Plus particulièrement, le marqueur fluorescent utilisé pour la mesure de la chitine dans les parois de cellules de champignons unicellulaires ou pluricellulaires par la méthode de microcopie HCA est le Calcofluor White.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle ledit marqueur fluorescent est le Calcofluor White.

Le degré de sensibilité d'une souche de champignon unicellulaire ou pluricellulaire est déterminé vis-à-vis d'un antifongique. Les antifongiques peuvent avoir différents modes d'action.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle l'antifongique agit sur la paroi de façon directe sur les Beta-glucans ou les mannoproteines ou indirecte via une action sur la membrane ou sur tout autre constituant de la cellule fongique.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle l'antifongique est choisi parmi le groupe comprenant ou étant constitué des antifongiques azolés ou autres molécules inhibitrices de la synthèse de l'ergosterol tels que les allylamines dont la terbinafine ou morpholines, ou des échinocandines.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, le groupe des antifongiques étant dépourvu des polyènes tels que l'amphotéricine B et la nystatine et des antifongiques à action directe sur la chitine.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle l'antifongique azolé est choisi parmi le groupe comprenant ou étant constitué à ce jour du fluconazole, du voriconazole, du posaconazole, de l'itraconazole et de l'isavuconazole.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle l'échinocandine est choisie parmi le groupe comprenant ou étant constitué à ce jour de l'anidulafungine, de la caspofungine, et de la micafungine.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle l'antifongique est choisi parmi le groupe des antifongiques dépourvu des polyènes tels que l'amphotéricine B et la nystatine, et dépourvu des antifongiques à action directe sur la chitine, plus particulièrement :
- parmi le groupe comprenant ou étant constitué des antifongiques azolés tels que le fluconazole, le voriconazole, le posaconazole, l'itraconazole et l'isavuconazole, ou autres molécules inhibitrices de la synthèse de l'ergosterol tels que les allylamines dont la terbinafine ou morpholines,
- ou parmi les échinocandines, l'échinocandine étant plus particulièrement choisie parmi le groupe comprenant ou étant constitué de l'anidulafungine, de la caspofungine et de la micafungine.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, le groupe des antifongiques comprenant en outre les polyènes tels que l'amphotéricine B et la nystatine et étant dépourvu des antifongiques à action directe sur la chitine.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle l'antifongique à tester est choisi parmi le groupe constitué du fluconazole, du posaconazole, du voriconazole, de l'itraconazole, de l'isavuconazole pour la classe des azolés ; de la caspofungine, de la micafungine et de l'anidulafungine pour la classe des échinocandines ; et de la nystatine et de l'amphotéricine B pour la classe des polyènes, ledit groupe étant dépourvu des antifongiques à action directe sur la chitine.

Dans un mode de réalisation particulier, le degré de sensibilité est déterminé pour des champignons unicellulaires vis-à-vis d'un antifongique.

Les champignons unicellulaires sont majoritairement représentés par les levures.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est une levure.

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la population de cellules de levures est choisie parmi le groupe comprenant ou étant constitué des genres *Candida, Cryptococcus, Saccharomyces, Trichosporon, Rhodotorula, Malassezia.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la population de cellules de levures est du genre *Candida.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle la levure du genre *Candida* est choisie parmi le groupe comprenant ou étant constitué des espèces : *Candida albicans, C. glabatra, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est choisi parmi le groupe comprenant ou étant constitué des genres *Aspergillus, Fusarium, Scedosporium, Lichteimia, Rhizopus, Rhizomucor, Mucor, Paecylomyces, Geotrichum.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est du genre *Aspergillus.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon du genre *Aspergillus* est choisi parmi le groupe comprenant ou étant constitué des espèces : *A. fumigatus, A. flavus, A. nidulans, A. terreus, A. versicolor, A. niger.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est du genre *Fusarium,* et notamment appartient à l'une des espèces: *Fusarium solani, Fusarium oxysporum.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est du genre *Scedosporium,* et notamment appartient à l'une des espèces: *Scedosporium apiospermum, Scedosporium prolificans.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est de l'ordre *Mucorales,* et notamment appartient à l'un des genres *Mucor, Lichteimia, Rhizopus, Rhizomucor.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon de l'ordre *Mucorales* est choisi parmi le groupe comprenant ou étant constitué des espèces : *Lichteimia corymbifera* , *Rhizopus oryzae, Rhizomucor pusillus, Mucor racemosa.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est du genre *Geotrichum.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est du genre *Paecylomyces.*

Selon un mode de réalisation particulier, la présente invention a pour objet l'utilisation de la variation éventuelle du taux de chitine dans une population de cellules d'une souche de champignon pour déterminer le degré de sensibilité de ladite souche de champignon vis-à-vis d'un antifongique, dans laquelle le champignon est choisi parmi le groupe comprenant ou étant constitué des genres *Aspergillus, Fusarium, Scedosporium, Lichteimia, Rhizopus, Rhizomucor, Mucor, Geotrichum, Paecylomyces,* et des espèces *Aspergillus fumigatus, Aspergillus flavus, Aspergillus niger, Aspergillus nidulans, Aspergillus terreus, Fusarium solani, Fusarium oxysporum, Scedosporium apiospermum, Scedosporium prolificans, Lichtei mia corymbifera* , *Rhizopus oryzae, Rhizomucor pusillus, Mucor racemosa.*

La présente invention a également pour objet un procédé de détermination du degré de sensibilité d'une population de cellules d'une souche de champignon vis-à-vis d'un antifongique.

Dans un mode de réalisation particulier, la présente invention a pour objet un procédé de détermination du degré de sensibilité d'une population de cellules d'une souche de champignon vis-à-vis d'un antifongique comprenant une étape de détermination de la variation éventuelle du taux de chitine dans la population de cellules de ladite souche de champignon en présence dudit antifongique par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype sensible, résistant ou intermédiaire, d'une population de cellules d'une souche de champignon vis-à-vis d'un antifongique comprenant une étape de détermination de la variation éventuelle du taux de chitine dans la population de cellules de ladite souche de champignon en présence dudit antifongique par rapport au taux de chitine d'une population de cellules de ladite souche de champignon en absence d'antifongique.

Selon un mode de réalisation particulier, la présente invention a pour objet un procédé de détermination du degré de sensibilité d'une population de cellules d'une souche de champignon, ledit champignon étant un champignon unicellulaire, vis-à-vis d'un antifongique, comprenant une étape de détermination de la variation éventuelle du taux de chitine dans la population de cellules de ladite souche de champignon en présence dudit antifongique par rapport au taux de chitine d'une population de cellules de ladite souche de champignon en absence d'antifongique.

Selon un mode de réalisation particulier, la présente invention a pour objet un procédé de détermination du degré de sensibilité d'une population de cellules d'une souche de champignon, ledit champignon étant un champignon pluricellulaire, vis-à-vis d'un antifongique, comprenant une étape de détermination de la variation éventuelle du taux de chitine dans la population de cellules de ladite souche de champignon en présence dudit antifongique par rapport au taux de chitine d'une population de cellules de ladite souche de champignon en absence d'antifongique.

Selon un mode de réalisation particulier, la présente invention a pour objet un procédé de détermination du degré de sensibilité d'une population de cellules d'une souche de champignon, ledit champignon étant un champignon unicellulaire, vis-à-vis d'un antifongique, comprenant une étape de détermination de la variation éventuelle du taux de chitine de la population de cellules de ladite souche de champignon en présence dudit antifongique par rapport au taux de chitine d'une population de cellules de ladite souche de champignon en absence d'antifongique, et une étape de détermination de la variation éventuelle du nombre de cellules dans ladite population de cellules de ladite souche de champignon en présence dudit antifongique, ladite variation du nombre de cellules étant déterminée par rapport au nombre de cellules d'une population de cellules de ladite souche de champignon en absence d'antifongique.

Selon un mode de réalisation particulier, la présente invention a pour objet un procédé de détermination du degré de sensibilité d'une population de cellules d'une souche de champignon, ledit champignon étant un champignon pluricellulaire, vis-à-vis d'un antifongique, comprenant une étape de détermination de la variation éventuelle du taux de chitine dans la population de cellules de ladite souche de champignon en présence dudit antifongique par rapport au taux de chitine d'une population de cellules de ladite souche de champignon en absence d'antifongique, et une étape de détermination de la variation éventuelle de la longueur de l'hyphe végétatif de germination dans ladite population de cellules de champignon en présence dudit antifongique, ladite variation éventuelle de la longueur de l'hyphe végétatif de germination étant déterminée par rapport à la longueur de l'hyphe végétatif de germination dans une population de cellules de ladite souche de champignon en absence d'antifongique.

Selon un mode de réalisation particulier, la présente invention a pour objet un procédé de détermination du degré de sensibilité correspondant à un phénotype résistant, d'une population de cellules d'une souche de champignon vis-à-vis d'un antifongique, comprenant une étape de détermination de la variation éventuelle du taux de chitine dans la population de cellules de ladite souche de champignon en présence dudit antifongique, ladite variation éventuelle étant une augmentation du taux de chitine inférieure à 10% ou une absence d'augmentation du taux de chitine, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype résistant, d'une population de cellules d'une souche de champignon vis-à-vis d'un antifongique, comprenant une étape de détermination de la variation éventuelle du taux de chitine dans la population de cellules de ladite souche de champignon en présence dudit antifongique, ladite variation éventuelle étant une augmentation du taux de chitine inférieure à 10% ou une diminution du taux de chitine ou un taux de chitine stationnaire, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype résistant, d'une population de cellules d'une souche de champignon vis-à-vis d'un antifongique, comprenant une étape de détermination de la variation éventuelle du taux de chitine dans la population de cellules de ladite souche de champignon en présence dudit antifongique, ladite variation éventuelle étant une augmentation du taux de chitine inférieure à 10% ou une diminution du taux de chitine inférieure à 20% ou un taux de chitine stationnaire, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype intermédiaire, d'une population de cellules d'une souche de champignon vis-à-vis d'un antifongique, comprenant une étape de détermination de la variation du taux de chitine dans la population de cellules de ladite souche de champignon en présence dudit antifongique, ladite variation étant une augmentation du taux de chitine de 10% à une valeur inférieure à 20%, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype sensible ou intermédiaire, d'une population de cellules de champignon vis-à-vis d'un antifongique, comprenant une étape de détermination de la variation du taux de chitine dans la population de cellules de ladite souche de champignon en présence dudit antifongique, ladite variation étant une augmentation du taux de chitine supérieure ou égale à 20%, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype sensible, d'une population de cellules d'une souche de champignon, ledit champignon étant un champignon unicellulaire, vis-à-vis d'un antifongique, comprenant une étape de détermination de la variation du taux de chitine dans la population de cellules de ladite souche de champignon en présence dudit antifongique, ladite variation étant une augmentation du taux de chitine supérieure ou égale à 20%, par rapport au taux de chitine d'une population de cellules de ladite souche de champignon en absence d'antifongique et une étape de détermination de la variation du nombre de cellules dans ladite population de cellules de ladite souche de champignon en présence dudit antifongique, ladite variation étant une diminution du nombre de cellules d'au moins 0,3 log par rapport au nombre de cellules d'une population de cellules de ladite souche de champignon en absence d'antifongique.

Selon un mode de réalisation particulier, la présente invention a pour objet un procédé de détermination du degré de sensibilité correspondant à un phénotype intermédiaire, d'une population de cellules d'une souche de champignon, ledit champignon étant un champignon unicellulaire, vis-à-vis d'un antifongique, comprenant une étape de détermination de la variation du taux de chitine dans la population de cellules de ladite souche de champignon en présence dudit antifongique, ladite variation étant une augmentation du taux de chitine supérieure ou égale à 20%, par rapport au taux de chitine d'une population de cellules de ladite souche de champignon en absence d'antifongique, et une étape de détermination de la variation éventuelle du nombre de cellules de ladite souche de champignon en présence dudit antifongique, ladite variation éventuelle étant une diminution du nombre de cellules de moins de 0,3 log ou un nombre de cellules stationnaire, par rapport au nombre de cellules dans une population de cellules de ladite souche de champignon en absence d'antifongique.

Selon un mode de réalisation particulier, la présente invention a pour objet un procédé de détermination du degré de sensibilité correspondant à un phénotype sensible ou intermédiaire, d'une population de cellules d'une souche de champignon, ledit champignon étant un champignon pluricellulaire, vis-à-vis d'un antifongique, comprenant une étape de détermination de la variation du taux de chitine dans la population de cellules de ladite souche de champignon en présence dudit antifongique, ladite variation étant une augmentation du taux de chitine supérieure ou égale à 20% par rapport au taux de chitine d'une population de cellules de ladite souche de champignon en absence d'antifongique, et une étape de détermination de la variation éventuelle de la longueur de l'hyphe végétatif de germination dans ladite population de cellules de ladite souche de champignon en présence dudit antifongique, ladite variation éventuelle de la longueur de l'hyphe végétatif de germination étant une diminution de la longueur dudit hyphe ou une longueur stationnaire dudit hyphe, par rapport à la longueur de l'hyphe végétatif de germination dans une population de cellules de ladite souche de champignon en absence d'antifongique.

Selon un mode de réalisation particulier, la présente invention a pour objet un procédé de détermination du degré de sensibilité correspondant à un phénotype sensible d'une population de cellules d'une souche de champignon vis-à-vis d'un antifongique, comprenant une étape de détermination de l'augmentation du taux de chitine dans la population de cellules de ladite souche de champignon en présence dudit antifongique par rapport au taux de chitine d'une population de cellules de ladite souche de champignon en absence d'antifongique, ladite augmentation étant d'au moins 20%, notamment 20%, 25 %, 30%, 35%, 40%, 45%, 50%, 55%, 60%, 65%, 70%, 75%, 80%, 85%, 90%, 95% et 100%.

Selon un mode de réalisation particulier, la présente invention a pour objet un procédé de détermination du degré de sensibilité d'une population de cellules d'une souche de champignon vis-à-vis d'un antifongique, comprenant une étape de détermination de la variation éventuelle du taux de chitine effectuée en présence d'un gradient de concentrations dudit antifongique.

Selon un mode de réalisation particulier, la présente invention a pour objet un procédé de détermination du degré de sensibilité d'une population de cellules d'une souche de champignon vis-à-vis d'un antifongique, dans lequel le gradient de concentrations d'antifongique est de 0.0009 à 130 µg/ml, de 0.0009 à 1 µg/ml, de 0.0009 à 5 µg/ml, de 0.0009 à 8 µg/ml ou de 0.0009 à 16 µg/ml.

Selon un mode de réalisation particulier, la présente invention a pour objet un procédé de détermination du degré de sensibilité d'une population de cellules d'une souche de champignon vis-à-vis d'un antifongique, dans lequel la variation éventuelle du taux de chitine dans la population de cellules de ladite souche de champignon en présence dudit antifongique par rapport au taux de chitine d'une population de cellules de ladite souche de champignon en absence d'antifongique est déterminée par méthode de fluorescence.

Selon un mode de réalisation particulier, la présente invention a pour objet un procédé de détermination du degré de sensibilité d'une population de cellules d'une souche de champignon vis-à-vis d'un antifongique, dans laquelle la méthode de fluorescence est une méthode de microscopie à fluorescence.

Selon un mode de réalisation particulier, la présente invention a pour objet un procédé de détermination du degré de sensibilité d'une population de cellules d'une souche de champignon vis-à-vis d'un antifongique, dans laquelle la méthode de microscopie à fluorescence est la microscopie automatisée à haut contenu d'images (HCA).

Selon un mode de réalisation particulier, la présente invention a pour objet un procédé de détermination du degré de sensibilité d'une population de cellules d'une souche de champignon vis-à-vis d'un antifongique, comprenant une étape de marquage fluorescent des cellules de ladite souche de champignon permettant la détermination de la variation éventuelle du taux de chitine.

Selon un mode de réalisation particulier, la présente invention a pour objet un procédé de détermination du degré de sensibilité d'une population de cellules d'une souche de champignon vis-à-vis d'un antifongique, comprenant une étape de marquage fluorescent des cellules de ladite souche de champignon effectuée à l'aide de Calcofluor White.

Selon un mode de réalisation particulier, la présente invention a pour objet un procédé de détermination du degré de sensibilité d'une population de cellules d'une souche de champignon vis-à-vis d'un antifongique, dans lequel la variation éventuelle du taux de chitine dans la population de cellules de ladite souche de champignon en présence dudit antifongique est déterminée par rapport au taux de chitine d'une population de cellules de ladite souche de champignon en absence d'antifongique, et dans lequel ledit antifongique agit sur la paroi de façon directe sur les Beta-glucans ou les mannoproteines ou indirecte via une action sur la membrane ou sur tout autre constituant de la cellule fongique.

Selon un mode de réalisation particulier, la présente invention a pour objet un procédé de détermination du degré de sensibilité d'une population de cellules d'une souche de champignon vis-à-vis d'un antifongique, dans lequel ledit antifongique est choisi parmi le groupe comprenant des antifongiques azolés ou autres molécules inhibitrices de la synthèse de l'ergosterol tels que les allylamines dont la terbinafine ou morpholines, ou des échinocandines.

Selon un mode de réalisation particulier, la présente invention a pour objet un procédé de détermination du degré de sensibilité d'une population de cellules d'une souche de champignon vis-à-vis d'un antifongique, dans lequel ledit antifongique est choisi parmi le groupe dépourvu des polyènes tels que l'amphotéricine B et la nystatine et des antifongiques à action directe sur la chitine.

Selon un mode de réalisation particulier, la présente invention a pour objet un procédé de détermination du degré de sensibilité d'une population de cellules d'une souche de champignon vis-à-vis d'un antifongique, dans lequel ledit antifongique est un antifongique azolé choisi parmi le groupe comprenant du voriconazole, du posaconazole, de l'itraconazole et de l'isavuconazole.

Selon un mode de réalisation particulier, la présente invention a pour objet un procédé de détermination du degré de sensibilité d'une population de cellules d'une souche de champignon vis-à-vis d'un antifongique, dans lequel ledit antifongique est une échinocandine choisie parmi le groupe comprenant l'anidulafungine, de la caspofungine, de la micafungine.

Selon un mode de réalisation particulier, la présente invention a pour objet un procédé de détermination du degré de sensibilité d'une population de cellules d'une souche de champignon vis-à-vis d'un antifongique, dans lequel ledit antifongique est choisi parmi le groupe des antifongiques dépourvu des polyènes tels que l'amphotéricine B et la nystatine, et dépourvu des antifongiques à action directe sur la chitine, plus particulièrement :
- parmi le groupe comprenant ou étant constitué des antifongiques azolés tels que le fluconazole, le voriconazole, le posaconazole, l'itraconazole et l'isavuconazole, ou autres molécules inhibitrices de la synthèse de l'ergosterol tels que les allylamines dont la terbinafine ou morpholines ;
- ou parmi les échinocandines, l'échinocandine étant plus particulièrement choisie parmi le groupe comprenant ou étant constitué de l'anidulafungine, de la caspofungine et de la micafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est un champignon pluricellulaire.

Selon un mode de réalisation particulier, la présente invention a pour objet un procédé de détermination du degré de sensibilité d'une population de cellules d'une souche de champignon vis-à-vis d'un antifongique, dans lequel ledit champignon est choisi dans le groupe comprenant *Aspergillus, Fusarium, Scedosporium, Lichteimia, Rhizopus, Mucor, Paecylomyces, Rhizomucor, Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Aspergillus.*

Selon un mode de réalisation particulier, la présente invention a pour objet un procédé de détermination du degré de sensibilité d'une population de cellules d'une souche de champignon vis-à-vis d'un antifongique, dans lequel ledit champignon du genre *Aspergillus* est choisi dans le groupe comprenant *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Aspergillus,* choisi dans le groupe comprenant les espèces *A. fumigatus, A. flavus, A. nidulans, A. terreus, A. versicolor, A. niger* et dans lequel ledit antifongique est choisi dans le groupe voriconazole, posaconazole, itraconazole, isavuconazole, micafungine, anidulafungine, caspofungine, amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon du genre *Aspergillus,* est choisi dans le groupe comprenant les espèces *A. fumigatus, A. flavus, A. nidulans, A. terreus, A. versicolor, A. niger .*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon du genre *Aspergillus,* est choisi dans le groupe comprenant les espèces *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'itraconazole et dans lequel ledit champignon du genre *Aspergillus,* est choisi dans le groupe comprenant les espèces *A. fumigatus, A. flavus, A. nidulans, A. terreus, A. versicolor, A. niger*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon du genre *Aspergillus,* est choisi dans le groupe comprenant les espèces *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules de champignon vis-à-vis d'un antifongique, dans lequel ledit antifongique est la micafungine et dans lequel ledit champignon du genre *Aspergillus* est choisi dans le groupe comprenant les espèces A. *fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'anidulafungine et dans lequel ledit champignon du genre *Aspergillus* est choisi dans le groupe comprenant les espèces *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est la caspofungine et dans lequel ledit champignon du genre *Aspergillus,* est choisi dans le groupe comprenant les espèces *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon du genre *Aspergillus,* est choisi dans le groupe comprenant les espèces *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est la nystatine et dans lequel ledit champignon du genre *Aspergillus,* est choisi dans le groupe comprenant les espèces *A. fumigatus, A. flavus, A. nidulans, A. terreus, A. versicolor, A. niger.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce A. *fumigatus* et dans lequel ledit antifongique est choisi dans le groupe voriconazole, posaconazole, itraconazole, isavuconazole, micafungine, anidulafungine, caspofungine, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce A. *flavus* et dans lequel ledit antifongique est choisi dans le groupe voriconazole, posaconazole, itraconazole, isavuconazole, micafungine, anidulafungine, caspofungine, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce A. *nidulans* et dans lequel ledit antifongique est choisi dans le groupe voriconazole, posaconazole, itraconazole, isavuconazole, micafungine, anidulafungine, caspofungine, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce A. *terreus* et dans lequel ledit antifongique est choisi dans le groupe voriconazole, posaconazole, itraconazole, isavuconazole, micafungine, anidulafungine, caspofungine, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce A. *versicolor* et dans lequel ledit antifongique est choisi dans le groupe voriconazole, posaconazole, itraconazole, isavuconazole, micafungine, anidulafungine, caspofungine, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce A. *niger* et dans lequel ledit antifongique est choisi dans le groupe voriconazole, posaconazole, itraconazole, isavuconazole, micafungine, anidulafungine, caspofungine, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Fusarium.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Fusarium,* choisi dans le groupe comprenant les espèces : *Fusarium solani, F. oxysporum.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Fusarium,* choisi dans le groupe comprenant les espèces *Fusarium solani, F. oxysporum* et dans lequel ledit antifongique est choisi dans le groupe voriconazole, isavuconazole, amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Fusarium,* choisi dans le groupe comprenant les espèces *Fusarium solani, F. oxysporum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon est du genre *Fusarium,* choisi dans le groupe comprenant les espèces *Fusarium solani, F. oxysporum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Fusarium,* choisi dans le groupe comprenant les espèces *Fusarium solani, F. oxysporum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est la nystatine et dans lequel ledit champignon est du genre *Fusarium,* choisi dans le groupe comprenant les espèces *Fusarium solani, F. oxysporum.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce F. *solani* et dans lequel ledit antifongique est choisi dans le groupe, voriconazole, isavuconazole, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce F. *oxysporum* et dans lequel ledit antifongique est choisi dans le groupe, voriconazole, isavuconazole, l'amphotéricine B et la nystatine.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Scedosporium.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Scedosporium,* choisi dans le groupe comprenant les espèces : *Scedosporium apiospermum, S. prolificans.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Scedosporium,* choisi dans le groupe comprenant les espèces : *Scedosporium apiospermum, S. prolificans* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Scedosporium,* choisi dans le groupe comprenant les espèces : *Scedosporium apiospermum, S. prolificans.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Scedosporium,* choisi dans le groupe comprenant les espèces : *Scedosporium apiospermum, S. prolificans.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon est du genre *Scedosporium,* choisi dans le groupe comprenant les espèces : *Scedosporium apiospermum, S. prolificans.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Scedosporium,* choisi dans le groupe comprenant les espèces : *Scedosporium apiospermum, S. prolificans.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce S. *apiospermum* et dans lequel ledit antifongique est choisi dans le groupe voriconazole, posaconazole, isavuconazole, l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce S. *prolificans* et dans lequel ledit antifongique est choisi dans le groupe voriconazole, posaconazole, isavuconazole, l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Mucor.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Mucor,* choisi dans le groupe comprenant l'espèce *Mucor racemosa.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Mucor,* choisi dans le groupe comprenant l'espèce *Mucor racemosa* et dans lequel ledit antifongique est choisi dans le groupe voriconazole, posaconazole, isavuconazole et l' amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Mucor,* choisi dans le groupe comprenant l'espèce *Mucor racemosa.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Mucor,* choisi dans le groupe comprenant l'espèce *Mucor racemosa.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole, et dans lequel ledit champignon est du genre *Mucor,* choisi dans le groupe comprenant l'espèce *Mucor racemosa.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Mucor,* choisi dans le groupe comprenant l'espèce *Mucor racemosa.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce M. *racemosa* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Lichteimia.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Lichteimia,* choisi dans le groupe comprenant l'espèce *Lichteimia corymbifera.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Lichteimia,* choisi dans le groupe comprenant l'espèce *Lichteimia corymbifera* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Lichteimia,* choisi dans le groupe comprenant l'espèce *Lichteimia corymbifera.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Lichteimia,* choisi dans le groupe comprenant l'espèce *Lichteimia corymbifera.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon est du genre *Lichteimia,* choisi dans le groupe comprenant l'espèce *Lichteimia corymbifera.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Lichteimia,* choisi dans le groupe comprenant l'espèce *Lichteimia corymbifera.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce L. *corymbifera* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Rhizopus.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Rhizopus,* choisi dans le groupe comprenant l'espèce *Rhizopus oryzae.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Rhizopus,* choisi dans le groupe comprenant l'espèce *Rhizopus oryzae* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Rhizopus,* choisi dans le groupe comprenant l'espèce *Rhizopus oryzae.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Rhizopus,* choisi dans le groupe comprenant l'espèce *Rhizopus oryzae.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon est du genre *Rhizopus,* choisi dans le groupe comprenant l'espèce *Rhizopus oryzae.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Rhizopus,* choisi dans le groupe comprenant l'espèce *Rhizopus oryzae.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce R. *oryzae* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Rhizomucor.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Rhizomucor,* choisi dans le groupe comprenant l'espèce *Rhizomucorpusillus.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Rhizomucor,* choisi dans le groupe comprenant l'espèce *Rhizomucor pusillus* et dans lequel ledit antifongique est choisi dans le groupe voriconazole, posaconazole, isavuconazole, et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Rhizomucor,* choisi dans le groupe comprenant l'espèce *Rhizomucor pusillus.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Rhizomucor,* choisi dans le groupe comprenant l'espèce *Rhizomucor pusillus.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole, et dans lequel ledit champignon est du genre *Rhizomucor,* choisi dans le groupe comprenant l'espèce *Rhizomucor pusillus.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Rhizomucor,* choisi dans le groupe comprenant l'espèce *Rhizomucor pusillus.*

Selon un mode de réalisation particulier, la présente invention a pour objet un procédé de détermination du degré de sensibilité d'une population de cellules d'une souche de champignon vis-à-vis d'un antifongique, dans lequel ledit champignon est de l'espèce R. *pusillis* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Geotrichum,* et dans lequel ledit antifongique est choisi dans le groupe fluconazole, voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le fluconazole et dans lequel ledit champignon est du genre *Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon est du genre *Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Geotrichum.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Paecylomyces.*

Selon un mode de réalisation particulier, la présente invention a pour objet un procédé de détermination du degré de sensibilité d'une population de cellules d'une souche de champignon vis-à-vis d'un antifongique, dans lequel ledit champignon est du genre *Paecylomyces,* et dans lequel ledit antifongique est choisi dans le groupe voriconazole, posaconazole, isavuconazole et l'amphotéricine B.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est du genre *Paecylomyces.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est du genre *Paecylomyces.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'isavuconazole et dans lequel ledit champignon est du genre *Paecylomyces.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'amphotéricine B et dans lequel ledit champignon est du genre *Paecylomyces.*

Selon un mode de réalisation particulier, la présente invention a pour objet un procédé de détermination du degré de sensibilité d'une population de cellules d'une souche de champignon vis-à-vis d'un antifongique, dans lequel ledit champignon est un champignon unicellulaire.

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure.

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure choisie dans le groupe *Candida, Cryptococcus, Saccharomyces, Trichosporon, Rhodotorula, Malassezia.*

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure du genre *Candida.*

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure du genre *Candida,* choisie dans le groupe comprenant les espèces : *Candida albicans,* C. *glabatra, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure du genre *Candida,* choisie dans le groupe comprenant les espèces *Candida albicans, C. glabatra, C. tropicalis, C. parapsilosis, C. krusei* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une souche de levure du genre *Candida,* choisie dans le groupe comprenant les souches SC5314, DSY296, TOP de *Candida albicans,* ATCC^{®}2001, Tg5 de C. *glabatra,* ATCC^{®}7349, 13/5 de C. *tropicalis,* ATCC^{®}22019, 8/21 de *C. parapsilosis,* ATCC^{®}6258, *GRE32* de C. *krusei,* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure de l'espèce *Candida albicans* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une souche de levure de l'espèce *Candida albicans* choisie dans le groupe SC5314, DSY296,TOP, et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure de l'espèce *Candida glabrata* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une souche de levure de l'espèce *Candida glabrata* choisie dans le groupe ATCC^{®}2001, Tg5, et dans lequel ledit antifongique est choisi dans le groupe fluconazole, voriconazole, posaconazole, micafungine, anidulafungine

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure de l'espèce *Candida tropicalis* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une souche de levure de l'espèce *Candida tropicalis* choisie dans le groupe ATTCC^{®}7349, 13/5, et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure de l'espèce *Candida parapsilosis* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une souche de levure de l'espèce *Candida parapsilosis* choisie dans le groupe ATCC^{®}22019, 8/21, et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une levure de l'espèce *Candida krusei* et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit champignon est une souche de levure de l'espèce *Candida krusei* choisie dans le groupe ATCC^{®}6258, GRE32 et dans lequel ledit antifongique est choisi dans le groupe fluconazole, voriconazole, posaconazole, micafungine, anidulafungine.

Selon un mode de réalisation particulier, la présente invention a pour objet un procédé de détermination du degré de sensibilité d'une population de cellules d'une souche de champignon vis-à-vis d'un antifongique, dans lequel ledit antifongique est le fluconazole et dans lequel ledit champignon est une levure du genre *Candida,* choisie dans le groupe comprenant les espèces *Candida albicans, C. glabatra, C. tropicalis, C. parapsilosis, C. krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le fluconazole et dans lequel ledit champignon est une souche de levure du genre *Candida,* choisie dans le groupe comprenant les souches SC5314, DSY296 TOP de *Candida albicans,* ATCC^{®}2001, Tg5 de C. *glabatra,* ATCC^{®}7349, 13/5 de C. *tropicalis,* ATCC^{®}22019, 8/21 de C. *parapsilosis,* ATCC^{®}6258, GRE32 de C. *krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est une levure du genre *Candida,* choisie dans le groupe comprenant les espèces *Candida albicans, C. glabatra, C. tropicalis, C. parapsilosis, C. krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le voriconazole et dans lequel ledit champignon est une souche de levure du genre *Candida,* choisie dans le groupe comprenant les souches SC5314, DSY296, TOP de *Candida albicans,* ATCC^{®}2001, Tg5 de *Candida glabatra,* ATCC^{®}7349, 13/5 de *C*. *tropicalis,* ATCC^{®}22019, 8/21 de C. *parapsilosis,* ATCC^{®}6258, GRE32 de C. *krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est une levure du genre *Candida,* choisie dans le groupe comprenant les espèces *Candida albicans, C. glabatra, C. tropicalis, C. parapsilosis, C. krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est le posaconazole et dans lequel ledit champignon est une souche de levure du genre *Candida,* choisie dans le groupe comprenant les souches SC5314, DSY296, TOP de *Candida albicans,* ATCC^{®}2001, Tg5 de *C*. *glabatra,* ATCC^{®}7349, 13/5 de *C*. *tropicalis,* ATCC^{®}22019, 8/21 de C. *parapsilosis,* ATCC^{®}6258, GRE32 de C. *krusei.*

Selon un mode de réalisation particulier, la présente invention a pour objet un procédé de détermination du degré de sensibilité d'une population de cellules de champignon vis-à-vis d'un antifongique, dans lequel ledit antifongique est la micafungine et dans lequel ledit champignon est une levure du genre *Candida,* choisie dans le groupe comprenant les espèces *Candida albicans, C. glabatra, C. tropicalis, C. parapsilosis, C. krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est la micafungine et dans lequel ledit champignon est une souche de levure du genre *Candida,* choisie dans le groupe comprenant les souches SC5314, DSY296, TOP de *Candida albicans,* ATCC^{®}2001, Tg5 de *C*. *glabatra,* ATCC^{®}7349, 13/5 de *C*. *tropicalis,* ATCC^{®}22019, 8/21 de C. *parapsilosis,* ATCC^{®}6258, GRE32 de C. *krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'anidulafungine et dans lequel ledit champignon est une levure du genre *Candida,* choisie dans le groupe comprenant les espèces *Candida albicans, C. glabatra, C. tropicalis, C. parapsilosis, C. krusei.*

Selon un mode de réalisation particulier, la présente invention a pour objet un procédé de détermination du degré de sensibilité d'une population de cellules d'une souche de champignon vis-à-vis d'un antifongique, dans lequel ledit antifongique est l'anidulafungine et dans lequel ledit champignon est une souche de levure du genre *Candida,* choisie dans le groupe comprenant les souches SC5314, DSY296, TOP de *Candida albicans,* ATCC^{®}2001, Tg5 de C. *glabatra,* ATCC^{®}7349, 13/5 de C. *tropicalis,* ATCC^{®}22019, 8/21 de C. *parapsilosis,* ATCC^{®}6258,GRE32 de C. *krusei.*

Selon un mode de réalisation particulier, la présente invention a pour objet un procédé de détermination du degré de sensibilité d'une population de cellules d'une souche de champignon vis-à-vis d'un antifongique comprenant une étape de mise en présence dudit antifongique et des cellules de ladite souche de champignon, avant l'étape de détermination de la variation éventuelle du taux de chitine.

Selon un mode de réalisation particulier, la présente invention a pour objet un procédé de détermination du degré de sensibilité d'une population de cellules d'une souche de champignon vis-à-vis d'un antifongique, dans lequel ladite étape de mise enprésence est d'une durée inférieure ou égale à 48h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est d'une durée inférieure ou égale à 24h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est d'au moins 6,5h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est d'une durée de 6,5h à 24h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est d'une durée de 6,5h à 24h, notamment de 6,5h à 8h, de 6,5h à 10h, de 6,5h à 12h, de 6,5h à14h, de 6,5h à 16h, de 6,5h à18h, de 6,5h à 20h, de 6,5h à 22h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est d'une durée de 6,5h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est d'une durée de 24h.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est effectuée à une température de 30 à 35°C.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est effectuée à une température de 30 à 35°C, notamment de 30°C à 31°C, de 30°C à 32°C, de 30°C à 33°C, de 30°C à 34°C.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est effectuée à une température de 30°C.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence est effectuée à une température de 35°C.

Selon un mode de réalisation particulier, la présente invention a pour objet un procédé de détermination du degré de sensibilité d'une population de cellules d'une souche de champignon vis-à-vis d'un antifongique, comprenant une étape de marquage des cellules de ladite souche de champignon par un marqueur fluorescent.

Selon un mode de réalisation particulier, la présente invention a pour objet un procédé de détermination du degré de sensibilité d'une population de cellules d'une souche de champignon vis-à-vis d'un antifongique, comprenant une étape de marquage des cellules de ladite souche de champignon par un marqueur fluorescent, ladite étape ayant lieu avant, pendant ou après l'étape de mise en présence dudit antifongique et des cellules de ladite souche de champignon.

Selon un mode de réalisation particulier, la présente invention a pour objet un procédé de détermination du degré de sensibilité d'une population de cellules d'une souche de champignon vis-à-vis d'un antifongique comprenant une étape de marquage des cellules de ladite souche de champignon par un marqueur fluorescent, ladite étape ayant lieu avant l'étape de mise en présence dudit antifongique et des cellules de ladite souche de champignon.

Selon un mode de réalisation particulier, la présente invention a pour objet un procédé de détermination du degré de sensibilité d'une population de cellules d'une souche de champignon vis-à-vis d'un antifongique, comprenant une étape de marquage des cellules de ladite souche de champignon par un marqueur fluorescent, ladite étape ayant lieu simultanément à l'étape de mise en présence dudit antifongique et des cellules de ladite souche de champignon.

Selon un mode de réalisation particulier, la présente invention a pour objet un procédé de détermination du degré de sensibilité d'une population de cellules d'une souche de champignon vis-à-vis d'un antifongique comprenant une étape de marquage des cellules de ladite souche de champignon par un marqueur fluorescent, ladite étape ayant lieu après l'étape de mise en présence dudit antifongique et des cellules de ladite souche de champignon.

Selon un mode de réalisation particulier, la présente invention a pour objet un procédé de détermination du degré de sensibilité d'une population de cellules d'une souche de champignon vis-à-vis d'un antifongique, comprenant une étape de mise en présence dudit antifongique et des cellules de ladite souche de champignon et une étape de marquage des cellules de ladite souche de champignon par un marqueur fluorescent, ledit marqueur étant le Calcofluor White.

Selon un mode de réalisation particulier, la présente invention a pour objet un procédé de détermination du degré de sensibilité d'une population de cellules d'une souche de champignon vis-à-vis d'un antifongique, comprenant une étape de détermination de la variation éventuelle du taux de chitine par quantification du taux de chitine dans les cellules de ladite souche de champignon par microscopie à fluorescence.

Selon un mode de réalisation particulier, la présente invention a pour objet un procédé de détermination du degré de sensibilité correspondant à un phénotype sensible d'une population de cellules d'une souche de champignon, ledit champignon étant un champignon unicellulaire, vis-à-vis d'un antifongique, comprenant une étape de détermination de la variation du taux de chitine, ladite variation étant une augmentation du taux de chitine supérieure ou égale à 20% dans la population de cellules de ladite souche de champignon en présence dudit antifongique, par rapport au taux de chitine d'une population de cellules de ladite souche de champignon en absence d'antifongique, par quantification de la teneur en chitine par microscopie à fluorescence, suivi d'une étape de détermination de la variation éventuelle du nombre de cellules dans la population de cellules de ladite souche de champignon en présence dudit antifongique par rapport au nombre de cellules d'une population de cellules de ladite souche de champignon en absence d'antifongique.

Selon un mode de réalisation particulier, la présente invention a pour objet un procédé de détermination du degré de sensibilité correspondant à un phénotype intermédiaire d'une population de cellules d'une souche de champignon, ledit champignon étant un champignon unicellulaire, vis-à-vis d'un antifongique, comprenant une étape de détermination de la variation du taux de chitine, ladite variation étant une augmentation du taux de chitine supérieure ou égale à 20% dans la population de cellules de ladite souche de champignon en présence dudit antifongique par rapport au taux de chitine d'une population de cellules de ladite souche de champignon en absence d'antifongique, par quantification de la teneur en chitine par microscopie à fluorescence, suivi d'une étape de détermination de la variation éventuelle du nombre de cellules dans la population de cellules de ladite souche de champignon en présence dudit antifongique par rapport au nombre de cellules d'une population de cellules de ladite souche de champignon en absence d'antifongique.

Selon un mode de réalisation particulier, la présente invention a pour objet un procédé de détermination du degré de sensibilité correspondant à un phénotype sensible d'une population de cellules d'une souche de champignon, ledit champignon étant un champignon pluricellulaire, vis-à-vis d'un antifongique, comprenant une étape de détermination de la variation du taux de chitine, ladite variation étant une augmentation du taux de chitine supérieure ou égale à 20% dans la population de cellules de ladite souche de champignon en présence dudit antifongique par rapport au taux de chitine d'une population de cellules de ladite souche de champignon en absence d'antifongique, par quantification de la teneur en chitine par microscopie à fluorescence, suivi d'une étape de détermination de la variation éventuelle de la longueur de l'hyphe végétatif de germination dans ladite population de cellules de ladite souche de champignon en présence dudit antifongique, par rapport à la longueur de l'hyphe végétatif de germination dans une population de cellules de ladite souche de champignon en absence d'antifongique.

Selon un mode de réalisation particulier, la présente invention a pour objet un procédé de détermination du degré de sensibilité correspondant à un phénotype intermédiaire d'une population de cellules d'une souche de champignon, ledit champignon étant un champignon pluricellulaire, vis-à-vis d'un antifongique, comprenant une étape de détermination de la variation du taux de chitine, ladite variation étant une augmentation du taux de chitine supérieure ou égale à 20% dans la population de cellules de ladite souche de champignon en présence dudit antifongique par rapport au taux de chitine d'une population de cellules de ladite souche de champignon en absence d'antifongique, par quantification de la teneur en chitine par microscopie à fluorescence, suivi d'une étape de détermination de la variation éventuelle de la longueur de l'hyphe végétatif de germination dans ladite population de cellules de ladite souche de champignon en présence dudit antifongique, par rapport à la longueur de l'hyphe végétatif de germination dans une population de cellules de ladite souche de champignon en absence d'antifongique.

Selon un mode de réalisation particulier, la présente invention a pour objet un procédé de détermination du degré de sensibilité d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de la variation éventuelle du taux de chitine dans le mélange de cellules de ladite souche de champignon marquées au marqueur fluorescent et d'antifongique, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une absence d'augmentation, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de la variation éventuelle du taux de chitine dans le mélange de cellules de ladite souche de champignon marquées au marqueur fluorescent et d'antifongique, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon unicellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de la variation éventuelle du taux de chitine dans le mélange de cellules de ladite souche de champignon marquées au marqueur fluorescent et d'antifongique, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de dénombrement des cellules dans le mélange de cellules de ladite souche de champignon marquées au marqueur fluorescent et d'antifongique ; puis d'
**f.** une étape de détermination de la variation éventuelle du nombre de cellules dans la population de cellules de ladite souche de champignon en présence d'antifongique par rapport au nombre de cellules dans une population de cellules de ladite souche de champignon en absence d'antifongique ;

lorsque ladite variation éventuelle du taux de chitine de la susdite étape d. est une augmentation inférieure à 10% ou une absence d'augmentation, il est déduit que ladite souche de champignon est de phénotype résistant;
lorsque ladite variation du taux de chitine de la susdite étape d. est une augmentation de 10% à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle du nombre de cellules de la susdite étape f. est une diminution du nombre de cellules de moins de 0,3 log ou un nombre de cellules stationnaire, il est déduit que ladite souche de champignon est de phénotype intermédiaire ;
lorsque ladite variation du taux de chitine de la susdite étape d. est une augmentation d'une valeur supérieure ou égale à 20%, et que ladite variation du nombre de cellules de la susdite étape f. est une diminution d'au moins 0,3 log, il est déduit que ladite souche de champignon est de phénotype sensible.

Selon un mode de réalisation particulier, la présente invention a pour objet un procédé de détermination du degré de sensibilité d'une population de cellules d'une souche de champignon, ledit champignon étant un champignon unicellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de la variation éventuelle du taux de chitine dans le mélange de cellules de ladite souche de champignon marquées au marqueur fluorescent et d'antifongique, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de dénombrement des cellules dans le mélange de cellules de ladite souche de champignon marquées au marqueur fluorescent et d'antifongique ; puis d'
**f.** une étape de détermination de la variation éventuelle du nombre de cellules dans la population de cellules de ladite souche de champignon en présence d'antifongique par rapport au nombre de cellules dans une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle du nombre de cellules de la susdite étape f. est une diminution du nombre de cellules de moins de 0,3 log ou un nombre de cellules stationnaire, il est déduit que ladite souche de champignon est de phénotype intermédiaire ;
lorsque ladite variation du taux de chitine de la susdite étape d. est une augmentation d'une valeur supérieure ou égale à 20%, et que ladite variation du nombre de cellules de la susdite étape f. est une diminution d'au moins 0,3 log, il est déduit que ladite souche de champignon est de phénotype sensible.

Selon un mode de réalisation particulier, la présente invention a pour objet un procédé de détermination du degré de sensibilité d'une population de cellules d'une souche de champignon, ledit champignon étant un champignon pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de la variation éventuelle du taux de chitine dans le mélange de cellules de ladite souche de champignon marquées au marqueur fluorescent et d'antifongique, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au marqueur fluorescent et d'antifongique ; puis d'
**f.** une étape de détermination de la variation éventuelle de la longueur de l'hyphe végétatif de germination dans la population de cellules de ladite souche de champignon en présence d'antifongique par rapport à la longueur de l'hyphe végétatif de germination dans une population de cellules de ladite souche de champignon en absence d'antifongique ;

lorsque ladite variation éventuelle du taux de chitine de la susdite étape d. est une absence d'augmentation ou une augmentation de moins de 10%, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de la longueur dudit hyphe ou une longueur stationnaire dudit hyphe, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de la variation éventuelle du taux de chitine dans le mélange de cellules de ladite souche de champignon marquées au marqueur fluorescent et d'antifongique, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au marqueur fluorescent et d'antifongique ; puis d'
**f.** une étape de détermination de la variation éventuelle de la longueur de l'hyphe végétatif de germination dans la population de cellules de ladite souche de champignon en présence d'antifongique par rapport à la longueur de l'hyphe végétatif de germination dans une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de la longueur dudit hyphe ou une longueur stationnaire dudit hyphe, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon vis-à-vis d'un antifongique, dans lequel ladite étape de mise en présence des cellules de ladite souche de champignon avec une gamme de concentration dudit antifongique de 0,0009 à 130 µg/ml, s'effectue à une température de 30 à 35°C pendant une durée inférieure ou égale à 48h, notamment pendant une durée de 6,5h à 24h, pour obtenir un mélange de cellules de ladite souche de champignon avec ledit antifongique.

Selon un mode de réalisation particulier, la présente invention a pour objet un procédé de détermination du degré de sensibilité d'une population de cellules d'une souche de champignon vis-à-vis d'un antifongique, dans lequel l'étape d'addition du marqueur fluorescent au mélange de cellules de ladite souche de champignon et dudit antifongique, obtenu après l'étape de mise en présence, est effectuée à l'aide du Calcofluor White, pour obtenir un mélange de cellules de ladite souche de champignon marquées au Calcofluor White et dudit antifongique.

Selon un mode de réalisation particulier, la présente invention a pour objet un procédé de détermination du degré de sensibilité d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10% ou une absence d'augmentation, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon unicellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de dénombrement des cellules dans le mélange de cellules de ladite souche de champignon marquées au Calcofluor White et d'antifongique; puis d'
**f.** une étape de détermination de la variation éventuelle du nombre de cellules en fonction de la concentration en antifongique dans la population de cellules de ladite souche de champignon, par rapport au nombre de cellules dans une population de cellules de ladite souche de champignon en absence d'antifongique ;

lorsque ladite variation éventuelle du taux de chitine de la susdite étape d. est une augmentation inférieure à 10% ou une absence d'augmentation du taux de chitine, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle du nombre de cellules de la susdite étape f. est une diminution du nombre de cellules de moins de 0,3 log ou un nombre de cellules stationnaire, il est déduit que ladite souche de champignon est de phénotype intermédiaire ;
lorsque ladite variation du taux de chitine de la susdite étape d. est une augmentation d'une valeur supérieure ou égale à 20%, et que ladite variation du nombre de cellules de la susdite étape f. est une diminution d'au moins 0,3 log, il est déduit que ladite souche de champignon est de phénotype sensible.

Selon un mode de réalisation particulier, la présente invention a pour objet un procédé de détermination du degré de sensibilité d'une population de cellules d'une souche de champignon, ledit champignon étant un champignon unicellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de dénombrement des cellules dans le mélange de cellules de ladite souche de champignon marquées au Calcofluor White et d'antifongique; puis d'
**f.** une étape de détermination de la variation éventuelle du nombre de cellules en fonction de la concentration en antifongique dans la population de cellules de ladite souche de champignon, par rapport au nombre de cellules dans une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle du nombre de cellules de la susdite étape f. est une diminution du nombre de cellules de moins de 0,3 log ou un nombre de cellules stationnaire, il est déduit que ladite souche de champignon est de phénotype intermédiaire ;
lorsque ladite variation du taux de chitine de la susdite étape d. est une augmentation d'une valeur supérieure ou égale à 20%, et que ladite variation du nombre de cellules de la susdite étape f. est une diminution d'au moins 0,3 log, il est déduit que ladite souche de champignon est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une absence d'augmentation, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de la longueur dudit hyphe ou une longueur stationnaire dudit hyphe, il est déduit que ladite souche de champignon est de phénotype sensible ou intermédiaire.

Selon un mode de réalisation particulier, la présente invention a pour objet un procédé de détermination de la sensibilité ou de la résistance d'une population de cellules d'une souche de champignon, ledit champignon étant un champignon pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de la longueur dudit hyphe ou une longueur stationnaire dudit hyphe, il est déduit que ladite souche de champignon est de phénotype sensible ou intermédiaire.

Selon un mode de réalisation particulier, la présente invention a pour objet un procédé de détermination du degré de sensibilité correspondant à un phénotype résistant d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination d'une absence d'augmentation du taux de chitine ou d'une augmentation du taux de chitine inférieure à 10 % dans le mélange de cellules de ladite souche de champignon marquées au marqueur fluorescent et d'antifongique, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype résistant d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination d'une augmentation du taux de chitine inférieure à 10 % ou d'une diminution du taux de chitine notamment inférieure à 20% ou d'un taux de chitine stationnaire, dans le mélange de cellules de ladite souche de champignon marquées au marqueur fluorescent et d'antifongique, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype résistant d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination d'une absence d'augmentation du taux de chitine ou d'une augmentation du taux de chitine inférieure à 10 %, dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype résistant d'une population de cellules d'une souche de champignon, ledit champignon étant un champignon unicellulaire ou pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination d'une augmentation du taux de chitine inférieure à 10 % ou d'une diminution du taux de chitine notamment inférieure à 20% ou d'un taux de chitine stationnaire, dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique.

Selon un mode de réalisation particulier, la présente invention a pour objet un procédé de détermination du degré de sensibilité correspondant à un phénotype intermédiaire d'une population de cellules d'une souche de champignon, ledit champignon étant un champignon unicellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de l'augmentation du taux de chitine de 10 % à une valeur inférieure à 20% dans le mélange de cellules de ladite souche de champignon marquées au marqueur fluorescent et d'antifongique, par rapport au taux de chitine d'une population de cellules de ladite souche de champignon en absence d'antifongique ; ou
**e.** une étape de détermination de l'augmentation du taux de chitine supérieure ou égale 20% dans le mélange de cellules de ladite souche de champignon marquées au marqueur fluorescent et d'antifongique, par rapport au taux de chitine d'une population de cellules de ladite souche de champignon en absence d'antifongique, suivi d'
**f.** une étape de dénombrement des cellules dans le mélange de cellules de ladite souche de champignon marquées au marqueur fluorescent et d'antifongique ; puis d'
**g.** une étape de détermination d'une diminution du nombre de cellules de moins de 0,3 log ou d'un nombre de cellules stationnaire, en fonction des concentrations en antifongique dans la population de cellules de ladite souche de champignon, par rapport au nombre de cellules dans une population de cellules de ladite souche de champignon en absence d'antifongique.

Selon un mode de réalisation particulier, la présente invention a pour objet un procédé de détermination du degré de sensibilité correspondant à un phénotype intermédiaire d'une population de cellules d'une souche de champignon, ledit champignon étant un champignon pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de l'augmentation du taux de chitine entre 10% et une valeur inférieure à 20% dans le mélange de cellules de ladite souche de champignon marquées au marqueur fluorescent et d'antifongique, par rapport au taux de chitine d'une population de cellules de ladite souche de champignon en absence d'antifongique ; ou
**e.** une étape de détermination de l'augmentation du taux de chitine supérieure ou égale 20% dans le mélange de cellules de ladite souche de champignon marquées au marqueur fluorescent et d'antifongique, par rapport au taux de chitine d'une population de cellules de ladite souche de champignon en absence d'antifongique, suivi d'
**f.** une étape de mesure de la longueur de l'hyphe végétatif de germination dans le mélange de cellules de ladite souche de champignon marquées au marqueur fluorescent et d'antifongique ; puis d'
**g.** une étape d'une détermination de la diminution de la longueur de l'hyphe végétatif de germination ou d'une longueur stationnaire dudit hyphe, en fonction des concentrations en antifongique dans la population de cellules de ladite souche de champignon, par rapport à la longueur de l'hyphe végétatif de germination d'une population de cellules de ladite souche de champignon en absence d'antifongique.

Selon un mode de réalisation particulier, la présente invention a pour objet un procédé de détermination du degré de sensibilité correspondant à un phénotype intermédiaire d'une population de cellules d'une souche de champignon, ledit champignon étant un champignon unicellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de l'augmentation du taux de chitine de 10% à une valeur inférieure à 20% dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ; ou
**e.** une étape de détermination de l'augmentation du taux de chitine supérieure ou égale 20% dans le mélange de cellules de ladite souche de champignon marquées au marqueur fluorescent et d'antifongique, par rapport au taux de chitine d'une population de cellules de ladite souche de champignon en absence d'antifongique, suivi d'
**f.** une étape de dénombrement des cellules dans le mélange de cellules de ladite souche de champignon marquées au Calcofluor White et d'antifongique, après la susdite étape de quantification du taux de chitine ; puis d'
**g.** une étape de détermination d'une diminution du nombre de cellules de moins de 0,3 log ou d'un nombre de cellules stationnaire, en fonction de la concentration en antifongique dans la population de cellules de ladite souche de champignon, par rapport au nombre de cellules dans une population de cellules de ladite souche de champignon en absence d'antifongique.

Selon un mode de réalisation particulier, la présente invention a pour objet un procédé de détermination du degré de sensibilité correspondant à un phénotype intermédiaire d'une population de cellules d'une souche de champignon, ledit champignon étant un champignon pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de l'augmentation du taux de chitine entre 10% et une valeur inférieure à 20% dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ; ou
**e.** une étape de détermination de l'augmentation du taux de chitine supérieure ou égale 20% dans le mélange de cellules de ladite souche de champignon marquées au marqueur fluorescent et d'antifongique, par rapport au taux de chitine d'une population de cellules de ladite souche de champignon en absence d'antifongique, suivi d'
**f.** une étape de mesure de la longueur de l'hyphe végétatif de germination dans le mélange de cellules de ladite souche de champignon marquées au Calcofluor White et d'antifongique, puis d'
**g.** une étape de détermination d'une diminution de la longueur de l'hyphe végétatif de germination ou d'une longueur stationnaire dudit hyphe, en fonction de la concentration en antifongique dans la population de cellules de ladite souche de champignon, par rapport à la longueur de l'hyphe végétatif de germination d'une population de cellules de ladite souche de champignon en absence d'antifongique.

Selon un mode de réalisation particulier, la présente invention a pour objet un procédé de détermination du degré de sensibilité correspondant à un phénotype sensible d'une population de cellules d'une souche de champignon, ledit champignon étant un champignon unicellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de l'augmentation du taux de chitine d'une valeur supérieure ou égale à 20% dans le mélange de cellules de ladite souche de champignon marquées au marqueur fluorescent et d'antifongique, par rapport au taux de chitine d'une population de cellules de ladite souche de champignon en absence d'antifongique ; suivi d'
**e.** une étape de dénombrement des cellules dans le mélange de cellules de ladite souche de champignon marquées au marqueur fluorescent et d'antifongique ; puis d'
**f.** une étape de détermination d'une diminution du nombre de cellules d'au moins 0,3 log en fonction des concentrations en antifongique dans la population de cellules de ladite souche de champignon, par rapport au nombre de cellules dans une population de cellules de ladite souche de champignon en absence d'antifongique.

Selon un mode de réalisation particulier, la présente invention a pour objet un procédé de détermination du degré de sensibilité correspondant à un phénotype sensible d'une population de cellules d'une souche de champignon, ledit champignon étant un champignon pluricellulaire, vis-à-vis d'un antifongique, comprenant :
**a.** une étape de mise en présence des cellules de ladite souche de champignon avec une concentration d'antifongique variant de 0,0009 à 130 µg/ml, pour obtenir un mélange de cellules de ladite souche de champignon avec l'antifongique ;
**b.** une étape d'addition d'un marqueur fluorescent au mélange de cellules de ladite souche de champignon et d'antifongique obtenu précédemment, pour obtenir un mélange de cellules de ladite souche de champignon marquées au marqueur fluorescent et d'antifongique;
**c.** une étape de quantification du taux de chitine par microscopie à fluorescence des cellules de ladite souche de champignon marquées dans le mélange obtenu à l'étape précédente ;
**d.** une étape de détermination de l'augmentation du taux de chitine d'une valeur supérieure ou égale à 20% dans le mélange de cellules de ladite souche de champignon marquées au marqueur fluorescent et d'antifongique, par rapport au taux de chitine d'une population de cellules de ladite souche de champignon en absence d'antifongique ; suivi d'
**e.** une étape de mesure de la longueur de l'hyphe végétatif de germination dans le mélange de cellules de ladite souche de champignon marquées au marqueur fluorescent et d'antifongique, puis d'
**f.** une étape de détermination d'une diminution de la longueur de l'hyphe végétatif de germination ou d'une longueur stationnaire dudit hyphe, en fonction des concentrations en antifongique dans la population de cellules de ladite souche de champignon, par rapport à la longueur de l'hyphe végétatif de germination d'une population de cellules de ladite souche de champignon en absence d'antifongique.

Selon un mode de réalisation particulier, la présente invention a pour objet un procédé de détermination du degré de sensibilité correspondant à un phénotype sensible d'une population de cellules d'une souche de champignon, ledit champignon étant un champignon unicellulaire, vis-à-vis d'un antifongique, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de l'augmentation du taux de chitine d'au moins 20% dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ; suivi d'
**e.** une étape de dénombrement des cellules dans le mélange de cellules de ladite souche de champignon marquées au Calcofluor White et d'antifongique, puis d'
**f.** une étape de détermination de la diminution du nombre de cellules d'au moins 0,3 log en fonction de la concentration en antifongique dans la population de cellules de ladite souche de champignon, par rapport au nombre de cellules dans une population de cellules de ladite souche de champignon en absence d'antifongique.

Selon un mode de réalisation particulier, la présente invention a pour objet un procédé de détermination du degré de sensibilité correspondant à un phénotype sensible d'une population de cellules d'une souche de champignon, ledit champignon étant un champignon pluricellulaire, vis-à-vis d'un antifongique, comprenant :
**a.** une étape de mise en présence des cellules de ladite souche de champignon avec un gradient de concentrations d'antifongique variant de 0,0009 à 130 µg/ml, à une température de 30 à 35°C pendant une durée inférieure ou égale à 48h, notamment pendant une durée de 6,5h à 24h, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de l'augmentation du taux de chitine d'une valeur supérieure ou égale à 20% dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ; suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination d'une diminution de la longueur de l'hyphe végétatif de germination ou d'une longueur stationnaire dudit hyphe, en fonction de la concentration en antifongique dans la population de cellules de ladite souche de champignon, par rapport à la longueur de l'hyphe végétatif de germination dans une population de cellules de ladite souche de champignon en absence d'antifongique.

Selon un mode de réalisation particulier, la présente invention a pour objet un procédé de détermination du degré de sensibilité correspondant à un phénotype sensible, d'une population de cellules de la souche SC5314 de *C*. *albicans* vis-à-vis du fluconazole, comprenant :
**a.** une étape de mise en présence des cellules de ladite souche SC5314 de *C*. *albicans* avec un gradient de concentrations de fluconazole variant de 0,015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 6,5h, à une température de 30°C; pour obtenir un mélange de cellules de ladite souche SC5314 de *C*. *albicans* avec le fluconazole ;
**b.** une étape d'addition du marqueur fluorescent Calcofluor White au mélange de cellules de ladite souche SC5314 de *C*. *albicans* et de fluconazole obtenu précédemment, pour obtenir un mélange de cellules de ladite souche SC5314 de *C*. *albicans* marquées au marqueur fluorescent Calcofluor White, et de fluconazole ;
**c.** une étape de quantification du taux de chitine par microscopie à fluorescence automatisée à haut contenu d'images, des cellules de ladite souche SC5314 de *C. albicans* marquées dans le mélange obtenu à l'étape précédente;
**d.** une étape de détermination de l'augmentation du taux de chitine d'une valeur supérieure ou égale à 20% dans le mélange de cellules de ladite souche de champignon marquées au marqueur fluorescent Calcofluor White et de fluconazole, en fonction de la concentration en fluconazole dans la population de cellules de ladite souche SC5314 de C. *albicans,* par rapport au taux de chitine d'une population de cellules de ladite souche SC5314 de C. *albicans* en absence de fluconazole ;
**e.** une étape de dénombrement des cellules dans le mélange de cellules ladite souche SC5314 de C. *albicans* marquées au marqueur fluorescent Calcofluor White et de fluconazole, après la susdite étape de quantification du taux de chitine ;
**f.** une étape de détermination de la diminution du nombre de cellules d'au moins 0,3 log en fonction de la concentration en fluconazole dans la population de cellules de ladite souche SC5314 de C. *albicans* par rapport au nombre de cellules dans une population de cellules de ladite souche SC5314 de C. *albicans* en absence de fluconazole.

Selon un mode de réalisation particulier, la présente invention a pour objet un procédé de détermination du degré de sensibilité correspondant à un phénotype sensible, d'une population de cellules de la souche SC5314 de C. *albicans* vis-à-vis du voriconazole, comprenant :
**a.** une étape de mise en présence des cellules de ladite souche SC5314 de C. *albicans* avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche SC5314 de C. *albicans* avec le voriconazole ;
**b.** une étape d'addition du marqueur fluorescent Calcofluor White au mélange de cellules de ladite souche SC5314 de C. *albicans* et de voriconazole obtenu précédemment, pour obtenir un mélange de cellules de ladite souche SC5314 de C. *albicans* marquées au marqueur fluorescent Calcofluor White, et de voriconazole;
**c.** une étape de quantification du taux de chitine par microscopie à fluorescence automatisée à haut contenu d'images, des cellules de ladite souche SC5314 de *C. albicans* marquées dans le mélange obtenu à l'étape précédente;
**d.** une étape de détermination de l'augmentation du taux de chitine d'une valeur supérieure ou égale à 20% dans le mélange de cellules de ladite souche SC5314 de C. *albicans* marquées au marqueur fluorescent Calcofluor White et de voriconazole, en fonction de la concentration en voriconazole dans la population de cellules de ladite souche SC5314 de C. *albicans,* par rapport au taux de chitine d'une population de cellules de ladite souche SC5314 de C. *albicans* en absence de voriconazole ; suivi d'
**e.** une étape de dénombrement des cellules dans le mélange de cellules de ladite souche SC5314 de C. *albicans* marquées au marqueur fluorescent Calcofluor White et de voriconazole, puis d'
**f.** une étape de détermination de la diminution du nombre de cellules d'au moins 0,3 log en fonction de la concentration en voriconazole dans la population de cellules de ladite souche SC5314 de C. *albicans* par rapport au nombre de cellules dans une population de cellules de ladite souche SC5314 de C. *albicans* en absence de voriconazole.

Selon un mode de réalisation particulier, la présente invention a pour objet un procédé de détermination du degré de sensibilité correspondant à un phénotype sensible, d'une population de cellules de la souche SC5314 de *C*. *albicans* vis-à-vis de la micafungine, comprenant :
**a.** une étape de mise en présence des cellules de ladite souche SC5314 de C. *albicans* avec un gradient de concentrations en micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche SC5314 de C. *albicans* avec la micafungine ;
**b.** une étape d'addition du marqueur fluorescent Calcofluor White au mélange de cellules de ladite souche SC5314 de C. *albicans* et de micafungine obtenu précédemment, pour obtenir un mélange de cellules de ladite souche SC5314 de C. *albicans* marquées au marqueur fluorescent Calcofluor White, et de micafungine;
**c.** une étape de quantification du taux de chitine par microscopie à fluorescence automatisée à haut contenu d'images, des cellules de ladite souche SC5314 de *C. albicans* marquées dans le mélange obtenu à l'étape précédente;
**d.** une étape de détermination de l'augmentation du taux de chitine d'une valeur supérieure ou égale à 20% dans le mélange de cellules de ladite souche SC5314 de C. *albicans* marquées au marqueur fluorescent Calcofluor White et de micafungine, en fonction de la concentration en micafungine dans la population de cellules de ladite souche SC5314 de C. *albicans,* par rapport au taux de chitine d'une population de cellules de ladite souche SC5314 de C. *albicans* en absence de micafungine ; suivi d'
**e.** une étape de dénombrement des cellules dans le mélange de cellules de ladite souche SC5314 de C. *albicans* marquées au marqueur fluorescent Calcofluor White et de micafungine ; puis d'
**f.** une étape de détermination de la diminution du nombre de cellules d'au moins 0,3 log en fonction de la concentration en micafungine dans la population de cellules de ladite souche SC5314 de *C*. *albicans* par rapport au nombre de cellules dans une population de cellules de ladite souche SC5314 de *C*. *albicans* en absence de micafungine.

Selon un mode de réalisation particulier, la présente invention a pour objet un procédé de détermination du degré de sensibilité correspondant à un phénotype résistant, d'une population de cellules de la souche DSY296 de *C*. *albicans* vis-à-vis du fluconazole, comprenant :
**a.** une étape de mise en présence des cellules de ladite souche DSY296 de *C*. *albicans* avec un gradient de concentrations de fluconazole variant de 0,015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 6,5h, à une température de 30°C; pour obtenir un mélange de cellules de ladite souche DSY296 de *C*. *albicans* avec le fluconazole ;
**b.** une étape d'addition du marqueur fluorescent Calcofluor White au mélange de cellules de ladite souche DSY296 de C. *albicans* et de fluconazole obtenu précédemment, pour obtenir un mélange de cellules de ladite souche DSY296 de *C*. *albicans* marquées au marqueur fluorescent Calcofluor White, et de fluconazole ;
**c.** une étape de quantification du taux de chitine par microscopie à fluorescence automatisée à haut contenu d'images, des cellules de ladite souche DSY296 de *C. albicans* marquées dans le mélange obtenu à l'étape précédente;
**d.** une étape de détermination de l'absence d'augmentation du taux de chitine ou de l'augmentation du taux de chitine inférieure à 10 % dans le mélange de cellules de ladite souche de champignon marquées au marqueur fluorescent Calcofluor White et de fluconazole, en fonction de la concentration en fluconazole dans la population de cellules de ladite souche DSY296 de *C*. *albicans,* par rapport au taux de chitine d'une population de cellules de ladite souche DSY296 de *C*. *albicans* en absence de fluconazole.

Selon un mode de réalisation particulier, la présente invention a pour objet un procédé de détermination du degré de sensibilité correspondant à un phénotype résistant, d'une population de cellules de la souche DSY296 de *C*. *albicans* vis-à-vis du fluconazole, comprenant :
**a.** une étape de mise en présence des cellules de ladite souche DSY296 de *C*. *albicans* avec un gradient de concentrations de fluconazole variant de 0.015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 6,5h, à une température de 30°C; pour obtenir un mélange de cellules de ladite souche DSY296 de *C*. *albicans* avec le fluconazole ;
**b.** une étape d'addition du marqueur fluorescent Calcofluor White au mélange de cellules de ladite souche DSY296 de *C*. *albicans* et de fluconazole obtenu précédemment, pour obtenir un mélange de cellules de ladite souche DSY296 de *C*. *albicans* marquées au marqueur fluorescent Calcofluor White, et de fluconazole ;
**c.** une étape de quantification du taux de chitine par microscopie à fluorescence automatisée à haut contenu d'images, des cellules de ladite souche DSY296 de *C. albicans* marquées dans le mélange obtenu à l'étape précédente;
**d.** une étape de détermination d'une augmentation du taux de chitine inférieure à 10 % ou d'une diminution du taux de chitine notamment inférieure à 20% ou d'un taux de chitine stationnaire, dans le mélange de cellules de ladite souche de champignon marquées au marqueur fluorescent Calcofluor White et de fluconazole, en fonction de la concentration en fluconazole dans la population de cellules de ladite souche DSY296 de *C*. *albicans,* par rapport au taux de chitine d'une population de cellules de ladite souche DSY296 de *C*. *albicans* en absence de fluconazole.

Selon un mode de réalisation particulier, la présente invention a pour objet un procédé de détermination du degré de sensibilité correspondant à un phénotype résistant, d'une population de cellules de la souche DSY296 de *C*. *albicans* vis-à-vis du voriconazole, comprenant :
**a.** une étape de mise en présence des cellules de ladite souche DSY296 de *C*. *albicans* avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche DSY296 de *C*. *albicans* avec le voriconazole ;
**b.** une étape d'addition du marqueur fluorescent Calcofluor White au mélange de cellules de ladite souche DSY296 de *C*. *albicans* et de voriconazole obtenu précédemment, pour obtenir un mélange de cellules de ladite souche DSY296 de *C*. *albicans* marquées au marqueur fluorescent Calcofluor White, et de voriconazole;
**c.** une étape de quantification du taux de chitine par microscopie à fluorescence automatisée à haut contenu d'images, des cellules de ladite souche DSY296 de *C. albicans* marquées dans le mélange obtenu à l'étape précédente;
**d.** une étape de détermination de l'absence d'augmentation du taux de chitine ou de l'augmentation du taux de chitine inférieure à 10 % dans le mélange de cellules de ladite souche DSY296 de *C*. *albicans* marquées au marqueur fluorescent Calcofluor White et de voriconazole, en fonction de la concentration en voriconazole dans la population de cellules de ladite souche DSY296 de *C*. *albicans,* par rapport au taux de chitine d'une population de cellules de ladite souche DSY296 de *C*. *albicans* en absence de voriconazole.

Selon un mode de réalisation particulier, la présente invention a pour objet un procédé de détermination du degré de sensibilité correspondant à un phénotype résistant, d'une population de cellules de la souche DSY296 de *C*. *albicans* vis-à-vis du voriconazole, comprenant :
**a.** une étape de mise en présence des cellules de ladite souche DSY296 de *C*. *albicans* avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche DSY296 de *C*. *albicans* avec le voriconazole ;
**b.** une étape d'addition du marqueur fluorescent Calcofluor White au mélange de cellules de ladite souche DSY296 de *C*. *albicans* et de voriconazole obtenu précédemment, pour obtenir un mélange de cellules de ladite souche DSY296 de *C*. *albicans* marquées au marqueur fluorescent Calcofluor White, et de voriconazole;
**c.** une étape de quantification du taux de chitine par microscopie à fluorescence automatisée à haut contenu d'images, des cellules de ladite souche DSY296 de *C. albicans* marquées dans le mélange obtenu à l'étape précédente;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche DSY296 de C. *albicans* marquées au marqueur fluorescent Calcofluor White et de voriconazole, en fonction de la concentration en voriconazole dans la population de cellules de ladite souche DSY296 de C. *albicans,* par rapport au taux de chitine d'une population de cellules de ladite souche DSY296 de C. *albicans* en absence de voriconazole.

Selon un mode de réalisation particulier, la présente invention a pour objet un procédé de détermination du degré de sensibilité correspondant à un phénotype résistant, d'une population de cellules de la souche TOP de *C*. *albicans* vis-à-vis de la micafungine, comprenant :
**a.** une étape de mise en présence des cellules de ladite souche TOP de *C*. *albicans* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche TOP de *C*. *albicans* avec la micafungine ;
**b.** une étape d'addition du marqueur fluorescent Calcofluor White au mélange de cellules de ladite souche TOP de *C*. *albicans* et de micafungine obtenu précédemment, pour obtenir un mélange de cellules de ladite souche TOP de *C. albicans* marquées au marqueur fluorescent Calcofluor White, et de micafungine;
**c.** une étape de quantification du taux de chitine par microscopie à fluorescence automatisée à haut contenu d'images, des cellules de ladite souche TOP de *C*. *albicans* marquées dans le mélange obtenu à l'étape précédente;
**d.** une étape de détermination de l'absence d'augmentation du taux de chitine ou de l'augmentation du taux de chitine inférieure à 10 % dans le mélange de cellules de ladite souche TOP de *C*. *albicans* marquées au marqueur fluorescent Calcofluor White et de micafungine, en fonction de la concentration en micafungine dans la population de cellules de ladite souche TOP de *C*. *albicans,* par rapport au taux de chitine d'une population de cellules de ladite souche TOP de *C*. *albicans* en absence de micafungine.

Selon un mode de réalisation particulier, la présente invention a pour objet un procédé de détermination du degré de sensibilité correspondant à un phénotype résistant, d'une population de cellules de la souche TOP de *C*. *albicans* vis-à-vis de la micafungine, comprenant :
**a.** une étape de mise en présence des cellules de ladite souche TOP de *C*. *albicans* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche TOP de *C*. *albicans* avec la micafungine ;
**b.** une étape d'addition du marqueur fluorescent Calcofluor White au mélange de cellules de ladite souche TOP de *C*. *albicans* et de micafungine obtenu précédemment, pour obtenir un mélange de cellules de ladite souche TOP de *C. albicans* marquées au marqueur fluorescent Calcofluor White, et de micafungine;
**c.** une étape de quantification du taux de chitine par microscopie à fluorescence automatisée à haut contenu d'images, des cellules de ladite souche TOP de *C*. *albicans* marquées dans le mélange obtenu à l'étape précédente;
**d.** une étape d'une augmentation du taux de chitine inférieure à 10% ou d'une diminution du taux de chitine notamment inférieure à 20% ou d'un taux de chitine stationnaire, dans le mélange de cellules de ladite souche TOP de *C*. *albicans* marquées au marqueur fluorescent Calcofluor White et de micafungine, en fonction de la concentration en micafungine dans la population de cellules de ladite souche TOP de *C*. *albicans,* par rapport au taux de chitine d'une population de cellules de ladite souche TOP de *C*. *albicans* en absence de micafungine.

Selon un mode de réalisation particulier, la présente invention a pour objet un procédé de détermination du degré de sensibilité correspondant à un phénotype intermédiaire, d'une population de cellules de la souche ATCC^{®}2001 de *C*. *glabrata* vis-à-vis du fluconazole, comprenant :
**a.** une étape de mise en présence des cellules de ladite souche ATCC^{®}2001 de *C. glabrata* avec un gradient de concentrations de fluconazole variant de 0,125 à 128 µg/ml, plus particulièrement de 0,125 à 0,250µg/ml, de 0,125 à 0,5 µg/ml, de 0,125 à 1 µg/ml, de 0,125 à 2 µg/ml, de 0,125 à 4 µg/ml, de 0,125 à 8 µg/ml, de 0,125 à 16 µg/ml, de 0,125 à 32 µg/ml, de 0,125 à 64 µg/ml, de 0,125 à 128 µg/ml, pendant une durée de 6,5h, à une température de 35°C; pour obtenir un mélange de cellules de ladite souche ATCC^{®}2001 de *C*. *glabrata* avec le fluconazole ;
**b.** une étape d'addition du marqueur fluorescent Calcofluor White au mélange de cellules de ladite souche ATCC^{®}2001 de C. *glabrata* et de fluconazole obtenu précédemment, pour obtenir un mélange de cellules de ladite souche ATCC^{®}2001 de *C*. *glabrata* marquées au marqueur fluorescent Calcofluor White, et de fluconazole;
**c.** une étape de quantification du taux de chitine par microscopie à fluorescence automatisée à haut contenu d'images, des cellules de ladite souche ATCC^{®}2001 de *C*. *glabrata* marquées dans le mélange obtenu à l'étape précédente ;
**d.** une étape de détermination de l'augmentation du taux de chitine de 10% à une valeur inférieure à 20% dans le mélange de cellules de ladite souche ATCC^{®}2001 de *C*. *glabrata* marquées au marqueur fluorescent Calcofluor White et de fluconazole, en fonction de la concentration en fluconazole dans la population de cellules de ladite souche ATCC^{®}2001 de *C*. *glabrata,* par rapport au taux de chitine d'une population de cellules de ladite souche ATCC^{®}2001 de *C*. *glabrata* en absence de fluconazole ; ou
**e.** une étape de détermination de l'augmentation du taux de chitine supérieure ou égale 20% dans le mélange de cellules de ladite souche ATCC^{®}2001 de *C*. *glabrata* marquées au marqueur fluorescent Calcofluor White et de fluconazole, par rapport au taux de chitine d'une population de cellules de ladite souche ATCC^{®}2001 de *C*. *glabrata* en absence de fluconazole, suivi d'
**f.** une étape de dénombrement des cellules dans le mélange de cellules de ladite souche ATCC^{®}2001 de *C*. *glabrata* marquées au marqueur fluorescent Calcofluor White et de fluconazole, après la susdite étape de quantification du taux de chitine ; puis d'
**g.** une étape de détermination de la diminution du nombre de cellules de moins de 0,3 log ou d'un nombre de cellules stationnaire, en fonction de la concentration en fluconazole dans la population de cellules de ladite souche ATCC^{®}2001 de *C. glabrata,* par rapport au nombre de cellules dans une population de cellules de ladite souche ATCC^{®}2001 de *C*. *glabrata* en absence de fluconazole.

Selon un mode de réalisation particulier, la présente invention a pour objet un procédé de détermination du degré de sensibilité correspondant à un phénotype sensible, d'une population de cellules de la souche ATCC^{®}2001 de *C*. *glabrata* vis-à-vis du voriconazole, comprenant :
**a.** une étape de mise en présence des cellules de ladite souche ATCC^{®}2001 de *C. glabrata* avec un gradient de concentrations de voriconazole variant de 0.007 à 8 µg/ml, plus particulièrement de 0,007 à 0,015 µg/ml, de 0,007 à 0,031 µg/ml, de 0,007 à 0,062 µg/ml, de 0,007 à 0,125 µg/ml, de 0,007 à 0,25 µg/ml, de 0,007 à 0,5 µg/ml, de 0,007 à 1 µg/ml, de 0,007 à 2 µg/ml, de 0,007 à 4 µg/ml, de 0,007 à 8 µg/ml, pendant une durée de 6,5h, à une température de 35°C; pour obtenir un mélange de cellules de ladite souche ATCC^{®}2001 de *C*. *glabrata* avec le voriconazole ;
**b.** une étape d'addition du marqueur fluorescent Calcofluor White au mélange de cellules de ladite souche ATCC^{®}2001 de *C*. *glabrata* et de voriconazole obtenu précédemment, pour obtenir un mélange de cellules de ladite souche ATCC^{®}2001 de *C*. *glabrata* marquées au marqueur fluorescent Calcofluor White, et de voriconazole;
**c.** une étape de quantification du taux de chitine par microscopie à fluorescence automatisée à haut contenu d'images, des cellules de ladite souche ATCC^{®}2001 de *C*. *glabrata* marquées dans le mélange obtenu à l'étape précédente ;
**d.** une étape de détermination de l'augmentation du taux de chitine d'une valeur supérieure ou égale à 20% dans le mélange de cellules de ladite souche ATCC^{®}2001 de *C*. *glabrata* marquées au marqueur fluorescent Calcofluor White et de voriconazole, en fonction de la concentration en voriconazole dans la population de cellules de ladite souche ATCC^{®}2001 de *C*. *glabrata,* par rapport au taux de chitine d'une population de cellules de ladite souche ATCC^{®}2001 de *C*. *glabrata* en absence de voriconazole ; suivi d'
**e.** une étape de dénombrement des cellules dans le mélange de cellules de ladite souche ATCC^{®}2001 de *C*. *glabrata* marquées au marqueur fluorescent Calcofluor White et de voriconazole, puis d' ;
**f.** une étape de détermination de la diminution du nombre de cellules d'au moins 0,3 log en fonction de la concentration en voriconazole dans la population de cellules de ladite souche ATCC^{®}2001 de *C*. *glabrata* par rapport au nombre de cellules dans une population de cellules de ladite souche ATCC^{®}2001 de *C*. *glabrata* en absence de voriconazole.

Selon un mode de réalisation particulier, la présente invention a pour objet un procédé de détermination du degré de sensibilité correspondant à un phénotype sensible, d'une population de cellules de la souche ATCC^{®}2001 de *C*. *glabrata* vis-à-vis de la micafungine, comprenant :
**a.** une étape de mise en présence des cellules de ladite souche ATCC^{®}2001 de *C. glabrata* avec un gradient de concentrations de micafungine variant de 0,0009 à 1 µg/ml, plus particulièrement 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 35°C; pour obtenir un mélange de cellules de ladite souche ATCC^{®}2001 de *C*. *glabrata* avec la micafungine ;
**b.** une étape d'addition du marqueur fluorescent Calcofluor White au mélange de cellules de ladite souche ATCC^{®}2001 de *C*. *glabrata* et de micafungine obtenu précédemment, pour obtenir un mélange de cellules de ladite souche ATCC^{®}2001 de *C*. *glabrata* marquées au marqueur fluorescent Calcofluor White, et de micafungine;
**c.** une étape de quantification du taux de chitine par microscopie à fluorescence automatisée à haut contenu d'images, des cellules de ladite souche ATCC^{®}2001 de *C*. *glabrata* marquées dans le mélange obtenu à l'étape précédente ;
**d.** une étape de détermination de l'augmentation du taux de chitine d'une valeur supérieure ou égale à 20% dans le mélange de cellules de ladite souche ATCC^{®}2001 de *C*. *glabrata* marquées au marqueur fluorescent Calcofluor White et de micafungine, en fonction de la concentration en micafungine dans la population de cellules de ladite souche ATCC^{®}2001 de *C*. *glabrata,* par rapport au taux de chitine d'une population de cellules de ladite souche ATCC^{®}2001 de *C*. *glabrata* en absence de micafungine ; suivi d'
**e.** une étape de dénombrement des cellules dans le mélange de cellules de ladite souche ATCC^{®}2001 de *C*. *glabrata* marquées au marqueur fluorescent Calcofluor White et de micafungine ; puis d'
**f.** une étape de détermination de la diminution du nombre de cellules d'au moins 0,3 log en fonction de la concentration en micafungine dans la population de cellules de ladite souche ATCC^{®}2001 de *C*. *glabrata* par rapport au nombre de cellules dans une population de cellules de ladite souche ATCC^{®}2001 de *C*. *glabrata* en absence de micafungine.

Selon un mode de réalisation particulier, la présente invention a pour objet un procédé de détermination du degré de sensibilité correspondant à un phénotype résistant, d'une population de cellules de la souche Tg5 de *C*. *glabrata* vis-à-vis du fluconazole, comprenant :
**a.** une étape de mise en présence des cellules de ladite souche Tg5 de *C*. *glabrata* avec un gradient de concentrations de fluconazole variant de 0 à 128 µg/ml, plus particulièrement de 0 à 0,125 µg/ml, de 0 à 0,250µg/ml, de 0 à 0,5 µg/ml, de 0 à 1 µg/ml, de 0 à 2 µg/ml, de 0 à 4 µg/ml, de 0 à 8 µg/ml, de 0 à 16 µg/ml, de 0 à 32 µg/ml, de 0 à 64 µg/ml, de 0 à 128 µg/ml, pendant une durée de 6,5h, à une température de 35°C; pour obtenir un mélange de cellules de ladite souche Tg5 de *C*. *glabrata* avec le fluconazole ;
**b.** une étape d'addition du marqueur fluorescent Calcofluor White au mélange de cellules de ladite souche Tg5 de *C*. *glabrata* et de fluconazole obtenu précédemment, pour obtenir un mélange de cellules de ladite souche Tg5 de *C*. *glabrata* marquées au marqueur fluorescent Calcofluor White, et de fluconazole;
**c.** une étape de quantification du taux de chitine par microscopie à fluorescence automatisée à haut contenu d'images, des cellules de ladite souche Tg5 de *C*. *glabrata* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche Tg5 de *C*. *glabrata* marquées au marqueur fluorescent Calcofluor White et de fluconazole, en fonction de la concentration en fluconazole dans la population de cellules de ladite souche Tg5 de *C*. *glabrata,* par rapport au taux de chitine d'une population de cellules de ladite souche Tg5 de *C*. *glabrata* en absence de fluconazole.

Selon un mode de réalisation particulier, la présente invention a pour objet un procédé de détermination du degré de sensibilité correspondant à un phénotype résistant, d'une population de cellules de la souche Tg5 de *C*. *glabrata* vis-à-vis du fluconazole, comprenant :
**a.** une étape de mise en présence des cellules de ladite souche Tg5 de *C*. *glabrata* avec un gradient de concentrations de fluconazole variant de 0 à 128 µg/ml, plus particulièrement de 0 à 0,125 µg/ml, de 0 à 0,250µg/ml, de 0 à 0,5 µg/ml, de 0 à 1 µg/ml, de 0 à 2 µg/ml, de 0 à 4 µg/ml, de 0 à 8 µg/ml, de 0 à 16 µg/ml, de 0 à 32 µg/ml, de 0 à 64 µg/ml, de 0 à 128 µg/ml, pendant une durée de 6,5h, à une température de 35°C; pour obtenir un mélange de cellules de ladite souche Tg5 de *C*. *glabrata* avec le fluconazole ;
**b.** une étape d'addition du marqueur fluorescent Calcofluor White au mélange de cellules de ladite souche Tg5 de *C*. *glabrata* et de fluconazole obtenu précédemment, pour obtenir un mélange de cellules de ladite souche Tg5 de *C*. *glabrata* marquées au marqueur fluorescent Calcofluor White, et de fluconazole;
**c.** une étape de quantification du taux de chitine par microscopie à fluorescence automatisée à haut contenu d'images, des cellules de ladite souche Tg5 de C. *glabrata* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche Tg5 de *C*. *glabrata* marquées au marqueur fluorescent Calcofluor White et de fluconazole, en fonction de la concentration en fluconazole dans la population de cellules de ladite souche Tg5 de *C*. *glabrata,* par rapport au taux de chitine d'une population de cellules de ladite souche Tg5 de *C. glabrata* en absence de fluconazole.

Selon un mode de réalisation particulier, la présente invention a pour objet un procédé de détermination du degré de sensibilité correspondant à un phénotype résistant, d'une population de cellules de la souche Tg5 de *C*. *glabrata* vis-à-vis du voriconazole, comprenant :
**a.** une étape de mise en présence des cellules de ladite souche Tg5 de *C*. *glabrata* avec un gradient de concentrations de voriconazole variant de 0,007 à 8 µg/ml, plus particulièrement de 0,007 à 0,015 µg/ml, de 0,007 à 0,031 µg/ml, de 0,007 à 0,062 µg/ml, de 0,007 à 0,125 µg/ml, de 0,007 à 0,25 µg/ml, de 0,007 à 0,5 µg/ml, de 0,007 à 1 µg/ml, de 0,007 à 2 µg/ml, de 0,007 à 4 µg/ml, de 0,007 à 8 µg/ml, pendant une durée de 6,5h, à une température de 35°C; pour obtenir un mélange de cellules de ladite souche Tg5 de *C*. *glabrata* avec le voriconazole ;
**b.** une étape d'addition du marqueur fluorescent Calcofluor White au mélange de cellules de ladite souche Tg5 de *C*. *glabrata* et de voriconazole obtenu précédemment, pour obtenir un mélange de cellules de ladite souche Tg5 de *C*. *glabrata* marquées au marqueur fluorescent Calcofluor White, et de voriconazole;
**c.** une étape de quantification du taux de chitine par microscopie à fluorescence automatisée à haut contenu d'images, des cellules de ladite souche Tg5 de *C*. *glabrata* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche Tg5 de *C*. *glabrata* marquées au marqueur fluorescent Calcofluor White et de voriconazole, en fonction de la concentration en voriconazole dans la population de cellules de ladite souche Tg5 de *C*. *glabrata,* par rapport au taux de chitine d'une population de cellules de ladite souche Tg5 de *C*. *glabrata* en absence de voriconazole.

Selon un mode de réalisation particulier, la présente invention a pour objet un procédé de détermination du degré de sensibilité correspondant à un phénotype résistant, d'une population de cellules de la souche Tg5 de *C*. *glabrata* vis-à-vis du voriconazole, comprenant :
**a.** une étape de mise en présence des cellules de ladite souche Tg5 de *C*. *glabrata* avec un gradient de concentrations de voriconazole variant de 0,007 à 8 µg/ml, plus particulièrement de 0,007 à 0,015 µg/ml, de 0,007 à 0,031 µg/ml, de 0,007 à 0,062 µg/ml, de 0,007 à 0,125 µg/ml, de 0,007 à 0,25 µg/ml, de 0,007 à 0,5 µg/ml, de 0,007 à 1 µg/ml, de 0,007 à 2 µg/ml, de 0,007 à 4 µg/ml, de 0,007 à 8 µg/ml, pendant une durée de 6,5h, à une température de 35°C; pour obtenir un mélange de cellules de ladite souche Tg5 de *C*. *glabrata* avec le voriconazole ;
**b.** une étape d'addition du marqueur fluorescent Calcofluor White au mélange de cellules de ladite souche Tg5 de *C*. *glabrata* et de voriconazole obtenu précédemment, pour obtenir un mélange de cellules de ladite souche Tg5 de *C*. *glabrata* marquées au marqueur fluorescent Calcofluor White, et de voriconazole;
**c.** une étape de quantification du taux de chitine par microscopie à fluorescence automatisée à haut contenu d'images, des cellules de ladite souche Tg5 de *C*. *glabrata* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche Tg5 de *C*. *glabrata* marquées au marqueur fluorescent Calcofluor White et de voriconazole, en fonction de la concentration en voriconazole dans la population de cellules de ladite souche Tg5 de *C. glabrata,* par rapport au taux de chitine d'une population de cellules de ladite souche Tg5 de *C*. *glabrata* en absence de voriconazole.

Selon un mode de réalisation particulier, la présente invention a pour objet un procédé de détermination du degré de sensibilité correspondant à un phénotype résistant, d'une population de cellules de la souche Tg5 de *C*. *glabrata* vis-à-vis de la micafungine, comprenant :
**a.** une étape de mise en présence des cellules de ladite souche Tg5 de *C*. *glabrata* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 35°C; pour obtenir un mélange de cellules de ladite souche Tg5 de *C*. *glabrata* avec la micafungine ;
**b.** une étape d'addition du marqueur fluorescent Calcofluor White au mélange de cellules de ladite souche Tg5 de *C*. *glabrata* et de micafungine obtenu précédemment, pour obtenir un mélange de cellules de ladite souche Tg5 de *C*. *glabrata* marquées au marqueur fluorescent Calcofluor White, et de micafungine;
**c.** une étape de quantification du taux de chitine par microscopie à fluorescence automatisée à haut contenu d'images, des cellules de ladite souche Tg5 de *C*. *glabrata* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche Tg5 de *C*. *glabrata* marquées au marqueur fluorescent Calcofluor White et de micafungine, en fonction de la concentration en micafungine dans la population de cellules de ladite souche Tg5 de *C*. *glabrata,* par rapport au taux de chitine d'une population de cellules de ladite souche Tg5 de *C*. *glabrata* en absence de micafungine.

Selon un mode de réalisation particulier, la présente invention a pour objet un procédé de détermination du degré de sensibilité correspondant à un phénotype résistant, d'une population de cellules de la souche Tg5 de *C*. *glabrata* vis-à-vis de la micafungine, comprenant :
**a.** une étape de mise en présence des cellules de ladite souche Tg5 de *C*. *glabrata* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 35°C; pour obtenir un mélange de cellules de ladite souche Tg5 de *C*. *glabrata* avec la micafungine ;
**b.** une étape d'addition du marqueur fluorescent Calcofluor White au mélange de cellules de ladite souche Tg5 de *C*. *glabrata* et de micafungine obtenu précédemment, pour obtenir un mélange de cellules de ladite souche Tg5 de *C*. *glabrata* marquées au marqueur fluorescent Calcofluor White, et de micafungine;
**c.** une étape de quantification du taux de chitine par microscopie à fluorescence automatisée à haut contenu d'images, des cellules de ladite souche Tg5 de *C*. *glabrata* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche Tg5 de *C*. *glabrata* marquées au marqueur fluorescent Calcofluor White et de micafungine, en fonction de la concentration en micafungine dans la population de cellules de ladite souche Tg5 de *C. glabrata,* par rapport au taux de chitine d'une population de cellules de ladite souche Tg5 de C. *glabrata* en absence de micafungine.

Selon un mode de réalisation particulier, la présente invention a pour objet un procédé de détermination du degré de sensibilité correspondant à un phénotype sensible, d'une population de cellules de la souche ATCC^{®}7349 de *C*. *tropicalis* vis-à-vis du fluconazole, comprenant :
**a.** une étape de mise en présence des cellules de ladite souche ATCC^{®}7349 de *C. tropicalis* avec un gradient de concentrations de fluconazole variant de 0,015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 24h, à une température de 30°C; pour obtenir un mélange de cellules de ladite souche ATCC^{®}7349 de *C*. *tropicalis* avec le fluconazole ;
**b.** une étape d'addition du marqueur fluorescent Calcofluor White au mélange de cellules de ladite souche ATCC^{®}7349 de *C*. *tropicalis* et de fluconazole obtenu précédemment, pour obtenir un mélange de cellules de ladite souche ATCC^{®}7349 de *C*. *tropicalis* marquées au marqueur fluorescent Calcofluor White, et de fluconazole;
**c.** une étape de quantification du taux de chitine par microscopie à fluorescence automatisée à haut contenu d'images, des cellules de ladite souche ATCC^{®}7349 de *C*. *tropicalis* marquées dans le mélange obtenu à l'étape précédente ;
**d.** une étape de détermination de l'augmentation du taux de chitine d'une valeur supérieure ou égale à 20% dans le mélange de cellules de ladite souche ATCC^{®}7349 de *C*. *tropicalis* marquées au marqueur fluorescent Calcofluor White et de fluconazole, en fonction de la concentration en fluconazole dans la population de cellules de ladite souche ATCC^{®}7349 de *C*. *tropicalis,* par rapport au taux de chitine d'une population de cellules de ladite souche ATCC^{®}7349 de *C*. *tropicalis* en absence de fluconazole ; suivi d'
**e.** une étape de dénombrement des cellules dans le mélange de cellules de ladite souche ATCC^{®}7349 de *C*. *tropicalis* marquées au marqueur fluorescent et de fluconazole; puis d'
**f.** une étape de détermination de la diminution du nombre de cellules d'au moins 0,3 log en fonction de la concentration en fluconazole dans la population de cellules de ladite souche ATCC^{®}7349 de *C*. *tropicalis* par rapport au nombre de cellules dans une population de cellules de ladite souche ATCC^{®}7349 de *C*. *tropicalis* en absence de fluconazole.

Selon un mode de réalisation particulier, la présente invention a pour objet un procédé de détermination du degré de sensibilité correspondant à un phénotype sensible, d'une population de cellules de la souche ATCC^{®}7349 de *C*. *tropicalis* vis-à-vis du voriconazole, comprenant :
**a.** une étape de mise en présence des cellules de ladite souche ATCC^{®}7349 de *C. tropicalis* avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche ATCC^{®}7349 de *C*. *tropicalis* avec le voriconazole ;
**b.** une étape d'addition du marqueur fluorescent Calcofluor White au mélange de cellules de ladite souche ATCC^{®}7349 de *C*. *tropicalis* et de voriconazole obtenu précédemment, pour obtenir un mélange de cellules de ladite souche ATCC^{®}7349 de *C*. *tropicalis* marquées au marqueur fluorescent Calcofluor White, et de voriconazole,
**c.** une étape de quantification du taux de chitine par microscopie à fluorescence automatisée à haut contenu d'images, des cellules de ladite souche ATCC^{®}7349 de *C*. *tropicalis* marquées dans le mélange obtenu à l'étape précédente ;
**d.** une étape de détermination de l'augmentation du taux de chitine d'une valeur supérieure ou égale à 20% dans le mélange de cellules de ladite souche ATCC^{®}7349 de *C*. *tropicalis* marquées au marqueur fluorescent Calcofluor White et de voriconazole, en fonction de la concentration en voriconazole dans la population de cellules de ladite souche ATCC^{®}7349 de *C*. *tropicalis,* par rapport au taux de chitine d'une population de cellules de ladite souche ATCC^{®}7349 de *C*. *tropicalis* en absence de voriconazole ; suivi d'
**e.** une étape de dénombrement des cellules dans le mélange de cellules de ladite souche ATCC^{®}7349 de *C*. *tropicalis* marquées au marqueur fluorescent Calcofluor White et de voriconazole; puis d'
**f.** une étape de détermination de la diminution du nombre de cellules d'au moins 0,3 log en fonction de la concentration en voriconazole dans la population de cellules de ladite souche ATCC^{®}7349 de *C*. *tropicalis* par rapport au nombre de cellules dans une population de cellules de ladite souche ATCC^{®}7349 de *C*. *tropicalis* en absence de voriconazole.

Selon un mode de réalisation particulier, la présente invention a pour objet un procédé de détermination du degré de sensibilité correspondant à un phénotype sensible, d'une population de cellules de la souche ATCC^{®}7349 de *C*. *tropicalis* vis-à-vis de la micafungine, comprenant :
**a.** une étape de mise en présence des cellules de ladite souche ATCC^{®}7349 de *C. tropicalis* avec un gradient de concentration de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche ATCC^{®}7349 de *C*. *tropicalis* avec la micafungine ;
**b.** une étape d'addition du marqueur fluorescent Calcofluor White au mélange de cellules de ladite souche ATCC^{®}7349 de *C*. *tropicalis* et de micafungine obtenu précédemment, pour obtenir un mélange de cellules de ladite souche ATCC^{®}7349 de *C*. *tropicalis* marquées au marqueur fluorescent Calcofluor White, et de micafungine;
**c.** une étape de quantification du taux de chitine par microscopie à fluorescence automatisée à haut contenu d'images, des cellules de ladite souche ATCC^{®}7349 de *C*. *tropicalis* marquées dans le mélange obtenu à l'étape précédente;
**d.** une étape de détermination de l'augmentation du taux de chitine d'une valeur supérieure ou égale à 20% dans le mélange de cellules de ladite souche ATCC^{®}7349 de *C*. *tropicalis* marquées au marqueur fluorescent Calcofluor White et de micafungine, en fonction de la concentration en micafungine dans la population de cellules de ladite souche ATCC^{®}7349 de *C*. *tropicalis,* par rapport au taux de chitine population de cellules de ladite souche ATCC^{®}7349 de *C*. *tropicalis* en absence de micafungine ; suivi d'
**e.** une étape de dénombrement des cellules dans le mélange de cellules de ladite souche ATCC^{®}7349 de *C*. *tropicalis* marquées au marqueur fluorescent Calcofluor white et de micafungine ; puis d'
**f.** une étape de détermination de la diminution du nombre de cellules d'au moins 0,3 log en fonction de la concentration en micafungine dans la population de cellules de ladite souche ATCC^{®}7349 de *C*. *tropicalis* par rapport au nombre de cellules dans une population de cellules de ladite souche ATCC^{®}7349 de *C*. *tropicalis* en absence de micafungine.

Selon un mode de réalisation particulier, la présente invention a pour objet un procédé de détermination du degré de sensibilité correspondant à un phénotype résistant, d'une population de cellules de la souche 13/5 de *C*. *tropicalis* vis-à-vis de la micafungine, comprenant :
**a.** une étape de mise en présence des cellules de ladite souche 13/5 de *C*. *tropicalis* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche 13/5 de *C*. *tropicalis* avec la micafungine ;
**b.** une étape d'addition du marqueur fluorescent Calcofluor White au mélange de cellules de ladite souche 13/5 de *C*. *tropicalis* et de micafungine obtenu précédemment, pour obtenir un mélange de cellules de ladite souche 13/5 de *C*. *tropicalis* marquées au marqueur fluorescent Calcofluor White, et de micafungine;
**c.** une étape de quantification du taux de chitine par microscopie à fluorescence automatisée à haut contenu d'images, des cellules de ladite souche 13/5 de *C*. *tropicalis* marquées dans le mélange obtenu à l'étape précédente;
**d.** une étape de détermination de l'absence d'augmentation du taux de chitine ou de l'augmentation du taux de chitine inférieure à 10 % dans le mélange de cellules de ladite souche 13/5 de *C. tropicalis* marquées au marqueur fluorescent Calcofluor White et de micafungine, en fonction de la concentration en micafungine dans la population de cellules de ladite souche 13/5 de *C. tropicalis,* par rapport au taux de chitine d'une population de cellules de ladite souche 13/5 de *C. tropicalis* en absence de micafungine.

Selon un mode de réalisation particulier, la présente invention a pour objet un procédé de détermination du degré de sensibilité correspondant à un phénotype résistant, d'une population de cellules de la souche 13/5 de *C. tropicalis* vis-à-vis de la micafungine, comprenant :
**a**. une étape de mise en présence des cellules de ladite souche 13/5 de *C. tropicalis* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche 13/5 de *C. tropicalis* avec la micafungine ;
**b.** une étape d'addition du marqueur fluorescent Calcofluor White au mélange de cellules de ladite souche 13/5 de *C. tropicalis* et de micafungine obtenu précédemment, pour obtenir un mélange de cellules de ladite souche 13/5 de *C. tropicalis* marquées au marqueur fluorescent Calcofluor White, et de micafungine;
**c.** une étape de quantification du taux de chitine par microscopie à fluorescence automatisée à haut contenu d'images, des cellules de ladite souche 13/5 de *C. tropicalis* marquées dans le mélange obtenu à l'étape précédente;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche 13/5 de *C. tropicalis* marquées au marqueur fluorescent Calcofluor White et de micafungine, en fonction de la concentration en micafungine dans la population de cellules de ladite souche 13/5 de *C. tropicalis,* par rapport au taux de chitine d'une population de cellules de ladite souche 13/5 de *C. tropicalis* en absence de micafungine.

Selon un mode de réalisation particulier, la présente invention a pour objet un procédé de détermination du degré de sensibilité correspondant à un phénotype sensible, d'une population de cellules de la souche ATCC^{®}22019 de *C. parapsilosis* vis-à-vis du fluconazole, comprenant :
**a.** une étape de mise en présence des cellules de ladite souche ATCC^{®}22019 de *C. parapsilosis* avec un gradient de concentrations de fluconazole variant de 0,015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 6,5h, à une température de 30°C; pour obtenir un mélange de cellules de ladite souche ATCC^{®}22019 de *C. parapsilosis* avec le fluconazole ;
**b.** une étape d'addition du marqueur fluorescent Calcofluor White au mélange de cellules de ladite souche ATCC^{®}22019 de *C. parapsilosis* et de fluconazole obtenu précédemment, pour obtenir un mélange de cellules de ladite souche ATCC^{®}22019 de *C. parapsilosis* marquées au marqueur fluorescent Calcofluor White, et de fluconazole;
**c.** une étape de quantification du taux de chitine par microscopie à fluorescence automatisée à haut contenu d'images, des cellules de ladite souche ATCC^{®}22019 de *C. parapsilosis* marquées dans le mélange obtenu à l'étape précédente ;
**d.** une étape de détermination de l'augmentation du taux de chitine d'une valeur supérieure ou égale à 20% dans le mélange de cellules de ladite souche ATCC^{®}22019 de *C. parapsilosis* marquées au marqueur fluorescent Calcofluor White et de fluconazole, en fonction de la concentration en fluconazole dans la population de cellules de ladite souche ATCC^{®}22019 de *C. parapsilosis,* par rapport au taux de chitine d'une population de cellules de ladite souche ATCC^{®}22019 de *C. parapsilosis* en absence de fluconazole ; suivi d'
**e.** une étape de dénombrement des cellules dans le mélange de cellules de ladite souche ATCC^{®}22019 de *C. parapsilosis* marquées au marqueur fluorescent Calcofluor White et de fluconazole ; puis d'
**f.** une étape de détermination de la diminution du nombre de cellules d'au moins 0,3 log en fonction de la concentration en fluconazole dans la population de cellules de ladite souche ATCC^{®}22019 de *C. parapsilosis* par rapport au nombre de cellules dans une population de cellules de ladite souche ATCC^{®}22019 de *C. parapsilosis* en absence de fluconazole.

Selon un mode de réalisation particulier, la présente invention a pour objet un procédé de détermination du degré de sensibilité correspondant à un phénotype sensible, d'une population de cellules de la souche ATCC^{®}22019 de *C. parapsilosis* vis-à-vis du voriconazole, comprenant :
**a.** une étape de mise en présence des cellules de ladite souche ATCC^{®}22019 de *C. parapsilosis* champignon avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche ATCC^{®}22019 de *C. parapsilosis* avec le voriconazole ;
**b.** une étape d'addition du marqueur fluorescent Calcofluor White au mélange de cellules de ladite souche ATCC^{®}22019 de *C. parapsilosis* et de voriconazole obtenu précédemment, pour obtenir un mélange de cellules de ladite souche ATCC^{®}22019 de *C. parapsilosis* marquées au marqueur fluorescent Calcofluor White, et de voriconazole,
**c.** une étape de quantification du taux de chitine par microscopie à fluorescence automatisée à haut contenu d'images, des cellules de ladite souche ATCC^{®}22019 de *C*. *parapsilosis* marquées dans le mélange obtenu à l'étape précédente;
**d.** une étape de détermination de l'augmentation du taux de chitine d'une valeur supérieure ou égale à 20% dans le mélange de cellules de ladite souche ATCC^{®}22019 de *C. parapsilosis* marquées au marqueur fluorescent Calcofluor White et de voriconazole, en fonction de la concentration en voriconazole dans la population de cellules de ladite souche ATCC^{®}22019 de *C. parapsilosis,* par rapport au taux de chitine d'une population de cellules de ladite souche ATCC^{®}22019 de *C. parapsilosis* en absence de voriconazole ; suivi d'
**e.** une étape de dénombrement des cellules dans le mélange de cellules de ladite souche ATCC^{®}22019 de *C. parapsilosis* marquées au marqueur fluorescent Calcofluor White et de voriconazole ; puis d'
**f.** une étape de détermination de la diminution du nombre de cellules d'au moins 0,3 log en fonction de la concentration en voriconazole dans la population de cellules de ladite souche ATCC^{®}22019 de *C. parapsilosis* par rapport au nombre de cellules dans une population de cellules de ladite souche ATCC^{®}22019 de *C. parapsilosis* en absence de voriconazole.

Selon un mode de réalisation particulier, la présente invention a pour objet un procédé de détermination du degré de sensibilité correspondant à un phénotype sensible, d'une population de cellules de la souche ATCC^{®}22019 de *C. parapsilosis* vis-à-vis de la micafungine, comprenant :
**a.** une étape de mise en présence des cellules de ladite souche ATCC^{®}22019 de *C. parapsilosis* champignon avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 24h, à une température de 30°C; pour obtenir un mélange de cellules de ladite souche ATCC^{®}22019 de *C. parapsilosis* avec la micafungine ;
**b.** une étape d'addition du marqueur fluorescent Calcofluor White au mélange de cellules de ladite souche ATCC^{®}22019 de *C. parapsilosis* et de micafungine obtenu précédemment, pour obtenir un mélange de cellules de ladite souche ATCC^{®}22019 de *C. parapsilosis* marquées au marqueur fluorescent Calcofluor White, et de micafungine;
**c.** une étape de quantification du taux de chitine par microscopie à fluorescence automatisée à haut contenu d'images, des cellules de ladite souche ATCC^{®}22019 de *C*. *parapsilosis* marquées dans le mélange obtenu à l'étape précédente;
**d.** une étape de détermination de l'augmentation du taux de chitine d'une valeur supérieure ou égale à 20% dans le mélange de cellules de ladite souche ATCC^{®}22019 de *C. parapsilosis* marquées au marqueur fluorescent Calcofluor White et de micafungine, en fonction de la concentration en micafungine dans la population de cellules de ladite souche ATCC^{®}22019 de *C. parapsilosis,* par rapport au taux de chitine d'une population de cellules de ladite souche ATCC^{®}22019 de *C. parapsilosis* en absence de micafungine ; suivi d'
**e.** une étape de dénombrement des cellules dans le mélange de cellules de ladite souche ATCC^{®}22019 de *C. parapsilosis* marquées au marqueur fluorescent Calcofluor White et de micafungine ; puis d'
**f.** une étape de détermination de la diminution du nombre de cellules d'au moins 0,3 log en fonction de la concentration en micafungine dans la population de cellules de ladite souche ATCC^{®}22019 de *C. parapsilosis* par rapport au nombre de cellules dans une population de cellules de ladite souche ATCC^{®}22019 de *C. parapsilosis* en absence de micafungine.

Selon un mode de réalisation particulier, la présente invention a pour objet un procédé de détermination du degré de sensibilité correspondant à un phénotype résistant, d'une population de cellules de la souche 8/21 de *C. parapsilosis* vis-à-vis du fluconazole, comprenant :
**a.** une étape de mise en présence des cellules de ladite souche 8/21 de *C. parapsilosis* avec un gradient de concentrations de fluconazole variant de 0,015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 6,5h, à une température de 30°C; pour obtenir un mélange de cellules de ladite souche 8/21 de *C. parapsilosis* avec le fluconazole ;
**b.** une étape d'addition du marqueur fluorescent Calcofluor White au mélange de cellules de ladite souche 8/21 de *C. parapsilosis* et de fluconazole obtenu précédemment, pour obtenir un mélange de cellules de ladite souche 8/21 de *C. parapsilosis* marquées au marqueur fluorescent Calcofluor White, et de fluconazole;
**c.** une étape de quantification du taux de chitine par microscopie à fluorescence automatisée à haut contenu d'images, des cellules de ladite souche 8/21 de *C. parapsilosis* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche 8/21 de *C. parapsilosis* marquées au marqueur fluorescent Calcofluor White et de fluconazole, en fonction de la concentration en fluconazole dans la population de cellules de ladite souche 8/21 de *C. parapsilosis,* par rapport au taux de chitine d'une population de cellules de ladite souche 8/21 de *C. parapsilosis* en absence de fluconazole.

Selon un mode de réalisation particulier, la présente invention a pour objet un procédé de détermination du degré de sensibilité correspondant à un phénotype résistant, d'une population de cellules de la souche 8/21 de *C. parapsilosis* vis-à-vis du fluconazole, comprenant :
**a.** une étape de mise en présence des cellules de ladite souche 8/21 de *C*. *parapsilosis* avec un gradient de concentrations de fluconazole variant de 0,015 à 16 µg/ml, plus particulièrement de 0,015 à 0,031 µg/ml, de 0,015 à 0,062 µg/ml, de 0,015 à 0,125 µg/ml, de 0,015 à 0,25 µg/ml, de 0,015 à 0,5 µg/ml, de 0,015 à 1 µg/ml, de 0,015 à 2 µg/ml, de 0,015 à 4 µg/ml, de 0,015 à 8 µg/ml, de 0,015 à 16 µg/ml, pendant une durée de 6,5h, à une température de 30°C; pour obtenir un mélange de cellules de ladite souche 8/21 de *C. parapsilosis* avec le fluconazole ;
**b.** une étape d'addition du marqueur fluorescent Calcofluor White au mélange de cellules de ladite souche 8/21 de *C. parapsilosis* et de fluconazole obtenu précédemment, pour obtenir un mélange de cellules de ladite souche 8/21 de *C. parapsilosis* marquées au marqueur fluorescent Calcofluor White, et de fluconazole;
**c.** une étape de quantification du taux de chitine par microscopie à fluorescence automatisée à haut contenu d'images, des cellules de ladite souche 8/21 de *C. parapsilosis* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche 8/21 de *C. parapsilosis* marquées au marqueur fluorescent Calcofluor White et de fluconazole, en fonction de la concentration en fluconazole dans la population de cellules de ladite souche 8/21 de *C. parapsilosis,* par rapport au taux de chitine d'une population de cellules de ladite souche 8/21 de *C. parapsilosis* en absence de fluconazole.

Selon un mode de réalisation particulier, la présente invention a pour objet un procédé de détermination du degré de sensibilité correspondant à un phénotype résistant, d'une population de cellules de la souche 8/21 de *C. parapsilosis* vis-à-vis du voriconazole, comprenant :
**a.** une étape de mise en présence des cellules de ladite souche 8/21 de *C. parapsilosis* avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche 8/21 de *C. parapsilosis* avec le voriconazole ;
**b.** une étape d'addition du marqueur fluorescent Calcofluor White au mélange de cellules de ladite souche de champignon et de voriconazole obtenu précédemment, pour obtenir un mélange de cellules de ladite souche 8/21 de *C. parapsilosis* marquées au marqueur fluorescent Calcofluor White, et de voriconazole,
**c.** une étape de quantification du taux de chitine par microscopie à fluorescence automatisée à haut contenu d'images, des cellules de ladite souche 8/21 de *C. parapsilosis* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche 8/21 de *C. parapsilosis* marquées au marqueur fluorescent Calcofluor White et de voriconazole, en fonction de la concentration en voriconazole dans la population de cellules de ladite souche 8/21 de *C. parapsilosis,* par rapport au taux de chitine d'une population de cellules de ladite souche 8/21 de *C. parapsilosis* en absence de voriconazole.

Selon un mode de réalisation particulier, la présente invention a pour objet un procédé de détermination du degré de sensibilité correspondant à un phénotype résistant, d'une population de cellules de la souche 8/21 de *C. parapsilosis* vis-à-vis du voriconazole, comprenant :
**a.** une étape de mise en présence des cellules de ladite souche 8/21 de *C. parapsilosis* avec un gradient de concentrations de voriconazole variant de 0,004 à 5 µg/ml, plus particulièrement de 0,004 à 0,009 µg/ml, de 0,004 à 0,019 µg/ml, de 0,004 à 0,039 µg/ml, de 0,004 à 0,078 µg/ml, de 0,004 à 0,156 µg/ml, de 0,004 à 0,312 µg/ml, de 0,004 à 0,0625 µg/ml, de 0,004 à 1,25 µg/ml, de 0,004 à 2,5 µg/ml, de 0,004 à 5 µg/ml, pendant une durée de 6,5h, à une température de 30°C; pour obtenir un mélange de cellules de ladite souche 8/21 de *C. parapsilosis* avec le voriconazole ;
**b.** une étape d'addition du marqueur fluorescent Calcofluor White au mélange de cellules de ladite souche de champignon et de voriconazole obtenu précédemment, pour obtenir un mélange de cellules de ladite souche 8/21 de *C. parapsilosis* marquées au marqueur fluorescent Calcofluor White, et de voriconazole,
**c.** une étape de quantification du taux de chitine par microscopie à fluorescence automatisée à haut contenu d'images, des cellules de ladite souche 8/21 de *C. parapsilosis* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche 8/21 de *C. parapsilosis* marquées au marqueur fluorescent Calcofluor White et de voriconazole, en fonction de la concentration en voriconazole dans la population de cellules de ladite souche 8/21 de *C. parapsilosis,* par rapport au taux de chitine d'une population de cellules de ladite souche 8/21 de *C. parapsilosis* en absence de voriconazole.

Selon un mode de réalisation particulier, la présente invention a pour objet un procédé de détermination du degré de sensibilité correspondant à un phénotype résistant, d'une population de cellules de la souche ATCC^{®}6258 de *C. krusei* vis-à-vis du fluconazole, comprenant :
**a.** une étape de mise en présence des cellules de ladite souche ATCC^{®}6258 de *C. krusei* avec un gradient de concentrations de fluconazole variant de 0,125 à 128 µg/ml, plus particulièrement de 0,125 à 0,250 µg/ml, de 0,125 à 0,5 µg/ml, de 0,125 à 1 µg/ml, de 0,125 à 2 µg/ml, de 0,125 à 4 µg/ml, de 0,125 à 8 µg/ml, de 0,125 à 16 µg/ml, de 0,125 à 32 µg/ml, de 0,125 à 64 µg/ml, de 0,125 à 128 µg/ml, pendant une durée de 6,5h, à une température de 30°C; pour obtenir un mélange de cellules de ladite souche ATCC^{®}6258 de *C. krusei* avec le fluconazole ;
**b.** une étape d'addition du marqueur fluorescent Calcofluor White au mélange de cellules de ladite souche ATCC^{®}6258 de *C. krusei* et de fluconazole obtenu précédemment, pour obtenir un mélange de cellules de ladite souche ATCC^{®}6258 de *C. krusei* marquées au marqueur fluorescent Calcofluor White, et de fluconazole;
**c.** une étape de quantification du taux de chitine par microscopie à fluorescence automatisée à haut contenu d'images, des cellules de ladite souche ATCC^{®}6258 de *C. krusei* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche ATCC^{®}6258 de *C*. *krusei* marquées au marqueur fluorescent Calcofluor White et de fluconazole, en fonction de la concentration en fluconazole dans la population de cellules de ladite souche ATCC^{®}6258 de *C. krusei,* par rapport au taux de chitine d'une population de cellules de ladite souche ATCC^{®}6258 de *C. krusei* en absence de fluconazole.

Selon un mode de réalisation particulier, la présente invention a pour objet un procédé de détermination du degré de sensibilité correspondant à un phénotype résistant, d'une population de cellules de la souche ATCC^{®}6258 de *C. krusei* vis-à-vis du fluconazole, comprenant :
**a.** une étape de mise en présence des cellules de ladite souche ATCC^{®}6258 de *C. krusei* avec un gradient de concentrations de fluconazole variant de 0.125 à 128 µg/ml, plus particulièrement de 0,125 à 0,250 µg/ml, de 0,125 à 0,5 µg/ml, de 0,125 à 1 µg/ml, de 0,125 à 2 µg/ml, de 0,125 à 4 µg/ml, de 0,125 à 8 µg/ml, de 0,125 à 16 µg/ml, de 0,125 à 32 µg/ml, de 0,125 à 64 µg/ml, de 0,125 à 128 µg/ml, pendant une durée de 6,5h, à une température de 30°C; pour obtenir un mélange de cellules de ladite souche ATCC^{®}6258 de *C. krusei* avec le fluconazole ;
**b.** une étape d'addition du marqueur fluorescent Calcofluor White au mélange de cellules de ladite souche ATCC^{®}6258 de *C. krusei* et de fluconazole obtenu précédemment, pour obtenir un mélange de cellules de ladite souche ATCC^{®}6258 de *C. krusei* marquées au marqueur fluorescent Calcofluor White, et de fluconazole;
**c.** une étape de quantification du taux de chitine par microscopie à fluorescence automatisée à haut contenu d'images, des cellules de ladite souche ATCC^{®}6258 de *C. krusei* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche ATCC^{®}6258 de *C. krusei* marquées au marqueur fluorescent Calcofluor White et de fluconazole, en fonction de la concentration en fluconazole dans la population de cellules de ladite souche ATCC^{®}6258 de *C*. *krusei,* par rapport au taux de chitine d'une population de cellules de ladite souche ATCC^{®}6258 de *C*. *krusei* en absence de fluconazole.

Selon un mode de réalisation particulier, la présente invention a pour objet un procédé de détermination du degré de sensibilité correspondant à un phénotype intermédiaire d'une population de cellules de la souche ATCC^{®}6258 de *C*. *krusei* vis-à-vis du voriconazole, comprenant :
**a.** une étape de mise en présence des cellules de ladite souche ATCC^{®}6258 de *C. krusei* avec un gradient de concentrations de voriconazole variant de 0,007 à 8 µg/ml, plus particulièrement de 0,007 à 0,015 µg/ml, de 0,007 à 0,031 µg/ml, de 0,007 à 0,062 µg/ml, de 0,007 à 0,125 µg/ml, de 0,007 à 0,25 µg/ml, de 0,007 à 0,5 µg/ml, de 0,007 à 1 µg/ml, de 0,007 à 2 µg/ml, de 0,007 à 4 µg/ml, de 0,007 à 8 µg/ml, pendant une durée de 24 h, à une température de 30°C; pour obtenir un mélange de cellules de ladite souche ATCC^{®}6258 de *C. krusei* avec le voriconazole ;
**b.** une étape d'addition d'un marqueur fluorescent Calcofluor White au mélange de cellules de ladite souche ATCC^{®}6258 de *C. krusei* et de voriconazole obtenu précédemment, pour obtenir un mélange de cellules de ladite souche ATCC^{®}6258 de *C. krusei* marquées au marqueur fluorescent Calcofluor White, et de voriconazole,
**c.** une étape de quantification du taux de chitine par microscopie à fluorescence automatisée à haut contenu d'images, des cellules de ladite souche ATCC^{®}6258 de *C. krusei* marquées dans le mélange obtenu à l'étape précédente ;
**d.** une étape de détermination de l'augmentation du taux de chitine de 10% à une valeur inférieure à 20% dans le mélange de cellules de ladite souche ATCC^{®}6258 de *C. krusei* marquées au marqueur fluorescent Calcofluor White et de voriconazole, en fonction de la concentration en voriconazole dans la population de cellules de ladite souche ATCC^{®}6258 de *C*. *krusei,* par rapport au taux de chitine d'une population de cellules de ladite souche ATCC^{®}6258 de *C*. *krusei* en absence de voriconazole ; ou
**e.** une étape de détermination de l'augmentation du taux de chitine supérieure ou égale à 20% dans le mélange de cellules de ladite souche ATCC^{®}6258 de *C. krusei* marquées au marqueur fluorescent Calcofluor White et de voriconazole, en fonction de la concentration en voriconazole dans la population de cellules de ladite souche ATCC^{®}6258 de *C*. *krusei,* par rapport au taux de chitine d'une population de cellules de ladite souche ATCC^{®}6258 de *C. krusei* en absence de voriconazole, suivi d'
f**.** une étape de dénombrement des cellules dans le mélange de cellules de ladite souche ATCC^{®}6258 de *C. krusei* marquées au marqueur fluorescent Calcofluor White et de voriconazole, après la susdite étape de quantification du taux de chitine ; puis d'
**g.** une étape de détermination de la diminution du nombre de cellules de moins de 0,3 log ou d'un nombre de cellules stationnaire, en fonction de la concentration en voriconazole dans la population de cellules de ladite souche ATCC^{®}6258 de *C*. *krusei* par rapport au nombre de cellules dans une population de cellules de ladite souche ATCC^{®}6258 de *C*. *krusei* en absence de voriconazole.

Selon un mode de réalisation particulier, la présente invention a pour objet un procédé de détermination du degré de sensibilité correspondant à un phénotype sensible, d'une population de cellules de la souche ATCC^{®}6258 de *C*. *krusei* vis-à-vis de la micafungine, comprenant :
**a.** une étape de mise en présence des cellules de ladite souche ATCC^{®}6258 de *C. krusei* avec un gradient de concentrations de micafungine variant de 0,0009 à 1 µg/ml, plus particulièrement de 0,0009 à 0,0019 µg/ml, de 0,0009 à 0,0039 µg/ml, de 0,0009 à 0,007 µg/ml, de 0,0009 à 0,015 µg/ml, de 0,0009 à 0,031 µg/ml, de 0,0009 à 0,062 µg/ml, de 0,0009 à 0,125 µg/ml, de 0,0009 à 0,25 µg/ml, de 0,0009 à 0,5 µg/ml, de 0,0009 à 1 µg/ml, pendant une durée de 6,5h, à une température de 30°C; pour obtenir un mélange de cellules de ladite souche ATCC^{®}6258 de *C*. *krusei* avec la micafungine ;
**b.** une étape d'addition du marqueur fluorescent Calcofluor White au mélange de cellules de ladite souche ATCC^{®}6258 de *C*. *krusei* et de micafungine obtenu précédemment, pour obtenir un mélange de cellules de ladite souche ATCC^{®}6258 de *C*. *krusei* marquées au marqueur fluorescent Calcofluor White, et de micafungine,
**c.** une étape de quantification du taux de chitine par microscopie à fluorescence automatisée à haut contenu d'images, des cellules de ladite souche ATCC^{®}6258 de *C*. *krusei* marquées dans le mélange obtenu à l'étape précédente ;
**d.** une étape de détermination de l'augmentation du taux de chitine d'une valeur supérieur ou égale à 20% dans le mélange de cellules de ladite souche ATCC^{®}6258 de *C*. *krusei* marquées au marqueur fluorescent Calcofluor White et de micafungine, en fonction de la concentration en micafungine dans la population de cellules de ladite souche ATCC^{®}6258 de *C*. *krusei,* par rapport au taux de chitine d'une population de cellules de ladite souche ATCC^{®}6258 de *C*. *krusei* en absence de micafungine ; suivi d'
**e.** une étape de dénombrement des cellules dans le mélange de cellules de ladite souche ATCC^{®}6258 de *C*. *krusei* marquées au marqueur fluorescent Calcofluor White et de micafungine ; puis d'
**f.** une étape de détermination de la diminution du nombre de cellules d'au moins 0,3 log en fonction de la concentration en micafungine dans la population de cellules de ladite souche ATCC^{®}6258 de *C*. *krusei* par rapport au nombre de cellules dans une population de cellules de ladite souche ATCC^{®}6258 de *C*. *krusei* en absence de micafungine.

Selon un mode de réalisation particulier, la présente invention a pour objet un procédé de détermination du degré de sensibilité correspondant à un phénotype résistant, d'une population de cellules de la souche GRE32 de *C*. *krusei* vis-à-vis du fluconazole, comprenant :
**a.** une étape de mise en présence des cellules de ladite souche GRE32 de *C*. *krusei* avec un gradient de concentrations de fluconazole variant de 0,125 à 128 µg/ml, plus particulièrement de 0,125 à 0,250 µg/ml, de 0,125 à 0,5 µg/ml, de 0,125 à 1 µg/ml, de 0,125 à 2 µg/ml, de 0,125 à 4 µg/ml, de 0,125 à 8 µg/ml, de 0,125 à 16 µg/ml, de 0,125 à 32 µg/ml, de 0,125 à 64 µg/ml, de 0,125 à 128 µg/ml, pendant une durée de 6,5h, à une température de 30°C; pour obtenir un mélange de cellules de ladite souche GRE32 de *C*. *krusei* avec le fluconazole ;
**b.** une étape d'addition du marqueur fluorescent Calcofluor White au mélange de cellules de ladite souche GRE32 de *C*. *krusei* et de fluconazole obtenu précédemment, pour obtenir un mélange de cellules de ladite souche GRE32 de *C*. *krusei* marquées au marqueur fluorescent Calcofluor White, et de fluconazole;
**c.** une étape de quantification du taux de chitine par microscopie à fluorescence automatisée à haut contenu d'images, des cellules de ladite souche GRE32 de *C. krusei* marquées dans le mélange obtenu à l'étape précédente ;
**d.** une étape de détermination de l'absence d'augmentation du taux de chitine ou de l'augmentation du taux de chitine inférieure à 10 % dans le mélange de cellules de ladite souche GRE32 de *C*. *krusei* marquées au marqueur fluorescent Calcofluor White et de fluconazole, en fonction de la concentration en fluconazole dans la population de cellules de ladite souche GRE32 de *C*. *krusei,* par rapport au taux de chitine d'une population de cellules de ladite souche GRE32 de *C*. *krusei* en absence de fluconazole.

Selon un mode de réalisation particulier, la présente invention a pour objet un procédé de détermination du degré de sensibilité correspondant à un phénotype résistant, d'une population de cellules de la souche GRE32 de *C*. *krusei* vis-à-vis du fluconazole, comprenant :
**a.** une étape de mise en présence des cellules de ladite souche GRE32 de *C*. *krusei* avec un gradient de concentrations de fluconazole variant de 0,125 à 128 µg/ml, plus particulièrement de 0,125 à 0,250 µg/ml, de 0,125 à 0,5 µg/ml, de 0,125 à 1 µg/ml, de 0,125 à 2 µg/ml, de 0,125 à 4 µg/ml, de 0,125 à 8 µg/ml, de 0,125 à 16 µg/ml, de 0,125 à 32 µg/ml, de 0,125 à 64 µg/ml, de 0,125 à 128 µg/ml, pendant une durée de 6,5h, à une température de 30°C; pour obtenir un mélange de cellules de ladite souche GRE32 de *C*. *krusei* avec le fluconazole ;
**b.** une étape d'addition du marqueur fluorescent Calcofluor White au mélange de cellules de ladite souche GRE32 de *C*. *krusei* et de fluconazole obtenu précédemment, pour obtenir un mélange de cellules de ladite souche GRE32 de *C*. *krusei* marquées au marqueur fluorescent Calcofluor White, et de fluconazole;
**c.** une étape de quantification du taux de chitine par microscopie à fluorescence automatisée à haut contenu d'images, des cellules de ladite souche GRE32 de *C. krusei* marquées dans le mélange obtenu à l'étape précédente ;
**d.** une étape de détermination d'une augmentation du taux de chitine inférieure à 10% ou d'une diminution du taux de chitine notamment inférieure à 20% ou d'un taux de chitine stationnaire, dans le mélange de cellules de ladite souche GRE32 de *C*. *krusei* marquées au marqueur fluorescent Calcofluor White et de fluconazole, en fonction de la concentration en fluconazole dans la population de cellules de ladite souche GRE32 de *C*. *krusei,* par rapport au taux de chitine d'une population de cellules de ladite souche GRE32 de *C*. *krusei* en absence de fluconazole.

Selon un mode de réalisation particulier, la présente invention a pour objet un procédé de détermination du degré de sensibilité correspondant à un phénotype intermédiaire d'une population de cellules de la souche GRE32 de *C*. *krusei* vis-à-vis du voriconazole, comprenant :
**a.** une étape de mise en présence des cellules de ladite souche GRE32 de *C. krusei* avec un gradient de concentrations de voriconazole variant de 0,007 à 8 µg/ml, plus particulièrement de 0,007 à 0,015 µg/ml, de 0,007 à 0,031 µg/ml, de 0,007 à 0,062 µg/ml, de 0,007 à 0,125 µg/ml, de 0,007 à 0,25 µg/ml, de 0,007 à 0,5 µg/ml, de 0,007 à 1 µg/ml, de 0,007 à 2 µg/ml, de 0,007 à 4 µg/ml, de 0,007 à 8 µg/ml, pendant une durée de 24h, à une température de 30°C; pour obtenir un mélange de cellules de ladite souche GRE32 de *C*. *krusei* avec le voriconazole ;
**b.** une étape d'addition d'un marqueur fluorescent Calcofluor White au mélange de cellules de ladite souche GRE32 de *C*. *krusei* et de voriconazole obtenu précédemment, pour obtenir un mélange de cellules de ladite souche GRE32 de *C*. *krusei* marquées au marqueur fluorescent Calcofluor White, et de voriconazole,
**c.** une étape de quantification du taux de chitine par microscopie à fluorescence automatisée à haut contenu d'images, des cellules de ladite souche GRE32 de *C. krusei* marquées dans le mélange obtenu à l'étape précédente ;
**d.** une étape de détermination de l'augmentation du taux de chitine de 10% à une valeur inférieure à 20% dans le mélange de cellules de ladite souche GRE32 de *C. krusei* marquées au marqueur fluorescent Calcofluor White et de voriconazole, en fonction de la concentration en voriconazole dans la population de cellules de ladite souche GRE32 de *C*. *krusei,* par rapport au taux de chitine d'une population de cellules de ladite souche GRE32 de *C. krusei* en absence de voriconazole ; ou
**e.** une étape de détermination de l'augmentation du taux de chitine supérieure ou égale à 20% dans le mélange de cellules de ladite souche GRE32 de *C. krusei* marquées au marqueur fluorescent Calcofluor White et de voriconazole, en fonction de la concentration en voriconazole dans la population de cellules de ladite souche GRE32 de *C. krusei,* par rapport au taux de chitine d'une population de cellules de ladite souche GRE32 de *C. krusei* en absence de voriconazole, suivi d'
**f.** une étape de dénombrement des cellules dans le mélange de cellules de ladite souche GRE32 de *C. krusei* marquées au marqueur fluorescent Calcofluor White et de voriconazole, après la susdite étape de quantification du taux de chitine ; puis d'
**g.** une étape de détermination de la diminution du nombre de cellules de moins de 0,3 log ou d'un nombre de cellules stationnaire, en fonction de la concentration en voriconazole dans la population de cellules de ladite souche GRE32 de *C. krusei* par rapport au nombre de cellules dans une population de cellules de ladite souche GRE32 de C. *krusei* en absence de voriconazole.

Selon un mode de réalisation particulier, la présente invention a pour objet un procédé de détermination du degré de sensibilité d'une population de cellules d'une souche de champignon, ledit champignon étant un champignon pluricellulaire du genre *Aspergillus* comprenant les espèces *A. fumigatus, A. flavus, A. nidulans, A. terreus, A. versicolor, A. niger,* vis-à-vis d'un antifongique choisi parmi le posaconazole, le voriconazole, l'itraconazole, l'isavuconazole, l'amphotéricine B et la nystatine, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Aspergillus* comprenant les espèces *A. fumigatus, A. flavus, A. nidulans, A. terreus, A. versicolor, A. niger,* vis-à-vis d'un antifongique choisi parmi la micafungine, l'anidulafungine et la caspofungine comprenant :
**a.** une étape de mise en présence des cellules de ladite souche de champignon avec un gradient de concentrations d'antifongique variant de 0,0017 à 2 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Fusarium* comprenant les espèces *Fusarium solani, Fusarium oxysporum,* vis-à-vis d'un antifongique choisi parmi le voriconazole, l'isavuconazole, l'amphotéricine B et la nystatine comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant;
lorsque ladite variation du taux de chitine de la susdite étape d. est une augmentation de 10 % à une valeur inférieure à 20%, il est déduit que ladite souche de champignon est de phénotype intermédiaire;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Scedosporium* comprenant les espèces *Scedosporium apiospermum, S. prolificans,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Mucor* comprenant l'espèce *Mucor racemosa,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant;
lorsque ladite variation du taux de chitine de la susdite étape d. est une augmentation de 10 % à une valeur inférieure à 20%, il est déduit que ladite souche de champignon est de phénotype intermédiaire;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Lichteimia* comprenant l'espèce *Lichteimia corymbifera,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Rhizopus* comprenant l'espèce *Rhizopus oryzae,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Rhizomucor* comprenant l'espèce *Rhizomucor pusillus,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Geotrichum,* vis-à-vis d'un antifongique choisi parmi le fluconazole, le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination du degré de sensibilité d'une population de cellules d'une souche de champignon, ledit champignon étant un champignon pluricellulaire du genre *Paecylomyces,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, il est déduit que ladite souche de champignon est de phénotype sensible ou intermédiaire.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Aspergillus* comprenant les espèces *A. fumigatus, A. flavus, A. nidulans, A. terreus, A. versicolor, A. niger,* vis-à-vis d'un antifongique choisi parmi le posaconazole, le voriconazole, l'itraconazole, l'isavuconazole, l'amphotéricine B et la nystatine comprenant :
**a.** une étape de mise en présence des cellules de ladite souche de champignon avec un gradient de concentrations d'antifongique variant de 0,007 à 125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Aspergillus* comprenant les espèces *A. fumigatus, A. flavus, A. nidulans, A. terreus, A. versicolor, A. niger,* vis-à-vis d'un antifongique choisi parmi la micafungine, l'anidulafungine et la caspofungine comprenant :
**a.** une étape de mise en présence des cellules de ladite souche de champignon avec un gradient de concentrations d'antifongique variant de 0,0017 à 8 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Fusarium* comprenant les espèces *F. solani et F. oxysporum,* vis-à-vis d'un antifongique choisi parmi le le voriconazole, l'isavuconazole, l'amphotéricine B et la nysatine comprenant :
**a.** une étape de mise en présence des cellules de ladite souche de champignon avec un gradient de concentrations d'antifongique variant de 0,007 à 125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Scedosporium* comprenant les espèces *S. apiospermum et S. prolificans,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Mucor* comprenant l'espèce *Mucor racemosa,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Lichteimia* comprenant l'espèce *Lichteimia corymbifera,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Rhizopus* comprenant l'espèce *Rhizopus oryzae,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Rhizomucor* comprenant l'espèce *Rhizomucor pusillus,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Geotrichum* vis-à-vis d'un antifongique choisi parmi le fluconazole, le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 125 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination de la sensibilité ou de la résistance d'une population de cellules d'une souche de champignon, ledit champignon étant un champignon pluricellulaire du genre *Paecylomyces,* vis-à-vis d'un antifongique choisi parmi le posaconazole, le voriconazole, l'isavuconazole et l'amphotéricine B, comprenant :
**a.** une étape de mise en présence des cellules de ladite souche de champignon avec un gradient de concentrations d'antifongique variant de 0,007 à 8 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique;
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination du degré de sensibilité d'une population de cellules d'une souche de champignon pluricellulaire, vis-à-vis d'un antifongique choisi parmi le fluconazole, le posaconazole, le voriconazole, l'itraconazole, l'isaconazole et l'amphotéricine B et la nystatine comprenant :
**a.** une étape de mise en présence des cellules de ladite souche avec un gradient de concentrations d'antifongique variant de 0,007 à 8 µg/ml, plus particulièrement de 0,007 à 0,015 µg/ml, de 0,007 à 0,031 µg/ml, de 0,007 à 0,062 µg/ml, de 0,007 à 0,125 µg/ml, de 0,007 à 0,25 µg/ml, de 0,007 à 0,5 µg/ml, de 0,007 à 1 µg/ml, de 0,007 à 2 µg/ml, de 0,007 à 4 µg/ml, de 0,007 à 8 µg/ml, pendant une durée de 6,5h, à une température de 30°C; pour obtenir un mélange de cellules de ladite souche avec ledit antifongique ;
**b.** une étape d'addition du marqueur fluorescent Calcofluor White au mélange de cellules de ladite souche et dudit antifongique obtenu précédemment, pour obtenir un mélange de cellules de ladite souche marquées au marqueur fluorescent Calcofluor White, et dudit antifongique;
**c.** une étape de quantification du taux de chitine par microscopie à fluorescence automatisée à haut contenu d'images, des cellules de ladite souche marquées dans le mélange obtenu à l'étape précédente ;
**d.** une étape de détermination de la variation éventuelle du taux de chitine dans le mélange de cellules de ladite souche marquées au marqueur fluorescent Calcofluor White et de micafungine, en fonction de la concentration en antifongique dans la population de cellules de ladite souche, par rapport au taux de chitine d'une population de cellules de ladite souche en absence d'antifongique ; suivi d'
**e.** une étape de détermination de la longueur de l'hyphe germinatif dans le mélange de cellules de ladite souche marquées au marqueur fluorescent Calcofluor White et d'antifongique ; puis d'
**f.** une étape de détermination de la variation éventuelle de de la longueur de l'hyphe germinatif en fonction de la concentration en antifongique dans la population de cellules de ladite souche, par rapport à la longueur de l'hyphe germinatif dans une population de cellules de ladite souche en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

Selon un mode de réalisation particulier, la présente invention a pour objet un procédé de détermination du degré de sensibilité d'une population de cellules d'une souche de champignon pluricellulaire, vis-à-vis d'un antifongique choisi parmi la micafungine l'anidulafungine et la caspofungine comprenant :
**a.** une étape de mise en présence des cellules de ladite souche avec un gradient de concentrations d'antifongique variant de 0,0017 à 2 µg/ml, plus particulièrement de 0,0017 à 0,003 µg/ml, de 0,0017 à 0,007 µg/ml, de 0,0017 à 0,015 µg/ml, de 0,0017 à 0,031 µg/ml, de 0,0017 à 0,062 µg/ml, de 0,0017 à 0,125 µg/ml, de 0,0017 à 0,25 µg/ml, de 0,0017 à 0,5 µg/ml, de 0,0017 à 1 µg/ml, de 0,0017 à 2 µg/ml, pendant une durée de 6,5h, à une température de 30°C; pour obtenir un mélange de cellules de ladite souche avec ledit antifongique ;
**b.** une étape d'addition du marqueur fluorescent Calcofluor White au mélange de cellules de ladite souche et dudit antifongique obtenu précédemment, pour obtenir un mélange de cellules de ladite souche marquées au marqueur fluorescent Calcofluor White, et dudit antifongique;
**c.** une étape de quantification du taux de chitine par microscopie à fluorescence automatisée à haut contenu d'images, des cellules de ladite souche marquées dans le mélange obtenu à l'étape précédente ;
**d.** une étape de détermination de la variation du taux de chitine dans le mélange de cellules de ladite souche marquées au marqueur fluorescent Calcofluor White et de micafungine, en fonction de la concentration en antifongique dans la population de cellules de ladite souche, par rapport au taux de chitine d'une population de cellules de ladite souche en absence d'antifongique ; suivi d'
**e.** une étape de détermination de la longueur de l'hyphe germinatif dans le mélange de cellules de ladite souche marquées au marqueur fluorescent Calcofluor White et d'antifongique ; puis d'
**f.** une étape de détermination de la variation de de la longueur de l'hyphe germinatif en fonction de la concentration en antifongique dans la population de cellules de ladite souche, par rapport à la longueur de l'hyphe germinatif dans une population de cellules de ladite souche en absence d'antifongique ;

lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de moins de 10% de la longueur dudit hyphe ou une longueur des hyphes germinatifs stationnaire, la souche est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution d'au moins 10% de la longueur dudit hyphe, la souche est de phénotype sensible.

### FIGURES

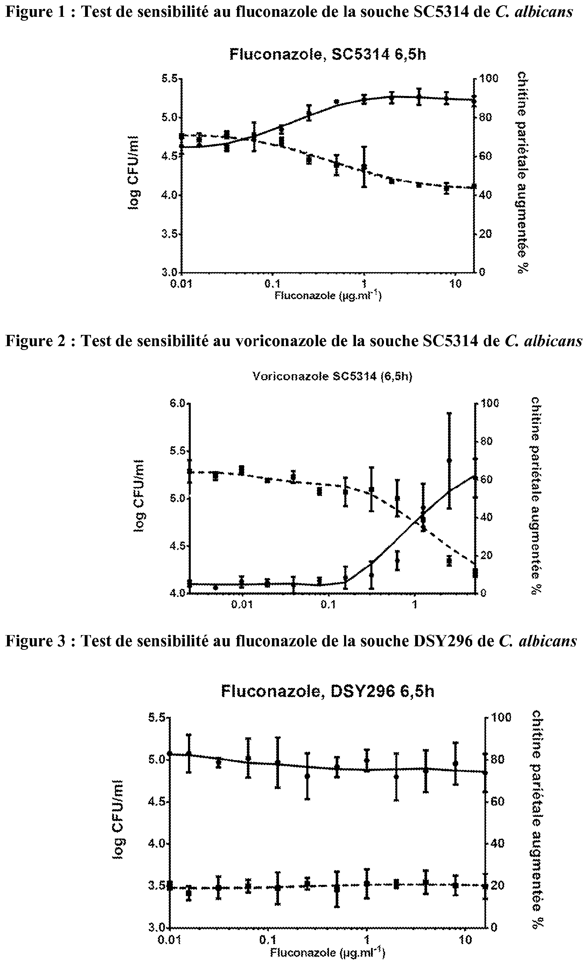

**Figure 1** **:** Elle représente l'effet du fluconazole sur des cellules de la souche SC5314 de C. *albicans.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).
**Figure 2** **:** Elle représente l'effet du voriconazole sur des cellules de la souche SC5314 de C. *albicans.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).
**Figure 3** **:** Elle représente l'effet du fluconazole sur des cellules de la souche DSY296 de C. *albicans.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).

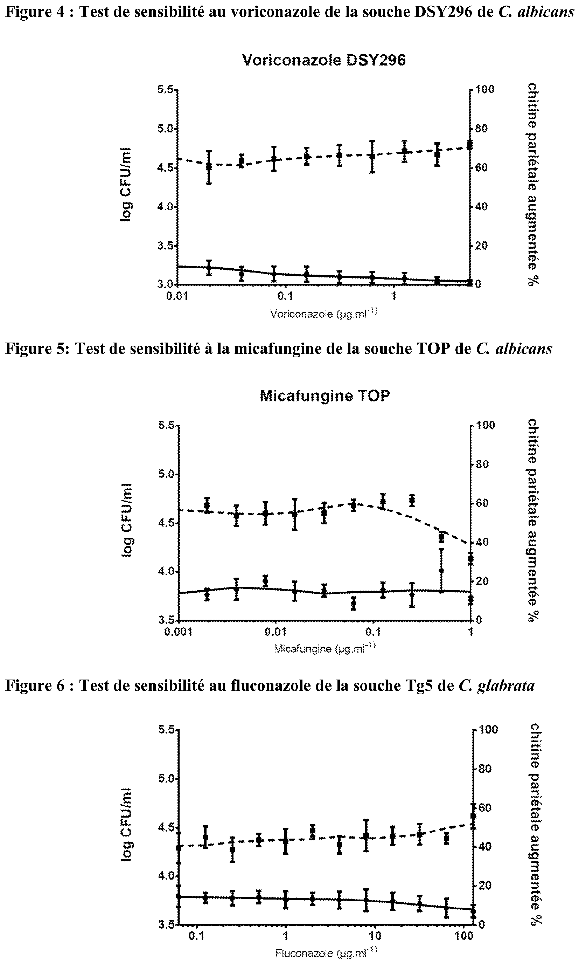

**Figure 4** **:** Elle représente l'effet du voriconazole sur des cellules de la souche DSY296 de C. *albicans.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).
**Figure 5** **:** Elle représente l'effet de la micafungine sur des cellules de la souche TOP de C. *albicans.* La courbe trait plein représente la variation du taux de chitine dans la paroi des cellules en fonction de la concentration en micafungine (échelle logarithmique). La courbe trait pointillé représente la variation du nombre de cellules dans le milieu en fonction de la concentration en micafungine (échelle logarithmique).
**Figure 6** **:** Elle représente l'effet du fluconazole sur des cellules de la souche Tg5 de C. *glabrata.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).

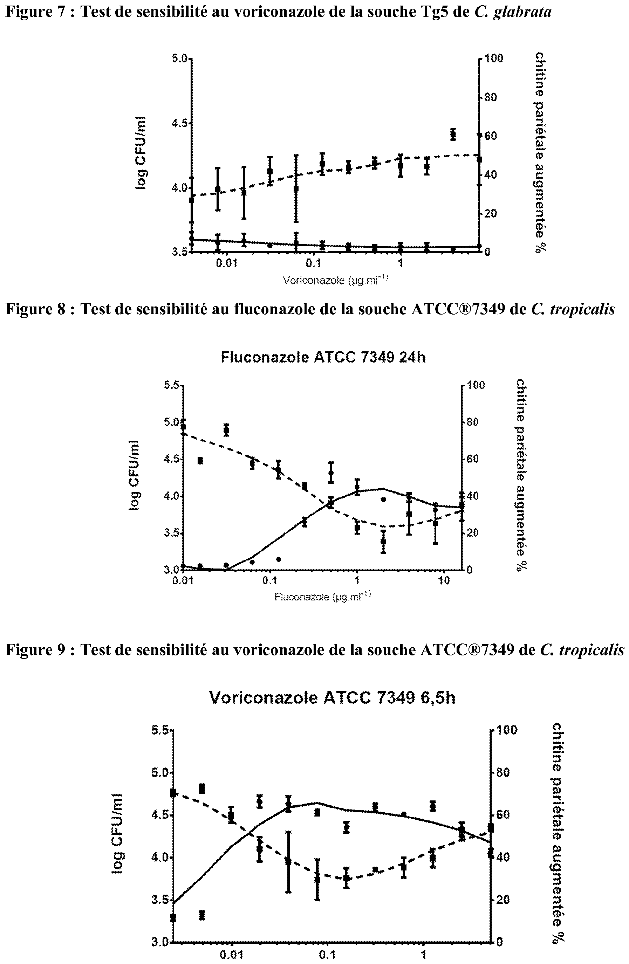

**Figure 7** **:** Elle représente l'effet du voriconazole sur des cellules de la souche Tg5 de C. *glabrata.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).
**Figure 8** : Elle représente l'effet du fluconazole sur des cellules de la souche ATCC^{®}7349 de *C. tropicalis.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).
**Figure 9** **:** Elle représente l'effet du voriconazole sur des cellules de la souche ATCC^{®}7349 de C. *tropicalis.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).

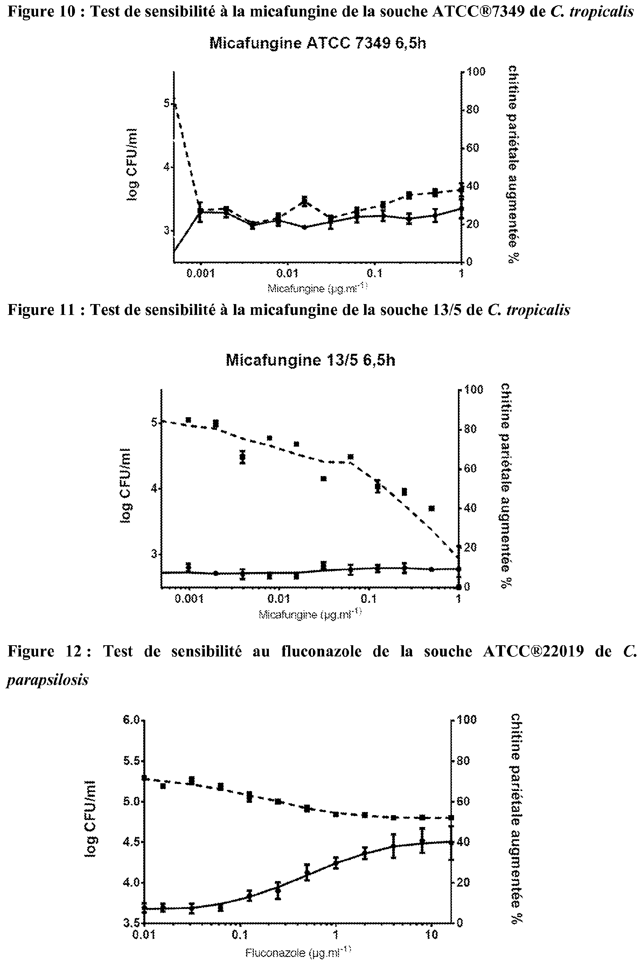

**Figure 10** **:** Elle représente l'effet de la micafungine sur des cellules de la souche ATCC^{®}7349 de *C*. *tropicalis.* La courbe trait plein représente la variation du taux de chitine dans la paroi des cellules en fonction de la concentration en micafungine (échelle logarithmique). La courbe trait pointillé représente la variation du nombre de cellules dans le milieu en fonction de la concentration en micafungine (échelle logarithmique).
**Figure 11** **:** Elle représente l'effet de la micafungine sur des cellules de la souche 13/5 de C. *tropicalis.* La courbe trait plein représente la variation du taux de chitine dans la paroi des cellules en fonction de la concentration en micafungine (échelle logarithmique). La courbe trait pointillé représente la variation du nombre de cellules dans le milieu en fonction de la concentration en micafungine (échelle logarithmique).
**Figure 12** **:** Elle représente l'effet du fluconazole sur des cellules de la souche ATCC^{®}22019 de *C. parapsilosis.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).

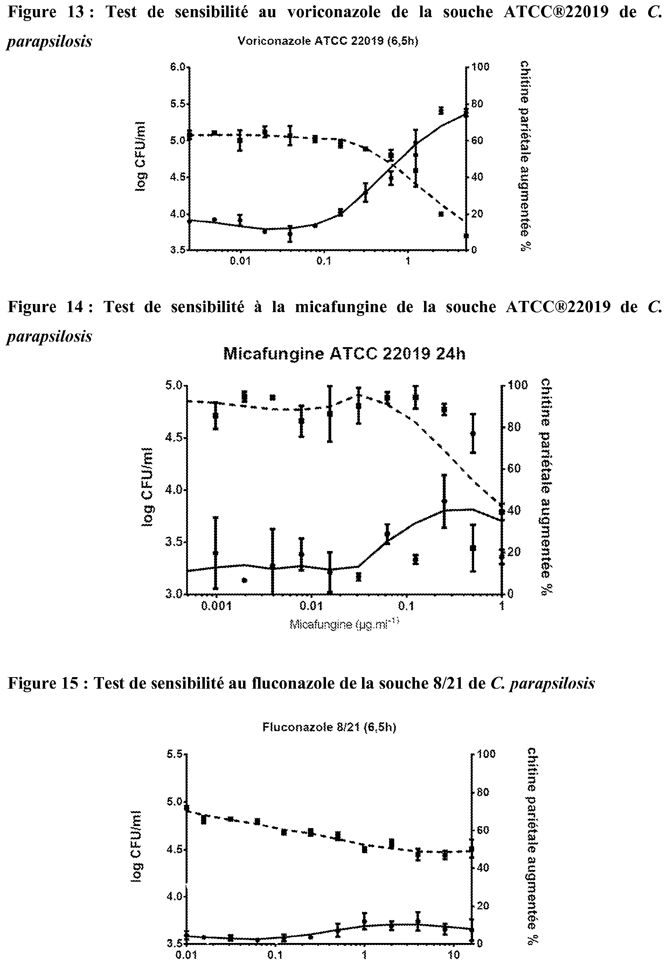

**Figure 13** **:** Elle représente l'effet du voriconazole sur des cellules de la souche ATCC^{®}22019 de *C. parapsilosis.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).
**Figure 14** **:** Elle représente l'effet de la micafungine sur des cellules de la souche ATCC^{®}22019 de *C. parapsilosis.* La courbe trait plein représente la variation du taux de chitine dans la paroi des cellules en fonction de la concentration en micafungine (échelle logarithmique). La courbe trait pointillé représente la variation du nombre de cellules dans le milieu en fonction de la concentration en micafungine (échelle logarithmique).
**Figure 15** **:** Elle représente l'effet du fluconazole sur des cellules de la souche 8/21 de C. *parapsilosis.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).

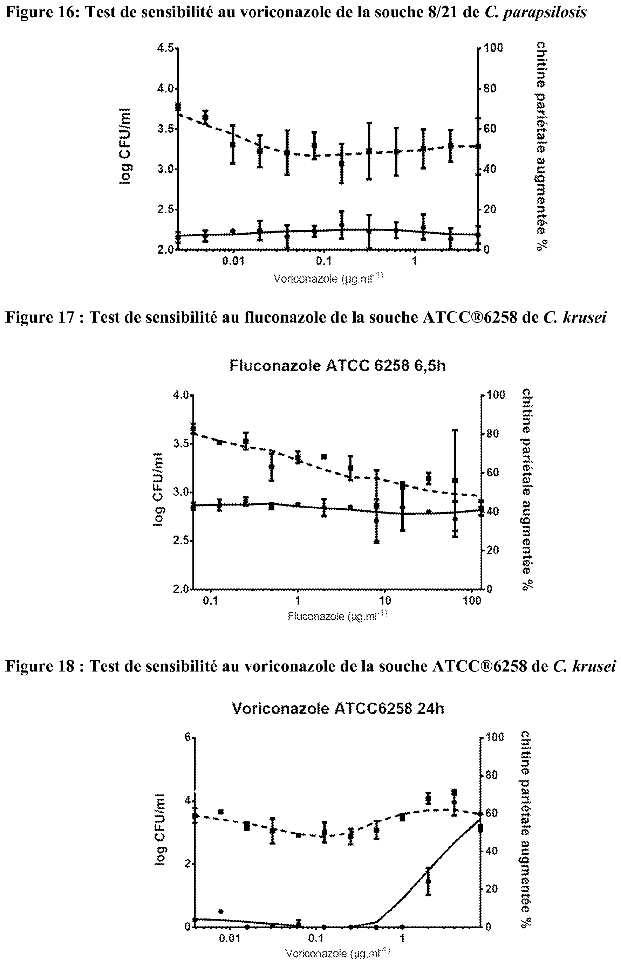

**Figure 16** **:** Elle représente l'effet du voriconazole sur des cellules de la souche 8/21 de C. *parapsilosis.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).
**Figure 17** **:** Elle représente l'effet du fluconazole sur des cellules de la souche ATCC^{®}6258 de C. *krusei.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).
**Figure 18** **:** Elle représente l'effet du voriconazole sur des cellules de la souche ATCC^{®}6258 de C. *krusei.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).

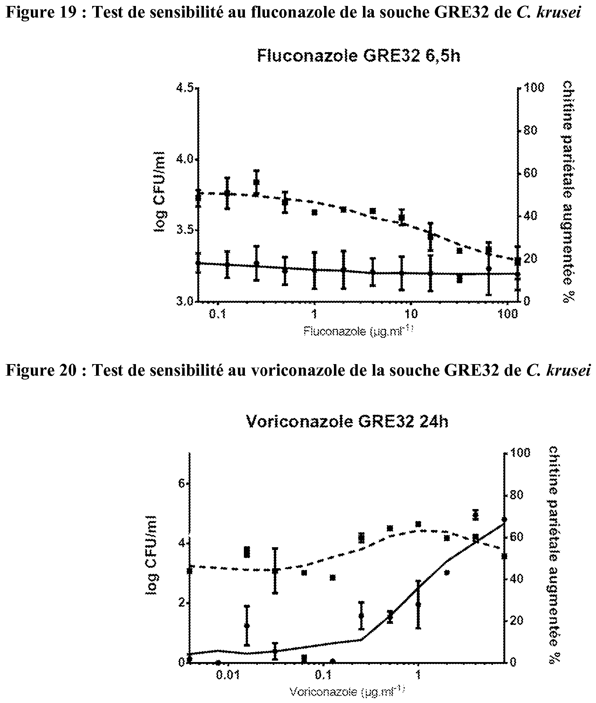

**Figure 19** **:** Elle représente l'effet du fluconazole sur des cellules de la souche GRE32 de C. *krusei.* La courbe trait plein représente la variation du taux de chitine dans la paroi des cellules en fonction de la concentration en fluconazole (échelle logarithmique). La courbe trait pointillé représente la variation du nombre de cellules dans le milieu en fonction de la concentration en fluconazole (échelle logarithmique).
**Figure 20** **:** Elle représente l'effet du voriconazole sur des cellules de la souche GRE32 de C. *krusei.* La courbe trait plein représente la variation du taux de chitine dans la paroi des cellules en fonction de la concentration en voriconazole (échelle logarithmique). La courbe trait pointillé représente la variation du nombre de cellules dans le milieu en fonction de la concentration en voriconazole (échelle logarithmique).

### EXEMPLES

### A- EXEMPLES CHAMPIGNONS UNICELLULAIRES

### I - Culture de champignon en présence d'antifongique

Les souches de Candida ont été préalablement incubées à 30°C pendant une nuit dans un milieu d'extrait de levure- peptone - dextrose (YPD) (1% de bacto peptone, 0.5% d'extrait de levure, 2% de glucose, 1.5% d'agar).

Des colonies de levures ont ensuite été prélevées des boites de milieuYPD et mises en suspension dans une solution saline à 0.9% NaCl dans laquelle la concentration cellulaire a été estimée par microscopie optique en utilisant des lames de numération Kova Slide.

Une dilution a été réalisée pour obtenir 15 ml d'un inoculum à 3.10⁶ CFU/ml, dans un milieu complet synthétique (SC) à pH 7 (2% de glucose, 0.5% d'ammonium sulfate, 0.17% de base azoté de levure, 0.2% de mélange synthétique complet et 10% d'HEPES 1,5M pH 7.2) pour *C. albicans, C. parapsilosis, C. tropicalis* et C. *krusei,* et dans une solution de RPMI 1640 pH 7.3 (10% d'HEPES 1M pH 7.2) pour C. *glabrata,* afin de réduire la formation de septa et d'amas.

1 ml d'inoculum a ensuite été ajouté à 2 ml de solution antifongique préparée dans un milieu SC ou RPMI selon l'espèce de champignon, de manière à obtenir une concentration finale en levure de 10⁶ CFU/ml et la concentration en antifongique voulue selon le tableau 1.

**Tableau 1 : Gradients de concentration de fluconazole, voriconazole et micafungine utilisés pour les espèces de levure Candida albicans, C. tropicalis, C. parapsilosis, C. glabrata et C. krusei.**

| Antifongique | Espèce | Concentration (µg/ml) | | | | | | | | | | |
|---|---|---|---|---|---|---|---|---|---|---|---|---|
| Fluconazole | *C. albicans, C. tropicalis, C. parapsilosis* | 16 | 8 | 4 | 2 | 1 | 0,5 | 0,25 | 0,125 | 0,062 | 0,031 | 0,015 |
| | *C. glabrata, C. krusei* | 128 | 64 | 32 | 16 | 8 | 4 | 2 | 1 | 0,5 | 0,25 | 0,125 |
| Voriconazole | *C. albicans, C. tropicalis, C. parapsilosis* | 5 | 2,5 | 1,25 | 0,625 | 0,312 | 0,156 | 0,078 | 0,039 | 0,019 | 0,009 | 0,004 |
| | *C. glabrata, C. krusei* | 8 | 4 | 2 | 1 | 0,5 | 0,25 | 0,125 | 0,062 | 0,031 | 0,015 | 0,007 |
| Micafungine | *C. albicans, C. glabrata, C. tropicalis, C. parapsilosis, C. krusei* | 1 | 0,5 | 0,25 | 0,125 | 0,062 | 0,031 | 0,015 | 0,007 | 0,004 | 0,002 | 0,001 |

Après homogénéisation, les cultures ont été placées à 30°C à 200 rpm, à l'exception des cultures de C. *glabrata* placées à 35°C à 200 rpm, et incubées pendant une durée de 6.5 h ou de 24h.

### II - Analyse en microscopie automatisée à haut contenu d'images (HCA)

Après incubation, 100 µl de la culture de champignons obtenue ci-dessus ont été transférées en triplicat dans des plaques de 96 puits, et 2.5 µl de Calcofluor-White (CFW) ont été ajoutés dans chaque puits afin de marquer la chitine des parois cellulaires des levures.

Une étape d'acquisition d'images par microscopie à fluorescence automatisée (ScanR screening station, Olympus), en utilisant un objectif x40 et un filtre pour le CFW, a permis d'obtenir 30 images par puits afin de capturer assez de cellules pour réaliser des données statistiques significatives. L'analyse de ces images a été réalisée par logiciel (ScanR analysis software, Olympus). Un traitement du bruit de fond a d'abord été réalisé afin d'améliorer le contraste entre la fluorescence des levures et le bruit de fond. Un seuil en pixel est alors défini. La segmentation des éléments fluorescents présents sur chacune des images acquises est ensuite réalisée grâce à un algorithme prédéfini dans le logiciel permettant sur la base de l'intensité de fluorescence de chaque élément de déterminer la limite de cet élément. Un paramètre de taille est ensuite appliqué pour sélectionner les levures et éliminer les agrégats et les débris fluorescents.

Chaque élément correspondant alors à une levure, il est donc possible, à partir de ces éléments définis précédemment, de définir l'intensité de fluorescence pour chacune des levures, cette intensité étant directement corrélée avec le contenu en chitine de leur paroi.

A partir desdits éléments définis, il est également possible de dénombrer automatiquement les levures.

Ces données sont ensuite traitées par un logiciel (GraphPad Prism) pour réaliser une représentation graphique de la variation du taux chitine de la paroi cellulaire et la variation du nombre de cellules en CFU/ml (unité formatrice de colonies par ml) en fonction de la concentration en antifongique, pour chaque couple souche de levure/antifongique.

Lorsqu'une augmentation du taux de chitine d'une valeur inférieure à 10% ou une diminution du taux de chitine inférieure à 20% ou un taux de chitine stationnaire sont observés par rapport au taux de chitine d'une population de cellules dudit champignon en absence d'antifongique, la souche est de phénotype résistant.

Lorsqu'une augmentation du taux de chitine d'une valeur de 10% à une valeur inférieure à 20% est observée par rapport au taux de chitine d'une population de cellules dudit champignon en absence d'antifongique, la souche est de phénotype intermédiaire.

Lorsqu'une augmentation du taux de chitine supérieure ou égale à 20% est observée par rapport au taux de chitine d'une population de cellules dudit champignon en absence d'antifongique, ainsi qu'une diminution du nombre de cellule de moins de 0,3 log ou un nombre de cellules stationnaire par rapport au nombre de cellules dans une population de cellules dudit champignon en absence d'antifongique, la souche est de phénotype intermédiaire.

Lorsqu'une augmentation du taux de chitine supérieure ou égale à 20% est observée par rapport au taux de chitine d'une population de cellules dudit champignon en absence d'antifongique, ainsi qu'une diminution du nombre de cellule d'au moins 0,3 log par rapport au nombre de cellules dans une population de cellules dudit champignon en absence d'antifongique, la souche est de phénotype sensible.

Il est à noter que l'ensemble des exemples de la présente demande comportent des graphiques présentant à la fois la courbe de variation du taux de chitine et celle du nombre de cellules, alors que le paramètre de la variation du nombre de cellule n'est pas utilisé dans certains cas pour la détermination de la sensibilité de la souche de champignon.

En effet, la génération des deux courbes est inhérente au logiciel utilisé pour la réalisation des présents exemples.

La considération d'un paramètre autre que le dénombrement cellulaire pour la détermination de la sensibilité d'une souche de champignon constitue l'originalité de cette invention, par rapport aux tests existants basés sur la mesure de la croissance de levures, lesquels sont soumis à la subjectivité du lecteur.

L'objectif de la présente demande est donc bien de trouver un autre critère pour déterminer la sensibilité d'une souche de champignon, le dénombrement cellulaire figurant à titre de complément d'informations dans la plupart des cas. Ce paramètre est toutefois nécessaire pour distinguer à un phénotype intermédiaire d'un phénotype sensible lorsque la variation du taux de chitine est supérieure ou égale à 20%.

### III - Analyse par la méthode comparative Etest^{®} commercialisé par Biomérieux

La méthode Etest^{®} est un test commercial de détermination de la sensibilité d'une souche, le plus fréquemment utilisé en routine dans les laboratoires cliniques de mycologie.

Les résultats obtenus par HCA ont été comparés à ceux obtenus avec ce test de sensibilité.

Les tests Etest^{®} ont été réalisés selon les instructions du fabricant. Après étalement d'un inoculum standardisé Mac Farland 0.5 (équivalent de 10⁸ CFU/ml) sur des boites de milieu RPMI, celles-ci ont été incubées 24h à 35°C, à l'exception de *C*. *glabrata* où l'incubation a été prolongée de 48h afin de pouvoir déterminer la concentration minimale d'inhibition (CMI).

Les résultats des CMI obtenues ont été interprétés selon les tables du CLSI (Clinical and Laboratory Standards Institute) et/ou de l'EUCAST (European Committee on Antimicrobial Susceptibility Testing) (Arendrup MC, Cuenca-Estrella M, Lass-Flörl C, Hope WW (2014) Breakpoints for antifungal agents: an update from EUCAST focussing on echinocandins against Candida spp and triazoles against Aspergillus spp. Drug Resist Updat 16(6):81-95. doi:10.1016/j.drup.2014.01.001) (cf. **Tableau 2** issu de Maubon D, Gamaud C, Calandra T, et al. (2014) Resistance of Candida spp. to antifungal drugs in the ICU: where are we now? Intensive Care Med. doi: 10.1007/s00134-014-3404-7) qui répertorient les seuils cliniques (CBP) pour les espèces les plus communes de levure du genre Candida en fonction de différents antifongiques, ou selon la table des seuils épidémiologiques (ECV).

La CMI permet ainsi de déterminer la sensibilité d'une souche à un antifongique. Les CBP ou seuils cliniques permettent d'interpréter cette CMI et de prédire un échec de traitement chez le patient. Les CBP permettent d'établir les catégories : sensible, intermédiaire et résistante.

Si la valeur de la CMI est dans la catégorie sensible, la probabilité d'échec du traitement est faible. Cette probabilité augmente dans les catégories Intermédiaire ou Résistant

Ces tables permettent de détecter les résistances acquises qui résultent principalement de la sélection de mutants soumis à la pression des médicaments chez les patients. Elles sont ainsi spécifiques des souches et ne devraient pas être confondues avec des résistances intrinsèques qui sont spécifiques des espèces.

**Tableau 2 : Seuils cliniques des tables du CLSI (Clinical and Laboratory Standards Institute) et/ou de l'EUCAST (European Committee on Antimicrobial Susceptibility Testing) pour des espèces de Candida (Source : Maubon D, Garnaud C, Calandra T, et al. (2014) Resistance of Candida spp. to antifungal drugs in the ICU: where are we now? Intensive Care Med. doi: 10.1007/s00134-014-3404-7). ND : pas de données, IP : données en préparation, IE : données insuffisantes.**

| ***Antifongique*** | | CMI (mg/L) concentration minimale d'inhibition | | | | | | | | | | | | | |
|---|---|---|---|---|---|---|---|---|---|---|---|---|---|---|---|
| | | *C. albicans* | | *C. glabrata* | | *C. krusei* | | *C. parapsilosis* | | *C. tropicalis* | | *C. guilliermondii* | | *Non-species related breakpoints¹* | |
| | | S≤ | R> | S≤ | R> | S≤ | R> | S≤ | R> | S≤ | R> | S≤ | R> | S≤ | R> |
| ***Amphotéricine B*** | | | | | | | | | | | | | | | |
| | *EUCAST* | 1 | 1 | 1 | 1 | 1 | 1 | 1 | 1 | 1 | 1 | IE | IE | IE | IE |
| | *CLSI* | ND | ND | ND | ND | ND | ND | ND | ND | ND | ND | ND | ND | 1 | |
| ***Fluconazole*** | | | | | | | | | | | | | | | |
| | *EUCAST* | 2 | 4 | 0.002 | 32 | - | - | 2 | 4 | 2 | 4 | IE² | IE² | 2 | 4 |
| | *CLSI* | 2 | 4 | 0.002 | 32 | - | - | 2 | 4 | 2 | 4 | ND | ND | ND | ND |
| ***Voriconazole*** | | | | | | | | | | | | | | | |
| | *EUCAST* | 0.12⁵ | 0.12⁵ | IE² | IE² | IE² | IE² | 0.12⁵ | 0.12⁵ | 0.12⁵ | 0.12⁵ | IE² | IE² | IE | IE |
| | *CLSI* | 0.12 | 0.5 | - | - | 0.5 | 1 | 0.12 | 0.5 | 0.12 | 0.5 | ND | ND | ND | ND |
| ***Posaconazole*** | | | | | | | | | | | | | | | |
| | *EUCAST* | 0.06 | 0.06 | IE2 | IE2 | IE2 | IE2 | 0.06 | 0.06 | 0.06 | 0.06 | IE² | IE² | IE | IE |
| | *CLSI* | ND | ND | ND | ND | ND | ND | ND | ND | ND | ND | ND | ND | ND | ND |
| ***Itraconazole*** | | | | | | | | | | | | | | | |
| | *EUCAST* | IP | IP | IP | IP | IP | IP | IP | IP | IP | IP | IP | IP | IP | IP |
| | *CLSI* | ND | ND | ND | ND | ND | ND | ND | ND | ND | ND | ND | ND | 0.125 | 0.5 |
| ***Anidulafunaine*** | | | | | | | | | | | | | | | |
| | *EUCAST* | 0.03 | 0.03 | 0.06 | 0.06 | 0.06 | 0.06 | 0.002 | 4 | 0.06 | 0.06 | IE² | IE² | IE | IE |
| | *CLSI* | 0.25 | 0.5 | 0.12 | 0.25 | 0.25 | 0.5 | 2 | 4 | 0.25 | 0.5 | 2 | 4 | ND | ND |
| ***Micafungine*** | | | | | | | | | | | | | | | |
| | *EUCAST* | 0.016 | 0.016 | 0.03 | 0.03 | IE⁴ | IE⁴ | 0.002 | 2 | IE⁴ | IE⁴ | IE⁴ | IE⁴ | IE | IE |
| | *CLSI* | 0.25 | 0.5 | 0.06 | 0.125 | 0.25 | 0.5 | 2 | 4 | 0.25 | 0.5 | 2 | 4 | ND | ND |
| ***Caspofungine*** | | | | | | | | | | | | | | | |
| | *EUCAST* | Note³ | Note³ | Note³ | Note³ | Note³ | Note³ | Note³ | Note³ | Note³ | Note³ | IE² | IE² | Note³ | Note³ |
| | *CLSI* | 0.25 | 0.5 | 0.12 | 0.25 | 0.25 | 0.5 | 2 | 4 | 0.25 | 0.5 | 2 | 4 | ND | ND |
| ***Flucytosine*** | | | | | | | | | | | | | | | |
| | *EUCAST* | ND | ND | ND | ND | ND | ND | ND | ND | ND | ND | ND | ND | ND | ND |
| | *CLSI* | ND | ND | ND | ND | ND | ND | ND | ND | ND | ND | ND | ND | 4 | 16 |

| | | | | | | | | | | | | | | | |
|---|---|---|---|---|---|---|---|---|---|---|---|---|---|---|---|
| ¹ Seuils déterminés principalement sur la base de données de pharmacocinétique et indépendants des espèces. Ils ne sont à utiliser que pour les organismes qui ne disposent pas de seuils spécifiques. ² Les seuils pour ces espèces sont en général plus élevés que pour *C*. *albicans.* ³ En raison d'une importante variation inter-laboratoire pour les CMI de la caspofungine, les seuils EUCAST n'ont été établis. ⁴ Les CMI pour *C*. *tropicalis* sont 1 à 2 dilutions plus élevées que pour *C*. *albicans* et *C*. *glabrata.* Dans l'étude clinique, le taux de succès thérapeutique a été légèrement inférieur pour *C. tropicalis* que pour *C. albicans* aux deux doses (100 et 150 mg par jour). Cependant, la différence n'a pas été significative et la pertinence clinique de cette différence n'est pas connue . Les CMI pour *C. krusei* sont 3 dilutions plus élevées que pour *C. albicans* et, de même, celles de *C*. *guilliermondii* sont 8 dilutions plus élevés. En outre, seul un petit nombre de cas implique ces espèces dans des essais cliniques. Les données sont insuffisantes pour considérer que la population sauvage de ces espèces peut être considéré comme sensible à la micafungine. ⁵ Les souches avec des valeurs de CMI au-dessus du seuil S/I sont rares ou non encore rapportées. Les tests d'identification et de sensibilité sur ces isolats doivent être répétés et si le résultat est confirmé, l'isolat envoyé à un laboratoire de référence. Jusqu'à ce qu'il y ait des preuves concernant la réponse clinique pour les isolats confirmés avec CMI au-dessus du seuil clinique résistant actuelle (en italique), ils doivent être signalés comme résistants. | | | | | | | | | | | | | | | |

### Exemple 1 : Test de sensibilité au fluconazole de la souche SC5314 de C. albicans

La souche SC5314 de *C. albicans* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de fluconazole préparés dans du milieu SC pour obtenir une concentration finale en levure de 10⁶ CFU/ml et de 16 µg/ml en fluconazole.

A partir de l'inoculum, la même manipulation est effectuée de manière à obtenir des cultures à 10⁶ CFU/ml et les concentrations suivantes en fluconazole : 0,015 µg/ml, 0,031 µg/ml, 0,062 µg/ml, 0,125 µg/ml, 0,25 µg/ml, 0,5 ug/ml, 1 µg/ml, 2 µg/ml, 4 µg/ml, 8 µg/ml, 16 µg/ml.

Un témoin sans fluconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 1, où la courbe en trait plein représente la variation du taux de chitine en fonction de la concentration en fluconazole.

Une augmentation d'une valeur supérieure ou égale à 20% du taux de chitine dans la paroi des cellules de la souche SC5314 de *C. albicans* en présence de fluconazole est observée par rapport au taux de chitine d'une population de cellules de la souche SC5314 de *C. albicans* en absence de fluconazole.

Le nombre de cellules pour chaque triplicat de chaque condition de concentration en fluconazole et sans fluconazole est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 1, où la courbe en trait pointillé représente la variation du nombre de cellules en fonction de la concentration en fluconazole.

Une diminution d'au moins 0,3 log du nombre de cellules de la souche SC5314 de *C. albicans* en fonction de la concentration en fluconazole dans le milieu est observée, par rapport au nombre de cellules dans une population de cellules de la souche SC5314 de *C*. *albicans* en absence de fluconazole.

Ces résultats témoignent du phénotype sensible de la souche SC5314 de *C*. *albicans* vis-à-vis du fluconazole.

Ce résultat est confirmé par celui obtenu via la méthode Etest. En effet, avec cette méthode une CMI de 0,125 µg/ml est obtenue et l'interprétation par les tables EUCAST et CLSI permet de déterminer qu'avec une CMI inférieure à 2 µg/ml pour le fluconazole cette souche est sensible à cet antifongique.

### Exemple 2 : Test de sensibilité au voriconazole de la souche SC5314 de C. albicans

La souche SC5314 de *C. albicans* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de voriconazole préparés dans du milieu SC pour obtenir une concentration finale en levure de 10⁶ CFU/ml et de 5 µg/ml en voriconazole.

A partir de l'inoculum la même manipulation est effectuée de manière à obtenir des cultures à 10⁶ CFU/ml et les concentrations suivantes en voriconazole : 0,004 µg/ml, 0,009 µg/ml, 0,019 µg/ml, 0,039 µg/ml, 0,078 µg/ml, 0,156 µg/ml, 0,312 µg/ml, 0,625 µg/ml, 1,25 µg/ml, 2,5 µg/ml, 5 µg/ml.

Un témoin sans voriconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en antifongique et sans antifongique.

Les données obtenues ont été moyennées pour chaque condition différente testée et présentées sous la forme d'un graphique présenté en Figure 2, où la courbe en trait plein représente la variation du taux de chitine en fonction de la concentration en voriconazole.

Une augmentation d'une valeur supérieure ou égale à 20% du taux de chitine dans la paroi des cellules de la souche SC5314 de *C. albicans* en présence de voriconazole est observée par rapport au taux de chitine des cellules de la souche SC5314 de *C. albicans* en absence de voriconazole.

Le nombre de cellules pour chaque triplicat de chaque condition de concentration en voriconazole et sans voriconazole est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 2, où la courbe en trait pointillé représente la variation du nombre de cellules en fonction de la concentration en voriconazole.

Une diminution d'au moins 0,3 log du nombre de cellules de la souche SC5314 de *C. albicans* en fonction de la concentration en voriconazole dans le milieu est observée, par rapport au nombre de cellules dans une population de cellules de la souche SC5314 de *C. albicans* en absence de voriconazole.

Ces résultats témoignent du phénotype sensible de la souche SC5314 de *C*. *albicans* vis-à-vis du voriconazole.

Ce résultat est confirmé par celui obtenu via la méthode Etest. En effet, avec cette méthode une CMI de 0.012 µg/ml est obtenue et l'interprétation par les tables EUCAST et CLSI permet de déterminer qu'avec une CMI inférieure à 0,12 µg/ml pour le voriconazole cette souche est sensible à cet antifongique.

### Exemple 3 : Test de sensibilité au fluconazole de la souche DSY296 de C. albicans

La souche DSY296 de *C. albicans* a été cultivée tel que décrit dans la partie Exemples paragraphe I de manière à obtenir un inoculum à 3.10⁶ CFU/ml dans 15 ml de milieu SC.

1 ml de cet inoculum a ensuite été ajouté à 2 ml de solution de fluconazole préparés dans du milieu SC pour obtenir une concentration finale en levure de 10⁶ CFU/ml et de 16 µg/ml en fluconazole.

A partir de l'inoculum la même manipulation est effectuée de manière à obtenir des cultures à 10⁶ CFU/ml et les concentrations suivantes en fluconazole : 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml, 16 µg/ml.

Un témoin sans fluconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 3, où la courbe en trait plein représente la variation du taux de chitine en fonction de la concentration en fluconazole.

Une diminution du taux de chitine inférieure à 20 % dans la paroi des cellules de la souche DSY296 de *C. albicans* en présence de fluconazole est observée, par rapport au taux de chitine des cellules de la souche DSY296 de *C. albicans* en absence de fluconazole .

Ce résultat témoigne du phénotype résistant de la souche DSY296 de *C. albicans* vis-à-vis du fluconazole.

Ce résultat est confirmé par celui obtenu via la méthode Etest. En effet, avec cette méthode une CMI de 96 µg/ml est obtenue et l'interprétation par les tables EUCAST et CLSI permet de déterminer qu'avec une CMI supérieure à 4 µg/ml pour le fluconazole cette souche est résistante à cet antifongique.

### Exemple 4 : Test de sensibilité au voriconazole de la souche DSY296 de C. albicans

La souche DSY296 de *C. albicans* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de voriconazole préparés dans du milieu SC pour obtenir une concentration finale en levure de 10⁶ CFU/ml et de 5 µg/ml en voriconazole.

A partir de l'inoculum la même manipulation est effectuée de manière à obtenir des cultures à 10⁶ CFU/ml et les concentrations suivantes en voriconazole : 0,004 µg/ml, 0,009 µg/ml, 0,019 ug/ml, 0,039 µg/ml, 0,078 µg/ml, 0,156 ug/ml, 0,312 µg/ml, 0,625 µg/ml, 1,25 µg/ml, 2,5 µg/ml, 5 µg/ml.

Un témoin sans voriconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en voriconazole et sans voriconazole.

Les données obtenues ont été moyennées pour chaque condition différente testée et présentées sous la forme d'un graphique présenté en Figure 4, où la courbe en trait plein représente la variation du taux de chitine en fonction de la concentration en voriconazole.

Une diminution du taux de chitine inférieure à 20% dans la paroi des cellules de la souche DSY296 de *C. albicans* en présence de voriconazole est observée par rapport au taux de chitine des cellules de la souche DSY296 de *C. albicans* en absence de voriconazole.

Ce résultat témoigne du phénotype résistant de la souche DSY296 de *C. albicans* vis-à-vis du voriconazole.

Ce résultat est confirmé par celui obtenu via la méthode Etest. En effet, avec cette méthode une CMI de 1 µg/ml est obtenue et l'interprétation par les tables EUCAST et CLSI permet de déterminer qu'avec une CMI respectivement supérieure à 0,12 µg/ml et supérieure à 0,5 µg/ml pour le voriconazole, cette souche est résistante à cet antifongique.

### Exemple 5 : Test de sensibilité à la micafungine de la souche TOP de C. albicans

La souche TOP de *C. albicans* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de micafungine préparés dans du milieu SC pour obtenir une concentration finale en levure de 10⁶ CFU/ml et de 1 µg/ml en micafungine.

A partir de l'inoculum la même manipulation est effectuée de manière à obtenir des cultures à 10⁶ CFU/ml et les concentrations suivantes en micafungine : 0,0009 µg/ml, 0,0019 µg/ml, 0,039 µg/ml, 0,007 µg/ml, 0,015 µg/ml, 0,031 µg/ml, 0,062 µg/ml, 0,125 µg/ml, 0,25 µg/ml, 0,5 µg/ml, 1 µg/ml.

Un témoin sans micafungine est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en micafungine et sans micafungine.

Les données obtenues ont été moyennées et pour chaque condition testée et présentées sous la forme d'un graphique présenté en Figure 5, où la courbe en trait plein représente la variation du taux de chitine en fonction de la concentration en micafungine.

Une augmentation du taux de chitine inférieure à 10% dans la paroi des cellules de la souche TOP de *C. albicans* en présence de micafungine est observée par rapport au taux de chitine des cellules de la souche TOP de *C. albicans* en absence de micafungine.

Ce résultat témoigne du phénotype résistant de la souche TOP de *C. albicans* vis-à-vis de lamicafungine.

Ce résultat est confirmé par celui obtenu via la méthode Etest. En effet, avec cette méthode une CMI de 1 µg/ml est obtenue et l'interprétation par les tables EUCAST et CLSI permet de déterminer qu'avec une CMI respectivement supérieure à 0,016 µg/ml et supérieure à 0,5 µg/ml pour la micafungine, cette souche est résistante à cet antifongique.

### Exemple 6 : Test de sensibilité au fluconazole de la souche Tg5 de C. glabrata

La souche Tg5 de *C. glabrata* a été cultivée tel que décrit dans la partie Exemples paragraphe I de manière à obtenir un inoculum à 3.10⁶ CFU/ml dans 15 ml de milieu RPMI.

1 ml de cet inoculum a ensuite été ajouté à 2 ml de solution de fluconazole préparés dans du milieu RPMI pour obtenir une concentration finale en levure de 10⁶ CFU/ml et de 128 µg/ml en fluconazole.

A partir de l'inoculum la même manipulation est effectuée de manière à obtenir des cultures à 10⁶ CFU/ml et les concentrations suivantes en fluconazole : 0,125 µg/ml, 0,25 µg/ml, 0,5 µg/ml, 1 µg/ml, 2 µg/ml, 4 µg/ml, 8 µg/ml, 16 µg/ml, 32 µg/ml, 64 µg/ml, 128 µg/ml.

Un témoin sans fluconazole est également préparé en ajoutant 2 ml de milieu RPMI.

Après incubation pendant 6,5h à 35°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 6, où la courbe en trait plein représente la variation du taux de chitine en fonction de la concentration en fluconazole.

Une diminution du taux de chitine inférieure à 20% dans la paroi des cellules de la souche Tg5 de *C. glabrata* en présence de fluconazole est observée par rapport au taux de chitine des cellules de la souche Tg5 de *C. glabrata* en absence de fluconazole.

Ce résultat témoigne du phénotype résistant de la souche Tg5 de *C*. *glabrata* vis-à-vis du fluconazole.

Ce résultat est confirmé par celui obtenu via la méthode Etest. En effet, avec cette méthode une CMI supérieure à 256 µg/ml est obtenue et l'interprétation par les tables EUCAST et CLSI permet de déterminer qu'avec une CMI supérieure à 32 µg/ml pour le fluconazole cette souche est résistante à cet antifongique.

### Exemple 7 : Test de sensibilité au voriconazole de la souche Tg5 de C. glabrata

La souche Tg5 de *C*. *glabrata* a été cultivée tel que décrit dans la partie Exemples paragraphe I de manière à obtenir un inoculum à 3.10⁶ CFU/ml dans 15 ml de milieu RPMI.

A 1 ml de cet inoculum a ensuite été ajouté à 2 ml de solution de voriconazole préparés dans du milieu RPMI pour obtenir une concentration finale en levure de 10⁶ CFU/ml et de 8 µg/ml en voriconazole.

A partir de l'inoculum la même manipulation est effectuée de manière à obtenir des cultures à 10⁶ CFU/ml et les concentrations suivantes en voriconazole : 0,007 µg/ml, 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml.

Un témoin sans voriconazole est également préparé en ajoutant 2 ml de milieu RPMI.

Après incubation pendant 6,5 h à 35°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en voriconazole et sans voriconazole.

Les données obtenues ont été moyennées pour chaque condition différente testée et présentées sous la forme d'un graphique présenté en Figure 7, où la courbe en trait plein représente la variation du taux de chitine en fonction de la concentration en voriconazole.

Une diminution du taux de chitine inférieure à 20% dans la paroi des cellules de la souche Tg5 de *C. glabrata* en présence de voriconazole est observée par rapport au taux de chitine des cellules de la souche Tg5 de *C. glabrata* en absence de voriconazole.

Ce résultat témoigne du phénotype résistant de la souche Tg5 de *C*. *glabrata* vis-à-vis du voriconazole.

Ce résultat est confirmé par celui obtenu via la méthode Etest. En effet, avec cette méthode une CMI de 8 µg/ml est obtenue et l'interprétation par la table EUCAST permet de déterminer qu'avec une CMI supérieure à 0,5 µg/ml pour le voriconazole cette souche est résistante à cet antifongique.

### Exemple 8 : Test de sensibilité au fluconazole de la souche ATCC^{®}7349 de C. tropicalis

La souche ATCC^{®}7349 de *C*. *tropicalis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de fluconazole préparés dans du milieu SC pour obtenir une concentration finale en levure de 10⁶ CFU/ml et de 16 µg/ml en fluconazole.

A partir de l'inoculum la même manipulation est effectuée de manière à obtenir des cultures à 10⁶ CFU/ml et les concentrations suivantes en fluconazole : 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml, 16 µg/ml.

Un témoin sans fluconazole est également préparé en ajoutant 2 ml de milieu SC.

Après incubation pendant 24h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 8, où la courbe en trait plein représente la variation du taux de chitine en fonction de la concentration en fluconazole.

Une augmentation d'une valeur supérieure ou égale à 20% du taux de chitine dans la paroi des cellules de la souche ATCC^{®}7349 de *C. tropicalis* en présence de fluconazole est observée par rapport au taux de chitine des cellules de la souche ATCC^{®}7349 de *C. tropicalis* en absence de fluconazole.

Le nombre de cellules pour chaque triplicata de chaque condition de concentration en fluconazole et sans fluconazole est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 8, où la courbe en trait pointillé représente la variation du nombre de cellules en fonction de la concentration en fluconazole.

Une diminution d'au moins 0,3 log du nombre de cellules de la souche ATCC^{®}7349 de *C. tropicalis* en fonction de la concentration en fluconazole dans le milieu est observée par rapport au nombre de cellules de la souche ATCC^{®}7349 de *C. tropicalis* dans une population de cellules en absence de fluconazole.

Ces résultats témoignent du phénotype sensible de la souche ATCC^{®}7349 de *C. tropicalis* vis-à-vis du fluconazole.

Ce résultat est confirmé par celui obtenu via la méthode Etest. En effet, avec cette méthode une CMI de 0,38 µg/ml est obtenue et l'interprétation par les tables EUCAST et CLSI permet de déterminer qu'avec une CMI inférieure à 2 µg/ml pour le fluconazole cette souche est sensible à cet antifongique.

### Exemple 9 : Test de sensibilité au voriconazole de la souche ATCC^{®}7349 de C. tropicalis

La souche ATCC^{®}7349 de *C*. *tropicalis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de voriconazole préparés dans du milieu SC pour obtenir une concentration finale en levure de 10⁶ CFU/ml et de 5 µg/ml en voriconazole.

A partir de l'inoculum la même manipulation est effectuée de manière à obtenir des cultures à 10⁶ CFU/ml et les concentrations suivantes en voriconazole : 0,004 µg/ml, 0,009 µg/ml, 0,019 µg/ml, 0,039 µg/ml, 0,078 µg/ml, 0,156 µg/ml, 0,312 µg/ml, 0,625 µg/ml, 1,25 µg/ml, 2,5 µg/ml, 5 µg/ml.

Un témoin sans voriconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en voriconazole et sans voriconazole.

Les données obtenues ont été moyennées pour chaque condition testée et présentées sous la forme d'un graphique présenté en Figure 9, où la courbe en trait plein représente la variation du taux de chitine en fonction de la concentration en voriconazole.

Une augmentation d'une valeur supérieure ou égale à 20% du taux de chitine dans la paroi des cellules de la souche ATCC^{®}7349 de *C. tropicalis* en présence de voriconazole est observée par rapport au taux de chitine des cellules de la souche ATCC^{®}7349 de *C. tropicalis* en absence de voriconazole.

Le nombre de cellules pour chaque triplicat de chaque condition de concentration en antifongique et sans antifongique est alors déterminé par analyse en microscopie automatisée à haut contenu d'image (HCA) tel que décrit dans la partie Exemples paragraphe II.

Les données obtenues ont été moyennées pour chaque condition testée et présentées sous la forme d'un graphique présenté en Figure 9, où la courbe en trait pointillé représente la variation du nombre de cellules en fonction de la concentration en voriconazole.

Une diminution d'au moins 0,3 log du nombre de cellules de la souche ATCC^{®}7349 de *C. tropicalis* en fonction de la concentration en voriconazole dans le milieu est observée par rapport au nombre de cellules de la souche ATCC^{®}7349 de *C. tropicalis* dans une population de cellules en absence de voriconazole.

Ces résultats témoignent du phénotype sensible de la souche ATCC^{®}7349 de *C. tropicalis* vis-à-vis du voriconazole.

Ce résultat est confirmé par celui obtenu via la méthode Etest. En effet, avec cette méthode une CMI de 0,023 µg/ml est obtenue et l'interprétation par les tables EUCAST et CLSI permet de déterminer qu'avec une CMI inférieure à 0,12 µg/ml pour le voriconazole cette souche est sensible à cet antifongique.

### Exemple 10 : Test de sensibilité à la micafungine de la souche ATCC^{®}7349 de C. tropicalis

La souche ATCC^{®}7349 de *C*. *tropicalis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de micafungine préparés dans du milieu SC pour obtenir une concentration finale en levure de 10⁶ CFU/ml et de 1 µg/ml en micafungine.

A partir de l'inoculum la même manipulation est effectuée de manière à obtenir des cultures à 10⁶ CFU/ml et les concentrations suivantes en micafungine : 0,0009 µg/ml, 0,019 µg/ml, 0,039µg/ml, 0,007 µg/ml, 0,015 µg/ml, 0,031 µg/ml, 0,062 µg/ml, 0,125 µg/ml, 0,25 µg/ml, 0,5 µg/ml, 1 µg/ml.

Un témoin sans micafungine est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour triplicat de chaque condition de concentration en micafungine et sans micafungine.

Les données obtenues ont été moyennées pour chaque condition différente testée et présentées sous la forme d'un graphique présenté en Figure 10, où la courbe en trait plein représente la variation du taux de chitine en fonction de la concentration en micafungine.

Une augmentation d'une valeur supérieure ou égale à 20% du taux de chitine dans la paroi des cellules de la souche ATCC^{®}7349 de *C. tropicalis* en présence de micafungine est observée par rapport à celui de cellules en absence de micafungine.

Le nombre de cellules pour chaque triplicat de chaque condition de concentration en micafungine et sans micafungine est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 10, où la courbe en trait pointillé représente la variation du nombre de cellules en fonction de la concentration en micafungine.

Une diminution d'au moins 0,3 log du nombre de cellules de la souche ATCC^{®}7349 de *C. tropicalis* en fonction de la concentration en micafungine dans le milieu est observée par rapport au nombre de cellules dans une population de cellules en absence de micafungine.

Ces résultats témoignent du phénotype sensible de la souche ATCC^{®}7349 de *C. tropicalis* vis-à-vis de la micafungine.

Ce résultat est confirmé par celui obtenu via la méthode Etest. En effet, avec cette méthode une CMI de 0,016 µg/ml est obtenue et l'interprétation par la table CLSI permet de déterminer qu'avec une CMI inférieure à 0,25 µg/ml pour la micafungine cette souche est sensible à cet antifongique.

### Exemple 11 : Test de sensibilité à la micafungine de la souche 13/5 de C. tropicalis

La souche 13/5 de *C. tropicalis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de micafungine préparés dans du milieu SC pour obtenir une concentration finale en levure de 10⁶ CFU/ml et de 1 µg/ml en micafungine.

A partir de l'inoculum la même manipulation est effectuée de manière à obtenir des cultures à 10⁶ CFU/ml et les concentrations suivantes en micafungine : 0,0009 µg/ml, 0,019 µg/ml, 0,039µg/ml, 0,007 µg/ml, 0,015 µg/ml, 0,031 µg/ml, 0,062 µg/ml, 0,125 µg/ml, 0,25 µg/ml, 0,5 µg/ml, 1 µg/ml.

Un témoin sans micafungine est également préparé en ajoutant 2 ml de milieu SC.

Après incubation pendant 6,5h à 30°C sous agitation à 200 rpm, 100 µl de chaque culture sont prélevées et transférées dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en micafungine et sans micafungine.

Les données obtenues ont été moyennées pour chaque condition différente testée et présentées sous la forme d'un graphique présenté en Figure 11, où la courbe en trait plein représente la variation du taux de chitine en fonction de la concentration en micafungine.

Une augmentation du taux de chitine inférieure à 10% dans la paroi des cellules de la souche 13/5 de *C. tropicalis* en présence de micafungine est observée par rapport au taux de chitine des cellules de la souche 13/5 de *C. tropicalis* en absence de micafungine Ce résultat témoigne du phénotype résistant de la souche 13/5 de *C. tropicalis* vis-à-vis de la micafungine.

Ce résultat est confirmé par celui obtenu via la méthode Etest. En effet, avec cette méthode une CMI de 1,5 µg/ml est obtenue et l'interprétation par la table CLSI permet de déterminer qu'avec une CMI supérieure à 0,5 µg/ml pour la micafungine cette souche est résistante à cet antifongique.

### Exemple 12 : Test de sensibilité au fluconazole de la souche ATCC^{®}22019 de C. parapsilosis

La souche ATCC^{®}22019 de *C. parapsilosis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de fluconazole préparés dans du milieu SC pour obtenir une concentration finale en levure de 10⁶ CFU/ml et de 16 µg/ml en fluconazole.

A partir de l'inoculum la même manipulation est effectuée de manière à obtenir des cultures à 10⁶ CFU/ml et les concentrations suivantes en fluconazole : 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml, 16 µg/ml.

Un témoin sans fluconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 12, où la courbe en trait plein représente la variation du taux de chitine en fonction de la concentration en fluconazole.

Une augmentation d'une valeur supérieure ou égale à 20% du taux de chitine dans la paroi des cellules de la souche ATCC^{®}22019 de *C. parapsilosis* en présence de fluconazole est observée par rapport au taux de chitine de cellules de la souche ATCC^{®}22019 de *C. parapsilosis* en absence de fluconazole.

Le nombre de cellules pour chaque triplicata de chaque condition de concentration en fluconazole et sans fluconazole est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 12, où la courbe en trait pointillé représente la variation du nombre de cellules en fonction de la concentration en fluconazole.

Une diminution d'au moins 0,3 log du nombre de cellules de la souche ATCC^{®}22019 de *C. parapsilosis* en fonction de la concentration en fluconazole dans le milieu est observée par rapport au nombre de cellules dans une population de cellules en absence de fluconazole.

Ces résultats témoignent du phénotype sensible de la souche ATCC^{®}22019 de *C. parapsilosis* vis-à-vis du fluconazole.

Ce résultat est confirmé par celui obtenu via la méthode Etest. En effet, avec cette méthode une CMI de 1 µg/ml est obtenue et l'interprétation par les tables EUCAST et CLSI permet de déterminer qu'avec une CMI inférieure à 2 µg/ml pour le fluconazole cette souche est sensible à cet antifongique.

### Exemple 13 : Test de sensibilité au voriconazole de la souche ATCC^{®}22019 de C. parapsilosis

La souche ATCC^{®}22019 de *C. parapsilosis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de voriconazole préparés dans du milieu SC pour obtenir une concentration finale en levure de 10⁶ CFU/ml et de 5 µg/ml en voriconazole.

A partir de l'inoculum la même manipulation est effectuée de manière à obtenir des cultures à 10⁶ CFU/ml et les concentrations suivantes en voriconazole : 0,004 µg/ml, 0,009 µg/ml, 0,019 µg/ml, 0,039 µg/ml, 0,078 µg/ml, 0,156 µg/ml, 0,312 µg/ml, 0,625 µg/ml, 1,25 µg/ml, 2,5 µg/ml, 5 µg/ml.

Un témoin sans voriconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans voriconazole.

Les données obtenues ont été moyennées pour chaque condition différente testée et présentées sous la forme d'un graphique présenté en Figure 13, où la courbe en trait plein représente la variation du taux de chitine en fonction de la concentration en voriconazole.

Une augmentation d'une valeur supérieure ou égale à 20% du taux de chitine dans la paroi des cellules de la souche ATCC^{®}22019 de *C. parapsilosis* en présence de voriconazole est observée par rapport au taux de chitine de cellules de la souche ATCC^{®}22019 de *C. parapsilosis* en absence de voriconazole.

Le nombre de cellules pour chaque triplicat de chaque condition de concentration en voriconazole et sans voriconazole est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 13, où la courbe en trait pointillé représente la variation du nombre de cellules en fonction de la concentration en voriconazole.

Une diminution d'au moins 0,3 log du nombre de cellules de la souche ATCC^{®}22019 de *C. parapsilosis* en fonction de la concentration en antifongique dans le milieu par rapport au nombre de cellules dans une population de cellules de la souche ATCC^{®}22019 de *C. parapsilosis* en absence d'antifongique.

Ces résultats témoignent du phénotype sensible de la souche ATCC^{®}22019 de *C. parapsilosis* vis-à-vis du voriconazole.

Ce résultat est confirmé par celui obtenu via la méthode Etest. En effet, avec cette méthode une CMI de 0,023 µg/ml est obtenue et l'interprétation par les tables EUCAST et CLSI permet de déterminer qu'avec une CMI inférieure à 0,12 µg/ml pour le voriconazole cette souche est sensible à cet antifongique.

### Exemple 14 : Test de sensibilité à la micafungine de la souche ATCC^{®}22019 de C. parapsilosis

La souche ATCC^{®}22019 de *C. parapsilosis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de micafungine préparés dans du milieu SC pour obtenir une concentration finale en levure de 10⁶ CFU/ml et de 1 µg/ml en micafungine.

A partir de l'inoculum la même manipulation est effectuée de manière à obtenir des cultures à 10⁶ CFU/ml et les concentrations suivantes en micafungine : 0,0009 µg/ml, 0,019 µg/ml, 0,039µg/ml, 0,007 µg/ml, 0,015 µg/ml, 0,031 µg/ml, 0,062 µg/ml, 0,125 µg/ml, 0,25 µg/ml, 0,5 µg/ml, 1 µg/ml.

Un témoin sans micafungine est également préparé en ajoutant 2 ml de milieu SC.

Après incubation pendant 24h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en micafungine et sans micafungine.

Les données obtenues ont été moyennées pour chaque condition différente testée et présentées sous la forme d'un graphique présenté en Figure 15, où la courbe en trait plein représente la variation du taux de chitine en fonction de la concentration en micafungine.

Une augmentation d'une valeur supérieure ou égale à 20% du taux de chitine dans la paroi des cellules de la souche ATCC^{®}22019 de *C. parapsilosis* en présence de micafungine est observée par rapport au taux de chitine de cellules de la souche ATCC^{®}22019 de *C. parapsilosis* en absence de micafungine.

Le nombre de cellules pour chaque triplicat de chaque condition de concentration en micafungine et sans micafungine est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 14, où la courbe en trait pointillé représente la variation du nombre de cellules en fonction de la concentration en micafungine.

Une diminution d'au moins 0,3 log du nombre de cellules de la souche ATCC^{®}22019 de *C. parapsilosis* en fonction de la concentration en micafungine dans le milieu est observée par rapport au nombre de cellules dans une population de cellules de la souche ATCC^{®}22019 de *C. parapsilosis* en absence de micafungine.

Ces résultats témoignent du phénotype sensible de la souche ATCC^{®}22019 de *C. parapsilosis* vis-à-vis de la micafungine.

Ce résultat est confirmé par celui obtenu via la méthode Etest. En effet, avec cette méthode une CMI de 0,19 µg/ml est obtenue et l'interprétation par la table CLSI permet de déterminer qu'avec une CMI inférieure à 2 µg/ml pour la micafungine cette souche est sensible à cet antifongique.

### Exemple 15 : Test de sensibilité au fluconazole de la souche 8/21 de C. parapsilosis

La souche 8/21 de *C. parapsilosis* a été cultivée tel que décrit dans la partie Exemples paragraphe I de manière à obtenir un inoculum à 3.10⁶ CFU/ml dans 15 ml de milieu SC.

1 ml de cet inoculum a ensuite été ajouté à 2 ml de solution de fluconazole préparés dans du milieu SC pour obtenir une concentration finale en levure de 10⁶ CFU/ml et de 16 µg/ml en fluconazole.

A partir de l'inoculum la même manipulation est effectuée de manière à obtenir des cultures à 10⁶ CFU/ml et les concentrations suivantes en fluconazole : 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml, 16 µg/ml.

Un témoin sans fluconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 15, où la courbe en trait plein représente la variation du taux de chitine en fonction de la concentration en fluconazole.

Une augmentation du taux de chitine inférieure à 10% dans la paroi des cellules de la souche 8/21 de *C. parapsilosis* en présence de fluconazole est observée par rapport au taux de chitine des cellules de la souche 8/21 de *C. parapsilosis* en absence de fluconazole.

Ce résultat témoigne du phénotype résistant de la souche 8/21 de *C. parapsilosis* vis-à-vis du fluconazole.

Ce résultat est confirmé par celui obtenu via la méthode Etest. En effet, avec cette méthode une CMI de 8 µg/ml est obtenue et l'interprétation par les tables EUCAST et CLSI permet de déterminer qu'avec une CMI supérieure à 4 µg/ml pour le fluconazole cette souche est résistante à cet antifongique.

### Exemple 16: Test de sensibilité au voriconazole de la souche 8/21 de C. parapsilosis

La souche 8/21 de *C. parapsilosis* a été cultivée tel que décrit dans la partie Exemples paragraphe I de manière à obtenir un inoculum à 3.10⁶ CFU/ml dans 15 ml de milieu SC.

A 1 ml de cet inoculum a ensuite été ajouté à 2 ml de solution de voriconazole préparés dans du milieu SC pour obtenir une concentration finale en levure de 10⁶ CFU/ml et de 5 µg/ml en voriconazole.

A partir de l'inoculum la même manipulation est effectuée de manière à obtenir des cultures à 10⁶ CFU/ml et les concentrations suivantes en voriconazole : 0,004 µg/ml, 0,009 µg/ml, 0,019 µg/ml, 0,039 µg/ml, 0,078 µg/ml, 0,156 µg/ml, 0,312 µg/ml, 0,625 µg/ml, 1,25 µg/ml, 2,5 µg/ml, 5 µg/ml.

Un témoin sans voriconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en voriconazole et sans voriconazole.

Les données obtenues ont été moyennées pour chaque condition différente testée et présentées sous la forme d'un graphique présenté en Figure 16, où la courbe en trait plein représente la variation du taux de chitine en fonction de la concentration en voriconazole.

Une augmentation du taux de chitine inférieure à 10% dans la paroi des cellules de la souche 8/21 de *C. parapsilosis* en présence de voriconazole est observée par rapport au taux de chitine des cellules de la souche 8/21 de *C. parapsilosis* en absence de voriconazole.

Ce résultat témoigne du phénotype résistant de la souche 8/21 de *C. parapsilosis* vis-à-vis du voriconazole.

Ce résultat est confirmé par celui obtenu via la méthode Etest et l'interprétation par la table EUCAST qui détermine qu'avec une CMI de 4 µg/ml pour le voriconazole cette souche est résistante à cet antifongique.

Ce résultat est confirmé par celui obtenu via la méthode Etest. En effet, avec cette méthode une CMI de 4 µg/ml est obtenue et l'interprétation par les tables EUCAST et CLSI permet de déterminer qu'avec une CMI respectivement supérieure à 0,12 µg/ml et supérieure à 0,5 µg/ml pour le voriconazole, cette souche est résistante à cet antifongique.

### Exemple 17 : Test de sensibilité au fluconazole de la souche ATCC^{®}6258 de C. krusei

La souche ATCC^{®}6258 de *C. krusei* a été cultivée tel que décrit dans la partie Exemples paragraphe I de manière à obtenir un inoculum à 3.10⁶ CFU/ml dans 15 ml de milieu SC.

1 ml de cet inoculum a ensuite été ajouté à 2 ml de solution de fluconazole préparés dans du milieu SC pour obtenir une concentration finale en levure de 10⁶ CFU/ml et de 128 µg/ml en fluconazole.

A partir de l'inoculum la même manipulation est effectuée de manière à obtenir des cultures à 10⁶ CFU/ml et les concentrations suivantes en fluconazole : 0,125 µg/ml, 0,25 µg/ml, 0,5 µg/ml, 1 µg/ml, 2 µg/ml, 4 µg/ml, 8 µg/ml, 16 µg/ml, 32 µg/ml, 64 µg/ml, 128 µg/ml.

Un témoin sans fluconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure17, où la courbe en trait plein représente la variation du taux de chitine en fonction de la concentration en fluconazole.

Une augmentation du taux de chitine inférieure à 10% dans la paroi des cellules de la souche ATCC^{®}6258 de *C. krusei* en présence de fluconazole est observée par rapport au taux de chitine des cellules de la souche ATCC^{®}6258 de *C. krusei* en absence de fluconazole.

Ce résultat témoigne du phénotype résistant de la souche ATCC^{®}6258 de *C. krusei* vis-à-vis du fluconazole.

Ce résultat est confirmé par celui obtenu via la méthode Etest. En effet, avec cette méthode une CMI de 64 µg/ml est obtenue et l'interprétation par la table EUCAST permet de déterminer qu'avec une CMI supérieure à 32 µg/ml pour le fluconazole cette souche est résistante à cet antifongique. L'interprétation est obtenue dans ce cas par extrapolation des données EUCAST et CLSI du *C*. *glabrata.*

### Exemple 18 : Test de sensibilité au voriconazole de la souche ATCC^{®}6258 de C. krusei

La souche ATCC^{®}6258 de *C*. *krusei* a été cultivée tel que décrit dans la partie Exemples paragraphe I de manière à obtenir un inoculum à 3.10⁶ CFU/ml dans 15 ml de milieu RPMI.

1 ml de cet inoculum a ensuite été ajouté à 2 ml de solution de voriconazole préparés dans du milieu RPMI pour obtenir une concentration finale en levure de 10⁶ CFU/ml et de 8 µg/ml en voriconazole.

A partir de l'inoculum la même manipulation est effectuée de manière à obtenir des cultures à 10⁶ CFU/ml et les concentrations suivantes en voriconazole : 0,007 µg/ml, 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml.

Un témoin sans voriconazole est également préparé en ajoutant 2 ml de milieu RPMI.

Après incubation pendant 24h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en voriconazole et sans voriconazole.

Les données obtenues ont été moyennées pour chaque condition différente testée et présentées sous la forme d'un graphique présenté en Figure 18, où la courbe en trait plein représente la variation du taux de chitine en fonction de la concentration en voriconazole.

Une augmentation d'une valeur supérieure ou égale à 20% du taux de chitine dans la paroi des cellules de la souche ATCC^{®}6258 de *C. krusei* en présence de voriconazole est observée par rapport au taux de chitine de cellules de la souche ATCC^{®}6258 de *C. krusei* en absence de voriconazole.

Le nombre de cellules pour chaque triplicata de chaque condition de concentration en voriconazole et sans voriconazole est alors déterminé par analyse en microcopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 18, où la courbe en trait pointillé représente la variation du nombre de cellules en fonction de la concentration en voriconazole.

Une diminution de moins de 0,3 log du nombre de cellules de la souche ATCC^{®}6258 de *C. krusei* en fonction de la concentration en antifongique dans le milieu par rapport au nombre de cellules dans une population de cellules de la souche ATCC^{®}6258 de *C*. *krusei* en absence d'antifongique.

Ce résultat témoigne du phénotype intermédiaire de la souche ATCC^{®}6258 de *C*. *krusei* vis-à-vis du voriconazole.

Ce résultat est confirmé par celui obtenu via la méthode Etest. En effet, avec cette méthode une CMI à 1 µg/ml est obtenue et l'interprétation par la table CLSI permet de déterminer qu'avec une CMI égale à 1 µg/ml pour le voriconazole cette souche est intermédiaire à cet antifongique.

### Exemple 19 : Test de sensibilité au fluconazole de la souche GRE32 de C. krusei

La souche GRE32 de *C. krusei* a été cultivée tel que décrit dans la partie Exemples paragraphe I de manière à obtenir un inoculum à 3.10⁶ CFU/ml dans 15 ml de milieu SC.

1 ml de cet inoculum a ensuite été ajouté à 2 ml de solution de fluconazole préparés dans du milieu SC pour obtenir une concentration finale en levure de 10⁶ CFU/ml et de 128 µg/ml en fluconazole.

A partir de l'inoculum la même manipulation est effectuée de manière à obtenir des cultures à 10⁶ CFU/ml et les concentrations suivantes en fluconazole : 0,125 µg/ml, 0,25 µg/ml, 0,5 µg/ml, 1 µg/ml, 2 µg/ml, 4 µg/ml, 8 µg/ml, 16 µg/ml, 32 µg/ml, 64 µg/ml, 128 µg/ml.

Un témoin sans fluconazole est également préparé en ajoutant 2 ml de milieu SC.

Après incubation pendant 6,5h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en fluconazole et sans fluconazole.

Les données obtenues ont été moyennées pour chaque condition différente testée et présentées sous la forme d'un graphique présenté en Figure 19, où la courbe en trait plein représente la variation du taux de chitine en fonction de la concentration en fluconazole.

Une diminution du taux de chitine inférieure à 20% dans la paroi des cellules de la souche GRE32 de *C. krusei* en présence de fluconazole est observée par rapport au taux de chitine des cellules de la souche GRE32 de *C. krusei* en absence de fluconazole.

Ce résultat témoigne du phénotype résistant de la souche GRE32 de *C. krusei* vis-à-vis du fluconazole.

Ce résultat est confirmé par celui obtenu via la méthode Etest. En effet, avec cette méthode une CMI de 64 µg/ml est obtenue et l'interprétation par la table EUCAST permet de déterminer qu'avec une CMI supérieure à 32 µg/ml pour le fluconazole cette souche est résistante à cet antifongique. L'interprétation est obtenue par extrapolation des données EUCAST et CLSI du C. *glabrata.*

### Exemple 20 : Test de sensibilité au voriconazole de la souche GRE32 de C. krusei

La souche GRE32 de *C. krusei* a été cultivée tel que décrit dans la partie Exemples paragraphe I de manière à obtenir un inoculum à 3.10⁶ CFU/ml dans 15 ml de milieu SC.

1 ml de cet inoculum a ensuite été ajouté à 2 ml de solution de voriconazole préparés dans du milieu RPMI pour obtenir une concentration finale en levure de 10⁶ CFU/ml et de 8 µg/ml en voriconazole.

A partir de l'inoculum la même manipulation est effectuée de manière à obtenir des cultures à 10⁶ CFU/ml et les concentrations suivantes en voriconazole : 0,007 µg/ml, 0,015 µg/ml, 0,031 µg/ml, 0,062 µg/ml, 0,125 µg/ml, 0,25 µg/ml, 0,5 µg/ml, 1 µg/ml, 2 µg/ml, 4 µg/ml, 8 µg/ml.

Un témoin sans voriconazole est également préparé en ajoutant 2 ml de milieu RPMI.

Après incubation pendant 24h à 30°C sous agitation à 200 rpm, 100 µl de chaque culture sont prélevés et transférés dans des plaques 96 puits. Cette opération est effectuée en triplicat, et 2,5 µl de CFW sont ensuite ajoutés dans chaque puits.

Le taux de chitine dans la paroi cellulaire des levures est ensuite mesuré par analyse en microscopie automatisée à haut contenu d'image (HCA) tel que décrit dans la partie Exemples paragraphe II, pour chaque triplicat de chaque condition de concentration en voriconazole et sans voriconazole.

Les données obtenues ont été moyennées pour chaque condition différente testée et présentées sous la forme d'un graphique présenté en Figure 20, où la courbe en trait plein représente la variation du taux de chitine en fonction de la concentration en voriconazole.

Une augmentation du taux de chitine supérieure ou égale à 20% dans la paroi des cellules de la souche GRE32 de *C. krusei* en présence de voriconazole est observée par rapport au taux de chitine de cellules de la souche GRE32 de *C. krusei* en absence de voriconazole.

Le nombre de cellules pour chaque triplicat de chaque condition de concentration en voriconazole et sans voriconazole est alors déterminé par analyse en microscopie automatisée à haut contenu d'image (HCA) tel que décrit dans la partie Exemples paragraphe II.

Les données obtenues ont été moyennées pour chaque condition différente testée et présentées sous la forme d'un graphique présenté en Figure 20, où la courbe en trait pointillé représente la variation du nombre de cellules en fonction de la concentration en voriconazole.

Une diminution de moins de 0,3 log du nombre de cellules de la souche GRE32 de *C. krusei* en fonction de la concentration en voriconazole dans le milieu par rapport au nombre de cellules dans une population de cellules de la souche GRE32 de *C. krusei* en absence voriconazole.

Ces résultats témoignent du phénotype intermédiaire de la souche GRE32 de *C. krusei* vis-à-vis du voriconazole.

Ce résultat est confirmé par celui obtenu via la méthode Etest. En effet, avec cette méthode une CMI de 1 µg/ml est obtenue et l'interprétation par la table CLSI permet de déterminer qu'avec une CMI égale à 1 µg/ml pour le voriconazole cette souche est intermédiaire à cet antifongique.

### B- EXEMPLES CHAMPIGNONS PLURICELLULAIRES

### I - Culture de champignon pluricellulaire en présence d'antifongique

Les souches de champignon pluricellulaire sont préalablement incubées à 30°C pendant une nuit dans un milieu d'extrait de levure- peptone - dextrose (YPD) (1% de bacto peptone, 0.5% d'extrait de levure, 2% de glucose, 1.5% d'agar).

Des conidies de champignon pluricellulaire sont ensuite prélevées des boites de milieu YPD et mises en suspension dans une solution saline à 0.9% NaCl dans laquelle la concentration cellulaire a été estimée par microscopie optique en utilisant des lames de numération Kova Slide.

Une dilution est réalisée pour obtenir un inoculum final à 10⁶ CFU/ml, dans un milieu complet synthétique (SC) à pH 7 (2% de glucose, 0.5% d'ammonium sulfate, 0.17% de base azoté de levure, 0.2% de mélange synthétique complet et 10% d'HEPES 1,5M pH 7.2) ou un milieu RPMI selon le champignon parmi les genres *Aspergillus, Fusarium, Scedosporium, Lichteimia, Rhizopus, Rhizomucor, Mucor, Paecylomyces,* et les espèces *Aspergillus fumigatus, Aspergillus flavus, Aspergillus niger, Aspergillus nidulans, Aspergillus terreus, Fusarium solani, Fusarium oxysporum, Scedosporium apiospermum, Scedosporium prolificans, Mucor racemosa, Lichteimia corymbifera, Rhizopus oryzae, Rhizomucor pusillus,* de manière à obtenir une concentration finale en levure de 10⁶ CFU/ml et la concentration en antifongique voulue selon le tableau 3.

**Tableau 3 : Gradients de concentration (µg/ml) de fluconazole, de posaconazole, du voriconazole, de l'iraconazole, de l'isavuconazole pour la classe des azolés et de la micafungine, anidulafungine et de la caspofungine pour la classe des échinocandines et amphotéricine B et nystatine pour la classe des polyènes, pour les espèces de champignons pluricellulaires.**

| Antifongique | Concentration (µg/ml) | | | | | | | | | | |
|---|---|---|---|---|---|---|---|---|---|---|---|
| fluconazole posaconazole voriconazole isavuconazole amphotéricine B nystatine | 8 | 4 | 2 | 1 | 0,5 | 0,25 | 0,125 | 0,062 | 0,031 | 0,015 | 0,007 |
| micafungine anidulafungine caspofungine | 2 | 1 | 0,5 | 0,25 | 0,125 | 0,062 | 0,031 | 0,015 | 0,007 | 0,003 | 0,0017 |

Après homogénéisation, les cultures sont placées à 30°C à 200 rpm et incubées pendant une durée de 4h, 6h ou de 24h.

### II - Analyse en microscopie automatisée à haut contenu d'images (HCA)

Après incubation, la culture de champignons obtenue ci-dessus est transférée en triplicat dans des plaques de 96 puits, et 2.5 µl de Calcofluor-White (CFW) ont été ajoutés dans chaque puits afin de marquer la chitine des parois cellulaires des conidies et des hyphes végétatifs de germination.

Une étape d'acquisition d'images par microscopie à fluorescence automatisée (ScanR screening station, Olympus), en utilisant un objectif x40 et un filtre pour le CFW, permet d'obtenir 30 images par puits afin de capturer assez de conidies et hyphes végétatifs de germination pour réaliser des mesures de la longueur des hyphes germinatifs et la proportion de conidies donnant des hyphes germinatifs. L'analyse de ces images est réalisée par le logiciel ScanR analysis software, Olympus. Un traitement du bruit de fond est d'abord réalisé afin d'améliorer le contraste entre la fluorescence des conidies et des hyphes germinatifs et le bruit de fond. Un seuil en pixel est alors défini. La segmentation des éléments fluorescents présents sur chacune des images acquises est ensuite réalisée grâce à un algorithme prédéfini dans le logiciel permettant sur la base de l'intensité de fluorescence de chaque élément de déterminer la limite de cet élément. Un paramètre de taille est ensuite appliqué pour sélectionner les conidies ainsi que les hyphes germinatifs et de mesurer la longueur de ces hyphes germinatifs.

Pour chaque élément correspondant alors à une conidie ou un hyphe germinatif, il est donc possible, à partir de ces éléments définis précédemment, de définir l'intensité de fluorescence pour chacun de ces éléments rapporté à leur surface, cette intensité étant directement corrélée avec le contenu en chitine de leur paroi.

Ces données sont ensuite traitées par un logiciel (GraphPad Prism) pour réaliser une représentation graphique de la variation du taux chitine de la paroi des conidies et des hyphes végétatifs et la variation de la longueur des hyphes végétatifs en fonction de la concentration en antifongique, pour chaque couple souche de champignon pluricellulaire /antifongique.

Lorsqu'une augmentation du taux de chitine d'une valeur inférieure à 10% ou une diminution du taux de chitine inférieure à 20% ou un taux de chitine stationnaire sont observés par rapport au taux de chitine d'une population de cellules dudit champignon en absence d'antifongique, la souche est de phénotype résistant.

Lorsqu'une augmentation du taux de chitine d'une valeur de 10% à une valeur inférieure à 20% est observée par rapport au taux de chitine d'une population de cellules dudit champignon en absence d'antifongique, la souche est de phénotype intermédiaire.

Lorsqu'une augmentation du taux de chitine supérieure ou égale à 20% est observée par rapport au taux de chitine d'une population de cellules dudit champignon en absence d'antifongique, ainsi qu'une diminution de la longueur des hyphes germinatifs de moins de 10% ou une longueur des hyphes germinatifs stationnaire par rapport à la longueur des hyphes germinatifs dans une population de cellules dudit champignon en absence d'antifongique, la souche est de phénotype intermédiaire.

Lorsqu'une augmentation du taux de chitine supérieure ou égale à 20% est observée par rapport au taux de chitine d'une population de cellules dudit champignon en absence d'antifongique, ainsi qu'une diminution qu'une diminution de la longueur des hyphes germinatifs d'au moins 10% par rapport au nombre de cellules dans une population de cellules dudit champignon en absence d'antifongique, la souche est de phénotype sensible.

La considération d'un paramètre autre que le dénombrement cellulaire pour la détermination de la sensibilité d'une souche de champignon constitue l'originalité de cette invention, par rapport aux tests existants basés sur la mesure de la croissance des champignons, lesquels sont soumis à la subjectivité du lecteur.

### III - Analyse par la méthode comparative Etest^{®} commercialisé par Biomérieux

La méthode Etest^{®} est un test commercial de détermination de la sensibilité d'une souche, le plus fréquemment utilisé en routine dans les laboratoires cliniques de mycologie.

Les résultats obtenus par HCA sont comparés à ceux obtenus avec ce test de sensibilité.

Les tests Etest^{®} sont réalisés selon les instructions du fabricant. Après étalement d'un inoculum standardisé Mac Farland 0.5 (équivalent de 10⁸ CFU/ml) sur des boites de milieu RPMI, celles-ci ont été incubées 24h à 35°C, afin de pouvoir déterminer la concentration minimale d'inhibition (CMI).

Les résultats des CMI obtenues sont interprétés selon les tables de l'EUCAST (European Committee on Antimicrobial Susceptibility Testing http://www.eucast.org/fileadmin/src/media/PDFs/EUCAST_files/AFST/Clinical_breakpoints/ Antifungal_breakpoints_v_8.0_November_2015.pdf) qui répertorient les seuils cliniques (CBP) pour les espèces les plus communes de champignons pluricellulaires du genre *Aspergillus* en fonction de différents antifongiques. Il n'existe pas de critères d'interprétation pour les autres genres de champignons pluricellulaires parmi lesquels *Fusarium, Scedosporium, Lichteimia, Rhizopus, Rhizomucor, Mucor, Paecylomyces.*

La CMI permet ainsi de déterminer la sensibilité d'une souche à un antifongique. Les CBP ou seuils cliniques permettent d'interpréter cette CMI et de prédire un échec de traitement chez le patient. Les CBP permettent d'établir les catégories : sensible, intermédiaire et résistante.

Si la valeur de la CMI est dans la catégorie sensible, la probabilité d'échec du traitement est faible. Cette probabilité augmente dans les catégories Intermédiaire ou Résistant

Ces tables permettent de détecter les résistances acquises qui résultent principalement de la sélection de mutants soumis à la pression des médicaments chez les patients. Elles sont ainsi spécifiques des souches et ne devraient pas être confondues avec des résistances intrinsèques qui sont spécifiques des espèces.

## Revendications

1. Utilisation de la variation éventuelle du taux de chitine dans une population de cellules d'une souche de champignon pour déterminer le degré de sensibilité de ladite souche de champignon vis-à-vis d'un antifongique, ladite variation étant déterminée par rapport au taux de chitine d'une population de cellules de ladite souche de champignon en absence d'antifongique, et de la variation éventuelle du nombre de cellules d'une population de cellules de ladite souche de champignon, ladite variation éventuelle du nombre de cellules étant déterminée par rapport au nombre de cellules d'une population de cellules de ladite souche de champignon en absence d'antifongique, et dans laquelle le degré de sensibilité correspond soit à un phénotype sensible, soit à un phénotype résistant, soit à un phénotype intermédiaire de ladite souche de champignon vis-à-vis d'un antifongique, ledit champignon étant un champignon unicellulaire :
- dans laquelle le phénotype résistant de ladite souche de champignon vis-à-vis dudit antifongique est déterminé par une augmentation du taux de chitine inférieure à 10 % ou une diminution du taux de chitine ou un taux de chitine stationnaire, par rapport au taux de chitine d'une population de cellules de ladite souche de champignon en absence d'antifongique, et dans laquelle le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100% ; ou
- dans laquelle le phénotype intermédiaire de ladite souche de champignon vis-à-vis dudit antifongique est déterminé
soit par une augmentation du taux de chitine de 10% à une valeur inférieure à 20%, par rapport au taux de chitine d'une population de cellules de ladite souche de champignon en absence d'antifongique,
soit par une augmentation du taux de chitine supérieure ou égale à 20% par rapport au taux de chitine d'une population de cellules de ladite souche de champignon en absence d'antifongique, et par une diminution du nombre de cellules de moins de 0,3 log ou un nombre de cellules stationnaire, par rapport au nombre de cellules d'une population de cellules de ladite souche de champignon en absence d'antifongique, et dans laquelle le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100% et éventuellement dans laquelle le seuil minimal de diminution du nombre de cellules dans ladite population de cellules de ladite souche de champignon est d'au moins 0,3 log, notamment 0,4 ; 0,5 ; 0,6 ; 0,7 ; 0,8 ; 0,9 ; 1 ; 1,1 ; 1,2 ; 1,3 ; 1,4 ; 1,5 ; 1,6 ; 1,7 ; 1,8 ; 1,9 ; 2 ; 2,1 ; 2,2 ; 2,3 ; 2,4 ; 2,5 ; 2,6 ; 2,7 ; 2,8 ; 2,9 ; 3; ou
- dans laquelle le phénotype sensible de ladite souche de champignon vis-à-vis dudit antifongique est déterminé par une augmentation du taux de chitine supérieure ou égale à 20% par rapport au taux de chitine d'une population de cellules de ladite souche de champignon en absence d'antifongique, et par une diminution du nombre de cellules d'au moins 0,3 log par rapport au nombre de cellules d'une population de cellules de ladite souche de champignon en absence d'antifongique, et dans laquelle le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100% et dans laquelle le seuil minimal de diminution du nombre de cellules dans ladite population de cellules de ladite souche de champignon est d'au moins 0,3 log, notamment 0,4 ; 0,5 ; 0,6 ; 0,7 ; 0,8 ; 0,9 ; 1 ; 1,1 ; 1,2 ; 1,3 ; 1,4 ; 1,5 ; 1,6 ; 1,7 ; 1,8 ; 1,9 ; 2 ; 2,1 ; 2,2 ; 2,3 ; 2,4 ; 2,5 ; 2,6 ; 2,7 ; 2,8 ; 2,9 ; 3.

2. Utilisation de la variation éventuelle du taux de chitine dans une population de cellules d'une souche de champignon pour déterminer le degré de sensibilité de ladite souche de champignon vis-à-vis d'un antifongique, ladite variation étant déterminée par rapport au taux de chitine d'une population de cellules de ladite souche de champignon en absence d'antifongique , et de la variation éventuelle de la longueur de l'hyphe végétatif de germination dans ladite population de cellules de ladite souche de champignon, pour déterminer le degré de sensibilité de ladite souche de champignon vis-à-vis d'un antifongique, ladite variation de la longueur de l'hyphe végétatif de germination étant déterminée par rapport à la longueur de l'hyphe végétatif de germination d'une population de cellules de ladite souche de champignon en absence d'antifongique, dans laquelle ledit champignon est un champignon pluricellulaire, et dans laquelle le degré de sensibilité correspond soit à un phénotype sensible, soit à un phénotype résistant, soit à un phénotype intermédiaire de ladite souche de champignon vis-à-vis d'un antifongique.

3. Utilisation de la variation éventuelle du taux de chitine dans une population de cellules d'une souche de champignon selon la revendication 2, ledit champignon étant un champignon pluricellulaire :
- dans laquelle le phénotype résistant de ladite souche de champignon vis-à-vis dudit antifongique est déterminé par une augmentation du taux de chitine inférieure à 10 % ou par une diminution du taux de chitine ou par un taux de chitine stationnaire, par rapport au taux de chitine d'une population de cellules de ladite souche de champignon en absence d'antifongique ; ou
- dans laquelle le phénotype intermédiaire de ladite souche de champignon vis-à-vis d'un antifongique est déterminé par une augmentation du taux de chitine de 10% à une valeur inférieure à 20%, par rapport au taux de chitine d'une population de cellules de ladite souche de champignon en absence d'antifongique ; ou
- dans laquelle le phénotype sensible ou intermédiaire de ladite souche de champignon vis-à-vis d'un antifongique est déterminé par une augmentation du taux de chitine supérieure ou égale à 20%, par rapport au taux de chitine d'une population de cellules de ladite souche de champignon en absence d'antifongique, et par la variation éventuelle de la longueur de l'hyphe végétatif de germination dans ladite population de cellules par rapport la longueur dudit hyphe dans une population de cellules de ladite souche de champignon en absence d'antifongique, ladite variation éventuelle étant une diminution de la longueur dudit hyphe ou une longueur stationnaire dudit hyphe, par rapport à la longueur de l'hyphe végétatif de germination d'une population de cellules de ladite souche de champignon en absence d'antifongique.

4. Utilisation de la variation éventuelle du taux de chitine dans une population de cellules d'une souche de champignon pour déterminer le degré de sensibilité de ladite souche de champignon vis-à-vis d'un antifongique selon les revendications 1 à 3, dans laquelle la variation éventuelle du taux de chitine est déterminée par fluorescence, notamment par une méthode de microscopie à fluorescence, plus particulièrement par la microscopie automatisée à haut contenu d'images (HCA) et dans laquelle la variation éventuelle du taux de chitine est déterminée notamment à l'aide d'un marqueur fluorescent, notamment le Calcofluor White.

5. Utilisation de la variation éventuelle du taux de chitine dans une population de cellules d'une souche de champignon pour déterminer le degré de sensibilité de ladite souche de champignon vis-à-vis d'un antifongique selon les revendications 1 à 4, dans laquelle l'antifongique est choisi parmi le groupe des antifongiques dépourvu des polyènes tels que l'amphotéricine B et la nystatine, et dépourvu des antifongiques à action directe sur la chitine, plus particulièrement :
- parmi le groupe comprenant ou étant constitué des antifongiques azolés tels que le fluconazole, le voriconazole, le posaconazole, l'itraconazole et l'isavuconazole, ou autres molécules inhibitrices de la synthèse de l'ergosterol tels que les allylamines dont la terbinafine ou morpholines ;
- ou parmi les échinocandines, l'échinocandine étant plus particulièrement choisie parmi le groupe comprenant ou étant constitué de l'anidulafungine, de la caspofungine et de la micafungine.

6. Utilisation de la variation éventuelle du taux de chitine dans une population de cellules d'une souche de champignon pour déterminer le degré de sensibilité de ladite souche de champignon vis-à-vis d'un antifongique selon les revendications 1, 4-5, dans laquelle le champignon est une levure, et dans laquelle la population de cellules de levures est choisie parmi le groupe comprenant ou étant constitué des genres *Candida, Cryptococcus, Saccharomyces, Trichosporon, Rhodotorula, Malassezia,* et plus particulièrement dans laquelle la levure du genre *Candida* est choisie parmi le groupe comprenant ou étant constitué des espèces : *Candida albicans, C. glabatra, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

7. Procédé de détermination du degré de sensibilité d'une population de cellules d'une souche de champignon unicellulaire vis-à-vis d'un antifongique comprenant une étape de détermination de la variation éventuelle du taux de chitine dans la population de cellules de ladite souche de champignon en présence d'antifongique par rapport au taux de chitine d'une population de cellules de ladite souche de champignon en absence d'antifongique, et une étape de détermination de la variation éventuelle du nombre de cellules dans ladite population de cellules de ladite souche de champignon en présence d'antifongique, ladite variation du nombre de cellules étant déterminée par rapport au nombre de cellules d'une population de cellules de ladite souche de champignon en absence d'antifongique, dans lequel ledit degré de sensibilité correspond à un phénotype sensible, résistant ou intermédiaire :
- dans lequel ledit degré de sensibilité correspond à un phénotype résistant et ledit procédé comprend une étape de détermination de la variation éventuelle du taux de chitine dans la population de cellules de ladite souche de champignon en présence d'antifongique, ladite variation éventuelle étant une augmentation du taux de chitine inférieure à 10% ou une diminution du taux de chitine ou un taux de chitine stationnaire, par rapport au taux de chitine d'une population de cellules de ladite souche de champignon en absence d'antifongique, et dans lequel le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100%; ou
- dans lequel ledit degré de sensibilité correspond à un phénotype intermédiaire et ledit procédé comprend
soit une étape de détermination de la variation du taux de chitine dans la population de cellules de ladite souche de champignon en présence d'antifongique, ladite variation étant une augmentation du taux de chitine de 10% à une valeur inférieure à 20%, par rapport au taux de chitine d'une population de cellules de ladite souche de champignon en absence d'antifongique,
soit une étape de détermination de la variation du taux de chitine dans la population de cellules de ladite souche de champignon en présence d'antifongique, ladite variation étant une augmentation du taux de chitine supérieure ou égale à 20%, par rapport au taux de chitine d'une population de cellules de ladite souche de champignon en absence d'antifongique, et une étape de détermination de la variation éventuelle du nombre de cellules de ladite souche de champignon en présence d'antifongique, ladite variation étant une diminution du nombre de cellules de moins de 0,3 log ou un nombre de cellules stationnaire, par rapport au nombre de cellules dans une population de cellules de ladite souche de champignon en absence d'antifongique, et dans lequel le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100% et éventuellement dans lequel le seuil minimal de diminution du nombre de cellules dans ladite population de cellules de ladite souche de champignon est d'au moins 0,3 log, notamment 0,4 ; 0,5 ; 0,6 ; 0,7 ; 0,8 ; 0,9 ; 1 ; 1,1 ; 1,2 ; 1,3 ; 1,4 ; 1,5 ; 1,6 ; 1,7 ; 1,8 ; 1,9 ; 2 ; 2,1 ; 2,2 ; 2,3 ; 2,4 ; 2,5 ; 2,6 ; 2,7 ; 2,8 ; 2,9 ; 3; ou
- dans lequel ledit degré de sensibilité correspond à un phénotype sensible et ledit procédé comprend une étape de détermination de la variation du taux de chitine dans la population de cellules de ladite souche de champignon en présence d'antifongique, ladite variation étant une augmentation du taux de chitine supérieure ou égale à 20%, par rapport au taux de chitine d'une population de cellules de ladite souche de champignon en absence d'antifongique et une étape de détermination de la variation du nombre de cellules dans ladite population de cellules de ladite souche de champignon en présence d'antifongique, ladite variation étant une diminution du nombre de cellules d'au moins 0,3 log par rapport au nombre de cellules d'une population de cellules de ladite souche de champignon en absence d'antifongique, et dans lequel le seuil minimal de cellules dans ladite population de cellules de ladite souche de champignon présentant une augmentation du taux de chitine est d'au moins 10%, notamment 20%, 25%, 30%, 35%, 40%, 45%, 50%, 55%, 60%, 65%, 70%, 75%, 80%, 85%, 90%, 95% et 100% et dans lequel le seuil minimal de diminution du nombre de cellules dans ladite population de cellules de ladite souche de champignon est d'au moins 0,3 log, notamment 0,4 ; 0,5 ; 0,6 ; 0,7 ; 0,8 ; 0,9 ; 1 ; 1,1 ; 1,2 ; 1,3 ; 1,4 ; 1,5 ; 1,6 ; 1,7 ; 1,8 ; 1,9 ; 2 ; 2,1 ; 2,2 ; 2,3 ; 2,4 ; 2,5 ; 2,6 ; 2,7 ; 2,8 ; 2,9 ; 3.

8. Procédé de détermination du degré de sensibilité d'une population de cellules d'une souche de champignon vis-à-vis d'un antifongique selon la revendication 7 dans lequel la variation éventuelle du taux de chitine dans la population de cellules de ladite souche de champignon en présence d'antifongique par rapport au taux de chitine d'une population de cellules de ladite souche de champignon en absence d'antifongique est déterminée par méthode de fluorescence, notamment par une méthode de microscopie à fluorescence, plus particulièrement par la microscopie automatisée à haut contenu d'images (HCA) et comprenant notamment une étape de marquage fluorescent des cellules de ladite souche de champignon permettant la détermination de la variation éventuelle du taux de chitine, ladite étape de marquage fluorescent des cellules de ladite souche de champignon étant notamment effectuée à l'aide de Calcofluor White.

9. Procédé de détermination du degré de sensibilité d'une population de cellules d'une souche de champignon vis-à-vis d'un antifongique selon les revendications 7 à 8, dans lequel ledit antifongique est choisi parmi le groupe des antifongiques dépourvu des polyènes tels que l'amphotéricine B et la nystatine, et dépourvu des antifongiques à action directe sur la chitine, plus particulièrement :
- parmi le groupe comprenant ou étant constitué des antifongiques azolés tels que le fluconazole, le voriconazole, le posaconazole, l'itraconazole et l'isavuconazole, ou autres molécules inhibitrices de la synthèse de l'ergosterol tels que les allylamines dont la terbinafine ou morpholines ;
- ou parmi les échinocandines, l'échinocandine étant plus particulièrement choisie parmi le groupe comprenant ou étant constitué de l'anidulafungine, de la caspofungine et de la micafungine.

10. Procédé de détermination du degré de sensibilité d'une population de cellules d'une souche de champignon vis-à-vis d'un antifongique selon les revendications 7 à 9, dans lequel ledit champignon unicellulaire est une levure, et dans lequel la population de cellules de levures est choisie parmi le groupe comprenant ou étant constitué des genres *Candida, Cryptococcus, Saccharomyces, Trichosporon, Rhodotorula, Malassezia,* et plus particulièrement dans lequel la population de cellules de levures du genre *Candida* est choisie parmi le groupe comprenant ou étant constitué des espèces : *Candida albicans, C. glabatra, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

11. Procédé de détermination du degré de sensibilité d'une population de cellules d'une souche de champignon vis-à-vis d'un antifongique selon les revendications 7 à 10 comprenant une étape de mise en présence dudit antifongique et des cellules de ladite souche de champignon, avant l'étape de détermination de la variation éventuelle du taux de chitine, ladite étape de mise en présence est inférieure ou égale à 48h, notamment d'une durée de 6,5h à 24h, et dans lequel ladite étape de mise de présence est effectuée à une température de 30 à 35°C.

12. Procédé de détermination du degré de sensibilité d'une population de cellules d'une souche de champignon, ledit champignon étant un champignon unicellulaire, vis-à-vis d'un antifongique selon les revendications 7 à 11, comprenant :
**a.** une étape de mise en présence des cellules de ladite souche de champignon avec un gradient de concentrations d'antifongique variant de 0,0009 à 130 µg/ml, à une température de 30 à 35°C pendant une durée inférieure ou égale à 48h, notamment une durée de 6,5h à 24h, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique,
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de dénombrement des cellules dans le mélange de cellules de ladite souche de champignon marquées au Calcofluor White et d'antifongique; puis d'
**f.** une étape de détermination de la variation éventuelle du nombre de cellules en fonction de la concentration en antifongique dans la population de cellules de ladite souche de champignon, par rapport au nombre de cellules dans une population de cellules de ladite souche de champignon en absence d'antifongique ;
lorsque ladite variation du taux de chitine de la susdite étape d. est une augmentation du taux de chitine inférieure à 10% ou une diminution du taux de chitine notamment inférieure à 20% ou un taux stationnaire il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation du nombre de cellules de la susdite étape f. est une diminution de moins de 0,3 log, il est déduit que ladite souche de champignon est de phénotype intermédiaire ;
lorsque ladite variation du taux de chitine de la susdite étape d. est une augmentation d'une valeur supérieure ou égale à 20%, et que ladite variation du nombre de cellules est une diminution d'au moins 0,3 log, il est déduit que ladite souche de champignon est de phénotype sensible.

13. Procédé de détermination du degré de sensibilité d'une population de cellules d'une souche de champignon vis-à-vis d'un antifongique, dans lequel ledit champignon est un champignon pluricellulaire, comprenant une étape de détermination de la variation éventuelle du taux de chitine dans la population de cellules de ladite souche de champignon en présence dudit antifongique par rapport au taux de chitine d'une population de cellules de ladite souche de champignon en absence d'antifongique, et une étape de détermination de la variation éventuelle de la longueur de l'hyphe végétatif de germination dans ladite population de cellules de champignon en présence dudit antifongique, ladite variation éventuelle de la longueur de l'hyphe végétatif de germination étant déterminée par rapport à la longueur de l'hyphe végétatif de germination dans une population de cellules de ladite souche de champignon en absence d'antifongique.

14. Procédé de détermination du degré de sensibilité d'une population de cellules d'une souche de champignon, ledit champignon étant un champignon pluricellulaire, vis-à-vis d'un antifongique selon la revendication 13, comprenant :
**a.** une étape de mise en présence des cellules de ladite souche de champignon avec un gradient de concentrations d'antifongique variant de 0,0009 à 130 µg/ml, pendant une durée inférieure ou égale à 48h, notamment pendant une durée de 6,5h à 24h, à une température de 30 à 35°C, pour obtenir un mélange de cellules de ladite souche de champignon avec l'antifongique ;
**b.** une étape d'addition du marqueur fluorescent Calcofluor White au mélange de cellules de ladite souche de champignon et d'antifongique obtenu précédemment, pour obtenir un mélange de cellules de ladite souche de champignon marquées au Calcofluor White et d'antifongique,
**c.** une étape de quantification du taux de chitine par microscopie à fluorescence automatisée à haut contenu d'images, des cellules de ladite souche de champignon marquées dans le mélange obtenu à l'étape précédente ;
**d.** une étape de détermination de la variation éventuelle du taux de chitine dans le mélange de cellules de ladite souche de champignon marquées au Calcofluor White et d'antifongique, en fonction des concentrations en antifongique dans la population de cellules de ladite souche de champignon, par rapport au taux de chitine d'une population de cellules de ladite souche de champignon en absence d'antifongique ;
et dans le cas où ladite variation éventuelle du taux de chitine est une augmentation du taux de chitine supérieure ou égale à 20%, la susdite étape d est suivi d'
**e.** une étape de mesure de la longueur de l'hyphe végétatif de germination dans le mélange de cellules de ladite souche de champignon marquées au Calcofluor White et d'antifongique; puis d'
**f.** une étape de détermination de la variation éventuelle de la longueur de l'hyphe végétatif de germination en fonction de la concentration en antifongique dans la population de cellules de ladite souche de champignon, par rapport à la longueur de l'hyphe végétatif de germination d'une population de cellules de ladite souche de champignon en absence d'antifongique ;
lorsque ladite variation éventuelle du taux de chitine de la susdite étape d. est une augmentation inférieure à 10 % ou une diminution notamment inférieure à 20% ou un taux stationnaire, il est déduit que ladite souche de champignon est de phénotype résistant ;
lorsque ladite variation du taux de chitine de la susdite étape d. est une augmentation de 10 % à une valeur inférieure à 20%, il est déduit que ladite souche de champignon est de phénotype intermédiaire ;
lorsque ladite variation du taux de chitine de la susdite étape d. est une augmentation d'une valeur supérieure ou égale à 20%, et que ladite variation éventuelle de la longueur de l'hyphe végétatif de germination de la susdite étape f. est une diminution de la longueur dudit hyphe ou une longueur stationnaire dudit hyphe, il est déduit que ladite souche de champignon est de phénotype sensible ou intermédiaire.

## Patentansprüche

1. Verwendung der möglichen Veränderung des Chitingehalts in einer Zellpopulation eines Pilzstammes zum Bestimmen des Empfindlichkeitsgrades des Pilzstammes gegenüber einem Antimykotikum, wobei die Veränderung in Bezug auf den Chitingehalt einer Zellpopulation des Pilzstammes in Abwesenheit eines Antimykotikums bestimmt wird, und der möglichen Veränderung der Zellzahl einer Zellpopulation des Pilzstammes, wobei die mögliche Veränderung der Zellzahl in Bezug auf die Zellzahl einer Zellpopulation des Pilzstammes in Abwesenheit eines Antimykotikums bestimmt wird, und wobei der Empfindlichkeitsgrad entweder einem empfindlichen Phänotyp, einem resistenten Phänotyp oder einem intermediären Phänotyp des Pilzstammes gegenüber einem Antimykotikum entspricht, wobei es sich bei dem Pilz um einen einzelligen Pilz handelt:
- wobei der gegenüber dem Antimykotikum resistente Phänotyp des Pilzstammes bestimmt wird durch einen Anstieg des Chitingehalts von weniger als 10 % oder eine Abnahme des Chitingehalts oder einen stationären Chitingehalt in Bezug auf den Chitingehalt einer Zellpopulation des Pilzstammes in Abwesenheit eines Antimykotikums, und wobei der Mindestschwellenwert der Zellen in der Zellpopulation des Pilzstammes, die einen Anstieg des Chitingehalts aufweisen, mindestens 10 % beträgt, insbesondere 20 %, 25 %, 30 %, 35 %, 40 %, 45 %, 50 %, 55 %, 60 %, 65 %, 70 %, 75 %, 80 %, 85 %, 90 %, 95 % und 100 %; oder
- wobei der gegenüber dem Antimykotikum intermediäre Phänotyp des Pilzstammes bestimmt wird
entweder durch einen Anstieg des Chitingehalts von 10 % auf einen Wert von weniger als 20 % in Bezug auf den Chitingehalt einer Zellpopulation des Pilzstammes in Abwesenheit eines Antimykotikums,
oder durch einen Anstieg des Chitingehalts von mehr als oder gleich 20 % in Bezug auf den Chitingehalt einer Zellpopulation des Pilzstammes in Abwesenheit eines Antimykotikums und durch eine Abnahme der Zellzahl um weniger als 0,3 log oder eine stationäre Zellzahl in Bezug auf die Zellzahl einer Zellpopulation des Pilzstammes in Abwesenheit eines Antimykotikums, und wobei der Mindestschwellenwert der Zellen in der Zellpopulation des Pilzstammes, die einen Anstieg des Chitingehalts aufweisen, mindestens 10 % beträgt, insbesondere 20 %, 25 %, 30 %, 35 %, 40 %, 45 %, 50 %, 55 %, 60 %, 65 %, 70 %, 75 %, 80 %, 85 %, 90 %, 95 % und 100 %, und wobei gegebenenfalls der Mindestschwellenwert für die Abnahme der Zellzahl in der Zellpopulation des Pilzstammes mindestens 0,3 log beträgt, insbesondere 0,4; 0,5; 0,6; 0,7; 0,8; 0,9; 1; 1,1; 1,2; 1,3; 1,4; 1,5; 1,6; 1,7; 1,8; 1,9; 2; 2,1; 2,2; 2,3; 2,4; 2,5; 2,6; 2,7; 2,8; 2,9; 3;
oder
- wobei der gegenüber dem Antimykotikum empfindliche Phänotyp des Pilzstammes bestimmt wird durch einen Anstieg des Chitingehalts von mehr oder gleich 20 % in Bezug auf den Chitingehalt einer Zellpopulation des Pilzstammes in Abwesenheit des Antimykotikums und durch eine Abnahme der Zellzahl um mindestens 0,3 log in Bezug auf die Zellzahl einer Zellpopulation des Pilzstammes in Abwesenheit des Antimykotikums, und wobei der Mindestschwellenwert für Zellen in der Zellpopulation des Pilzstammes, die einen Anstieg des Chitingehalts aufweisen, mindestens 10 % beträgt, insbesondere 20 %, 25 %, 30 %, 35 %, 40 %, 45 %, 50 %, 55 %, 60 %, 65 %, 70 %, 75 %, 80 %, 85 %, 90 %, 95 % und 100 %, und wobei der Mindestschwellenwert für die Abnahme der Zellzahl in der Zellpopulation des Pilzstammes mindestens 0,3 log beträgt, insbesondere 0,4; 0,5; 0,6; 0,7; 0,8; 0,9; 1; 1,1; 1,2; 1,3; 1,4; 1,5; 1,6; 1,7; 1,8; 1,9; 2; 2,1; 2,2; 2,3; 2,4; 2,5; 2,6; 2,7; 2,8; 2,9; 3.

2. Verwendung der möglichen Veränderung des Chitingehalts in einer Zellpopulation eines Pilzstammes zum Bestimmen des Empfindlichkeitsgrades des Pilzstammes gegenüber einem Antimykotikum, wobei die Veränderung in Bezug auf den Chitingehalt einer Zellpopulation des Pilzstammes in Abwesenheit eines Antimykotikums bestimmt wird, und der möglichen Veränderung der Länge der vegetativen Keimungshyphe in der Zellpopulation des Pilzstammes, um den Empfindlichkeitsgrad des Pilzstammes gegenüber einem Antimykotikum zu bestimmen, wobei die Veränderung der Länge der vegetativen Keimungshyphe in Bezug auf die Länge der vegetativen Keimungshyphe einer Zellpopulation des Pilzstammes in Abwesenheit eines Antimykotikums bestimmt wird, wobei es sich bei dem Pilz um einen mehrzelligen Pilz handelt und wobei der Empfindlichkeitsgrad entweder einem empfindlichen Phänotyp, einem resistenten Phänotyp oder einem intermediären Phänotyp des Pilzstammes gegenüber einem Antimykotikum entspricht.

3. Verwendung der möglichen Veränderung des Chitingehalts in einer Zellpopulation eines Pilzstammes nach Anspruch 2, wobei es sich bei dem Pilz um einen mehrzelligen Pilz handelt:
- wobei der gegenüber dem Antimykotikum resistente Phänotyp des Pilzstammes bestimmt wird durch einen Anstieg des Chitingehalts von weniger als 10 % oder durch eine Abnahme des Chitingehalts oder durch einen stationären Chitingehalt in Bezug auf mit den Chitingehalt einer Zellpopulation des Pilzstammes in Abwesenheit des Antimykotikums; oder
- wobei der gegenüber einem Antimykotikum intermediäre Phänotyp des Pilzstammes durch einen Anstieg des Chitingehalts von 10 % auf einen Wert von weniger als 20 % in Bezug auf den Chitingehalt einer Zellpopulation des Pilzstammes in Abwesenheit eines Antimykotikums bestimmt wird; oder
- wobei der gegenüber einem Antimykotikum empfindliche oder intermediäre Phänotyp des Pilzstammes durch einen Anstieg des Chitingehalts von mehr als oder gleich 20 % in Bezug auf den Chitingehalt einer Zellpopulation des Pilzstammes in Abwesenheit eines Antimykotikums, und durch die mögliche Veränderung der Länge der vegetativen Keimungshyphe in der Zellpopulation in Bezug auf die Länge der Hyphe in einer Zellpopulation des Pilzstammes in Abwesenheit eines Antimykotikums bestimmt wird, wobei es sich bei der möglichen Veränderung um eine Abnahme der Länge der Hyphe oder eine stationäre Länge der Hyphe handelt in Bezug auf die Länge der vegetativen Keimungshyphe einer Zellpopulation des Pilzstammes in Abwesenheit eines Antimykotikums.

4. Verwendung der möglichen Veränderung des Chitingehalts in einer Zellpopulation eines Pilzstammes zum Bestimmen des Empfindlichkeitsgrades des Pilzstammes gegenüber einem Antimykotikum nach den Ansprüchen 1 bis 3, wobei die mögliche Veränderung des Chitingehalts durch Fluoreszenz bestimmt wird, insbesondere durch ein Verfahren der Fluoreszenzmikroskopie, spezieller durch automatisierte Mikroskopie mit hohem Bildinhalt (HCA), und wobei die mögliche Veränderung des Chitingehalts insbesondere mit Hilfe eines Fluoreszenzmarkers, insbesondere Calcofluor White, bestimmt wird.

5. Verwendung der möglichen Veränderung des Chitingehalts in einer Zellpopulation eines Pilzstammes zur Bestimmung des Empfindlichkeitsgrades des Pilzstammes gegenüber einem Antimykotikum nach den Ansprüchen 1 bis 4, wobei das Antimykotikum aus der Gruppe der Antimykotika ausgewählt ist, die frei von Polyenen wie Amphotericin B und Nystatin und frei von Antimykotika mit direkter Wirkung auf Chitin sind, spezieller:
- aus der Gruppe, die Azol-Antimykotika wie Fluconazol, Voriconazol, Posaconazol, Itraconazol und Isavuconazol oder andere Moleküle, die die Synthese von Ergosterol hemmen, wie Allylamine, darunter Terbinafin oder Morpholine, umfasst oder aus diesen besteht;
- oder aus den Echinocandinen, wobei das Echinocandin insbesondere aus der Gruppe ausgewählt wird, die Anidulafungin, Caspofungin und Micafungin umfasst oder aus diesen besteht.

6. Verwendung der möglichen Veränderung des Chitingehalts in einer Zellpopulation eines Pilzstammes zum Bestimmen des Empfindlichkeitsgrades des Pilzstammes gegenüber einem Antimykotikum nach den Ansprüchen 1, 4-5, wobei es sich bei dem Pilz um eine Hefe handelt, und wobei die Hefezellpopulation aus der Gruppe ausgewählt ist, die die Gattungen *Candida, Cryptococcus, Saccharomyces, Trichosporon, Rhodotorula, Malassezia* umfasst oder aus diesen besteht, und spezieller wobei die Hefe der Gattung *Candida* aus der Gruppe ausgewählt ist, die die den folgenden Arten umfasst oder aus ihnen ausgewählt ist: *Candida albicans, C. glabatra, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

7. Verfahren zur Bestimmung des Empfindlichkeitsgrades einer Zellpopulation eines einzelligen Pilzstammes gegenüber einem Antimykotikum, umfassend einen Schritt zur Bestimmung der möglichen Veränderung des Chitingehalts in der Zellpopulation des Pilzstammes in Gegenwart des Antimykotikums in Bezug auf den Chitingehalt einer Zellpopulation des Pilzstammes in Abwesenheit des Antimykotikums, und einen Schritt zur Bestimmung der möglichen Veränderung der Zellzahl in der Zellpopulation des Pilzstammes in Gegenwart des Antimykotikums, wobei die Veränderung der Zellzahl in Bezug auf die Zellzahl einer Zellpopulation des Pilzstammes in Abwesenheit des Antimykotikums bestimmt wird, wobei der Empfindlichkeitsgrad einem empfindlichen, resistenten oder intermediären Phänotyp entspricht:
- wobei der Empfindlichkeitsgrad einem resistenten Phänotyp entspricht und das Verfahren einen Schritt zur Bestimmung der möglichen Veränderung des Chitingehalts in der Zellpopulation des Pilzstammes in Gegenwart eines Antimykotikums umfasst, wobei es sich bei der möglichen Veränderung um einen Anstieg des Chitingehalts um weniger als 10 % oder eine Abnahme des Chitingehalts oder einen stationären Chitingehalt handelt in Bezug auf den Chitingehalt einer Zellpopulation des Pilzstammes in Abwesenheit eines Antimykotikums, und wobei der Mindestschwellenwert der Zellen in der Zellpopulation des Pilzstammes, die einen Anstieg des Chitingehaltes aufweisen, mindestens 10 % beträgt, insbesondere 20 %, 25 %, 30 %, 35 %, 40 %, 45 %, 50 %, 55 %, 60 %, 65 %, 70 %, 75 %, 80 %, 85 %, 90 %, 95 % und 100 %; oder
- wobei der Empfindlichkeitsgrad einem intermediären Phänotyp entspricht und das Verfahren umfasst
entweder einen Schritt zur Bestimmung der Veränderung des Chitingehalts in der Zellpopulation des Pilzstammes in Gegenwart eines Antimykotikums, wobei es sich bei der Veränderung um einen Anstieg des Chitingehalts von 10 % auf einen Wert von weniger als 20 % handelt in Bezug auf den Chitingehalt einer Zellpopulation des Pilzstammes in Abwesenheit eines Antimykotikums,
oder einen Schritt zur Bestimmung der Veränderung des Chitingehaltes in der Zellpopulation des Pilzstammes in Gegenwart eines Antimykotikums, wobei es sich bei der Veränderung um einen Anstieg des Chitingehaltes von mehr als oder gleich 20 % handelt in Bezug auf den Chitingehalt einer Zellpopulation des Pilzstammes in Abwesenheit des Antimykotikums, und einen Schritt zur Bestimmung der möglichen Veränderung der Zellzahl des Pilzstammes in Gegenwart eines Antimykotikums, wobei es sich bei der Veränderung um eine Abnahme der Zellzahl um weniger als 0,3 log oder eine stationäre Zellzahl im Vergleich zur Zellzahl in einer Zellpopulation des Pilzstammes in Abwesenheit des Antimykotikums handelt, und wobei der Mindestschwellenwert der Zellen in der Zellpopulation des Pilzstammes, die einen Anstieg des Chitingehalts aufweisen, mindestens 10 % beträgt, insbesondere 20 %, 25 %, 30 %, 35 %, 40 %, 45 %, 50 %, 55 %, 60 %, 65 %, 70 %, 75 %, 80 %, 85 %, 90 %, 95 % und 100 %, und wobei gegebenenfalls der Mindestschwellenwert für die Abnahme der Zellzahl in der Zellpopulation des Pilzstammes mindestens 0,3 log beträgt, insbesondere 0,4; 0,5; 0,6; 0,7; 0,8; 0,9; 1; 1,1; 1,2; 1,3; 1,4; 1,5; 1,6; 1,7; 1,8; 1,9; 2; 2,1; 2,2; 2,3; 2,4; 2,5; 2,6; 2,7; 2,8; 2,9; 3; oder
- wobei der Empfindlichkeitsgrad einem empfindlichen Phänotyp entspricht und das Verfahren einen Schritt zur Bestimmung der Veränderung des Chitingehalts in der Zellpopulation des Pilzstammes in Gegenwart eines Antimykotikums umfasst, wobei es sich bei der Veränderung um einen Anstieg des Chitingehalts um mehr als oder gleich 20 % handelt in Bezug auf den Chitingehalt einer Zellpopulation des Pilzstammes in Abwesenheit des Antimykotikums, und einen Schritt zur Bestimmung der Veränderung der Zellzahl in der Zellpopulation des Pilzstammes in Gegenwart des Antimykotikums, wobei es sich bei der Veränderung um eine Abnahme der Zellzahl um mindestens 0,3 log im Vergleich zur Zellzahl einer Zellpopulation des Pilzstammes in Abwesenheit des Antimykotikums handelt, und wobei der Mindestschwellenwert der Zellen in der Zellpopulation des Pilzstammes, die einen Anstieg des Chitingehalts aufweisen, mindestens 10 % beträgt, insbesondere 20 %, 25 %, 30 %, 35 %, 40 %, 45 %, 50 %, 55 %, 60 %, 65 %, 70 %, 75 %, 80 %, 85 %, 90 %, 95 % und 100 %, und wobei der minimale Schwellenwert für die Abnahme der Zellzahl in der Zellpopulation des Pilzstammes mindestens 0,3 log beträgt, insbesondere 0,4; 0,5; 0,6; 0,7; 0,8; 0,9; 1; 1,1; 1,2; 1,3; 1,4; 1,5; 1,6; 1,7; 1,8; 1,9; 2; 2,1; 2,2; 2,3; 2,4; 2,5; 2,6; 2,7; 2,8; 2,9; 3.

8. Verfahren zur Bestimmung des Empfindlichkeitsgrades einer Zellpopulation eines Pilzstammes gegenüber einem Antimykotikum nach Anspruch 7, wobei die mögliche Veränderung des Chitingehalts in der Zellpopulation des Pilzstammes in Gegenwart eines Antimykotikums im Verhältnis zu dem Chitingehalt in einer Zellpopulation des Pilzstammes in Abwesenheit des Antimykotikums durch eine Fluoreszenzmethode, insbesondere durch eine Methode der Fluoreszenzmikroskopie, spezieller durch automatisierte Mikroskopie mit hohem Bildinhalt (HCA) bestimmt wird und insbesondere einen Schritt der Fluoreszenzmarkierung der Zellen des Pilzstammes umfasst, die die Bestimmung der möglichen Veränderung des Chitinanteils ermöglicht, wobei der Schritt der Fluoreszenzmarkierung der Zellen des Pilzstamms insbesondere mit Hilfe von Calcofluor White ausgeführt wird.

9. Verfahren zur Bestimmung des Empfindlichkeitsgrades einer Zellpopulation eines Pilzstammes gegenüber einem Antimykotikum nach den Ansprüchen 7 bis 8, wobei das Antimykotikum aus der Gruppe der Antimykotika ausgewählt ist, die frei von Polyenen wie Amphotericin B und Nystatin und frei von Antimykotika mit direkter Wirkung auf Chitin sind, spezieller:
- aus der Gruppe, die Azol-Antimykotika wie Fluconazol, Voriconazol, Posaconazol, Itraconazol und Isavuconazol oder andere Moleküle, die die Synthese von Ergosterol hemmen, wie Allylamine, darunter Terbinafin oder Morpholine, umfasst oder aus diesen besteht,
- oder aus den Echinocandinen, wobei das Echinocandin spezieller aus der Gruppe ausgewählt ist, die Anidulafungin, Caspofungin und Micafungin umfasst oder aus diesen besteht.

10. Verfahren zur Bestimmung des Empfindlichkeitsgrades einer Zellpopulation eines Pilzstammes gegenüber einem Antimykotikum nach den Ansprüchen 7 bis 9, wobei es sich bei dem einzelligen Pilz um eine Hefe handelt und wobei die Hefezellpopulation aus der Gruppe ausgewählt ist, die die Gattungen *Candida, Cryptococcus, Sacchcironiyces, Trichosporon, Rhodotorula, Malassezia* umfasst oder aus diesen besteht, und wobei spezieller die Hefezellpopulation der Gattung *Candida* ausgewählt ist aus der Gruppe, die die folgenden Arten umfasst oder aus diesen besteht: *Candida albicans, C. glabatra, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

11. Verfahren zur Bestimmung des Empfindlichkeitsgrades einer Zellpopulation eines Pilzstammes gegenüber einem Antimykotikum nach den Ansprüchen 7 bis 10, umfassend einen Schritt des Zusammenbringens des Antimykotikums und der Zellen des Pilzstammes vor dem Schritt zur Bestimmung der möglichen Veränderung des Chitingehalts, wobei der Schritt des Zusammenbringens weniger als oder gleich 48 Stunden beträgt, insbesondere von einer Dauer von 6,5 Stunden bis 24 Stunden ist, und wobei der Schritt des Zusammenbringens bei einer Temperatur von 30 bis 35°C durchgeführt wird.

12. Verfahren zur Bestimmung des Empfindlichkeitsgrades einer Zellpopulation eines Pilzstammes, wobei es sich bei dem Pilz um einen einzelligen Pilz handelt, gegenüber einem Antimykotikum nach den Ansprüchen 7 bis 11, Folgendes umfassend:
a. einen Schritt des Zusammenbringens der Zellen des Pilzstammes mit einem Gradienten von Antimykotikumkonzentrationen, die von 0,0009 bis 130 µg/ml variieren, bei einer Temperatur von 30 bis 35 °C für eine Dauer von weniger als oder gleich 48 Stunden, insbesondere eine Dauer von 6,5 Stunden bis 24 Stunden, um ein Gemisch aus Zellen des Pilzstammes mit dem Antimykotikum zu erhalten;
b. einen Schritt der Zugabe des Fluoreszenzmarkers Calcofluor White zu dem zuvor erhaltenen Gemisch aus den Zellen des Pilzstammes und dem Antimykotikum, um ein Gemisch aus mit Calcofluor White markierten Zellen des Pilzstammes und dem Antimykotikum zu erhalten;
c. einen Schritt der Quantifizierung des Chitingehalts der markierten Zellen des Pilzstammes in dem im vorherigen Schritt erhaltenen Gemisch durch automatisierte Fluoreszenzmikroskopie mit hohem Bildinhalt,
d. einen Schritt zur Bestimmung der möglichen Veränderung des Chitingehalts in dem Gemisch aus den mit Calcofluor White markierten Zellen des Pilzstammes und dem Antimykotikum in Abhängigkeit von den Konzentrationen des Antimykotikums in der Zellpopulation des Pilzstammes in Abhängigkeit von dem Chitingehalt einer Zellpopulation des Pilzstammes in Abwesenheit des Antimykotikums;
und in dem Fall, dass die mögliche Veränderung des Chitingehalts einen Anstieg des Chitingehalts um mehr als oder gleich 20 % ist, folgt auf den oberen Schritt d
e. ein Schritt des Zählens der Zellen in dem Gemisch aus mit Calcofluor White markierten Zellen des Pilzstammes und dem Antimykotikum; und dann
f. ein Schritt zur Bestimmung der möglichen Veränderung der Zellzahl in Abhängigkeit von der Konzentration des Antimykotikums in der Zellpopulation des Pilzstammes in Bezug auf die Zellzahl in einer Zellpopulation des Pilzstammes in Abwesenheit des Antimykotikums;
wenn es sich bei der Veränderung des Chitingehalts in Schritt d. um einen Anstieg des Chitingehalts um weniger als 10 % oder eine Verringerung des Chitingehalts, insbesondere um weniger als 20 % oder einen stationären Wert handelt, wird abgeleitet, dass der Pilzstamm einen resistenten Phänotyp aufweist;
wenn es sich bei der Veränderung des Chitingehalts in Schritt d. um einen Anstieg von 10 % auf einen Wert von weniger als 20 % handelt, wird abgeleitet, dass der Pilzstamm einen intermediären Phänotyp aufweist;
wenn es sich bei der Veränderung des Chitingehalts in Schritt d. um einen Anstieg um einen Wert von mehr als oder gleich 20 % handelt und es sich bei der Veränderung der Zellzahl in Schritt f. um eine Abnahme um weniger als 0,3 log handelt, wird abgeleitet, dass der Pilzstamm einen intermediären Phänotyp aufweist;
wenn es sich bei der Veränderung des Chitingehalts in Schritt d. um einen Anstieg um einen Wert von 20 % oder mehr handelt und es sich bei der Veränderung der Zellzahl um eine Abnahme um mindestens 0,3 log handelt, wird abgeleitet, dass der Pilzstamm einen empfindlichen Phänotyp aufweist.

13. Verfahren zur Bestimmung des Empfindlichkeitsgrades einer Zellpopulation eines Pilzstammes gegenüber einem Antimykotikum, wobei es sich bei dem Pilz um einen mehrzelligen Pilz handelt, umfassend einen Schritt zur Bestimmung der möglichen Veränderung des Chitinanteils in der Zellpopulation des Pilzstammes in Gegenwart des Antimykotikums in Bezug auf den Chitinanteil einer Zellpopulation des Pilzstammes in Abwesenheit des Antimykotikums und einen Schritt zur Bestimmung der möglichen Veränderung der Länge der vegetativen Keimungshyphe in der Population von Pilzzellen in Gegenwart des Antimykotikums, wobei die mögliche Veränderung der Länge der vegetativen Keimungshyphe im Verhältnis zur Länge der vegetativen Keimungshyphe in einer Zellpopulation des Pilzstammes in Abwesenheit des Antimykotikums bestimmt wird.

14. Verfahren zur Bestimmung des Empfindlichkeitsgrades einer Zellpopulation eines Pilzstammes, wobei es sich bei dem Pilz um einen mehrzelligen Pilz handelt, gegenüber einem Antimykotikum nach Anspruch 13, Folgendes umfassend:
a. einen Schritt des Zusammenbringens der Zellen des Pilzstammes mit einem Gradienten von Antimykotikum-Konzentrationen, die von 0,0009 bis 130 µg/ml variieren, für eine Dauer von weniger oder gleich 48 Stunden, insbesondere für eine Dauer von 6,5 Stunden bis 24 Stunden, bei einer Temperatur von 30 bis 35 °C, um ein Gemisch aus Zellen des Pilzstammes mit dem Antimykotikum zu erhalten;
b. einen Schritt der Zugabe des Fluoreszenzmarkers Calcofluor White zu dem zuvor erhaltenen Gemisch aus Zellen des Pilzstammes und dem Antimykotikum, um ein Gemisch aus mit Calcofluor White markierten Zellen des Pilzstammes und dem Antimykotikum zu erhalten;
c. einen Schritt der Quantifizierung des Chitingehalts der markierten Zellen des Pilzstammes in dem im vorherigen Schritt erhaltenen Gemisch durch automatisierte Fluoreszenzmikroskopie mit hohem Bildinhalt;
d. einen Schritt zur Bestimmung der möglichen Veränderung des Chitingehalts in dem Gemisch aus mit Calcofluor White markierten Zellen des Pilzstammes und dem Antimykotikum gemäß der Konzentrationen des Antimykotikums in der Zellpopulation des Pilzstammes im Verhältnis zu dem Chitingehalt einer Zellpopulation des Pilzstammes in Abwesenheit des Antimykotikums;
und für den Fall, dass es sich bei der möglichen Veränderung des Chitingehalts um einen Anstieg des Chitingehalts um 20 % oder mehr handelt, folgt auf den oberen Schritt d
e. ein Schritt zur Messung der Länge der vegetativen Keimungshyphe in dem Gemisch aus mit Calcofluor White markierten Zellen des Pilzstammes und dem Antimykotikum; und dann
f. ein Schritt zur Bestimmung der möglichen Veränderung der Länge der vegetativen Keimungshyphe in Abhängigkeit von der Konzentration des Antimykotikums in der Zellpopulation des Pilzstammes im Vergleich zur Länge der vegetativen Keimungshyphe einer Zellpopulation des Pilzstammes in Abwesenheit des Antimykotikums;
wenn es sich bei der möglichen Veränderung des Chitingehalts in Schritt d. um einen Anstieg von weniger als 10 % oder eine Abnahme insbesondere von weniger als 20 % oder um einen stationären Wert handelt, wird abgeleitet, dass der Pilzstamm einen resistenten Phänotyp aufweist;
wenn es sich bei Veränderung des Chitingehalts in Schritt d. um einen Anstieg um 10 % auf einen Wert von weniger als 20 % handelt, wird abgeleitet, dass der Pilzstamm einen intermediären Phänotyp aufweist,
wenn es sich bei der Veränderung des Chitingehalts in Schritt d. um einen Anstieg um einen Wert von 20 % oder mehr handelt und es sich bei der mögliche Änderung der Länge der vegetativen Keimungshyphe aus dem vorgenannten Schritt f. um eine Abnahme der Länge der Hyphe oder eine stationäre Länge der Hyphe handelt, wird abgeleitet, dass der Pilzstamm einen empfindlichen oder intermediären Phänotyp aufweist.

## Claims

1. A use of the possible variation in the chitin rate in a population of cells of a fungus strain to determine the sensitivity degree of said fungus strain to an antifungal, said variation being determined in relation to the chitin rate of a population of cells of said fungus strain in the absence of antifungal, and of the possible variation in the number of cells of a population of cells of said fungus strain, said possible variation in the number of cells being determined in relation to the number of cells of a population of cells of said fungus strain in the absence of antifungal, and in which the sensitivity degree corresponds either to a sensitive phenotype, or to a resistant phenotype, or to an intermediate phenotype of said fungus strain with respect to an antifungal, said fungus being a unicellular fungus:
- in which the resistant phenotype of said fungus strain with respect to said antifungal is determined by an increase in the chitin rate of less than 10% or a decrease in the chitin rate or a stationary chitin rate, relative to the chitin rate of a population of cells of said fungus strain in the absence of antifungal, and in which the minimum cell threshold in said population of cells of said fungus strain presenting an increase in the chitin rate is at least 10%, in particular 20%, 25%, 30%, 35%, 40%, 45%, 50%, 55%, 60%, 65%, 70%, 75%, 80%, 85%, 90%, 95% and 100%; or
- in which the intermediate phenotype of said fungus strain with respect to said antifungal is determined
either by an increase in the chitin rate of 10% to a value less than 20%, compared to the chitin rate of a population of cells of said fungus strain in the absence of antifungal,
or by an increase in the chitin rate greater than or equal to 20% relative to the chitin rate of a population of cells of said fungus strain in the absence of antifungal, and by a reduction in the number of cells of less than 0.3 log or a stationary number of cells, relative to the number of cells of a population of cells of said fungus strain in the absence of antifungal, and in which the minimum cell threshold in said population of cells of said fungus strain presenting an increase in the chitin rate is at least 10%, in particular 20%, 25%, 30%, 35 %, 40%, 45%, 50%, 55%, 60%, 65%, 70%, 75%, 80%, 85%, 90%, 95% and 100% and possibly in which the minimum threshold for reducing the number of cells in said cell population of said fungus strain is at least 0.3 log, in particular 0.4; 0.5; 0.6; 0.7; 0.8; 0.9; 1; 1.1; 1.2; 1.3; 1.4; 1.5; 1.6; 1.7; 1.8; 1.9; 2; 2.1; 2.2; 2.3; 2.4; 2.5; 2.6; 2.7; 2.8; 2.9; 3;
or
- in which the sensitive phenotype of said fungus strain with respect to said antifungal is determined by an increase in the chitin rate greater than or equal to 20% relative to the chitin rate of a population of cells of said fungus strain in the absence of antifungal, and by a reduction in the number of cells of at least 0.3 log relative to the number of cells of a population of cells of said fungus strain in the absence of antifungal, and in which the minimum cell threshold in said population of cells of said fungus strain presenting an increase in the chitin rate is at least 10%, in particular 20%, 25%, 30%, 35%, 40%, 45%, 50%, 55%, 60%, 65%, 70%, 75%, 80%, 85%, 90%, 95% and 100% and in which the minimum threshold for reducing the number of cells in said population of cells of said fungus strain is at least 0.3 log, in particular 0.4; 0.5; 0.6; 0.7; 0.8; 0.9; 1; 1.1; 1.2; 1.3; 1.4; 1.5; 1.6; 1.7; 1.8; 1.9; 2; 2.1; 2.2; 2.3; 2.4; 2.5; 2.6; 2.7; 2.8; 2.9; 3.

2. The use of the possible variation in the chitin rate in a population of cells of a fungus strain to determine the sensitivity degree of said fungus strain to an antifungal, said variation being determined in relation to the chitin rate of a population of cells of said fungus strain in the absence of antifungal, and of the possible variation in the length of the vegetative germination hypha in said population of cells of said fungus strain, to determine the degree sensitivity of said fungus strain to an antifungal, said variation in the length of the vegetative germination hypha being determined in relation to the length of the vegetative germination hypha of a population of cells of said fungus strain in the absence of antifungal, in which said fungus is a multicellular fungus, and in which the sensitivity degree corresponds either to a sensitive phenotype, or to a resistant phenotype, or to an intermediate phenotype of said fungus strain with respect to an antifungal.

3. The use of the possible variation in the chitin rate in a population of cells of a fungus strain according to claim 2, said fungus being a multicellular fungus:
- in which the resistant phenotype of said fungus strain with respect to said antifungal is determined by an increase in the chitin rate of less than 10% or by a decrease in the chitin rate or by a stationary chitin rate, relative to the chitin rate of a population of cells of said fungus strain in the absence of antifungal; or
- in which the intermediate phenotype of said fungus strain with respect to an antifungal is determined by an increase in the chitin rate from 10% to a value less than 20%, relative to the chitin rate of a population of cells of said fungus strain in the absence of antifungal; or
- in which the sensitive or intermediate phenotype of said fungus strain with respect to an antifungal is determined by an increase in the chitin rate greater than or equal to 20%, relative to the chitin rate of a population of cells of said fungus strain in the absence of antifungal, and by the possible variation of the vegetative germination hypha length in said population of cells relative to the length of said hypha in a population of cells of said fungus strain in the absence of antifungal, said possible variation being a reduction in the length of said hypha or a stationary length of said hypha, relative to the length of the vegetative germination hypha of a population of cells of said fungus strain in the absence of antifungal.

4. The use of the possible variation in the chitin rate in a population of cells of a fungus strain to determine the sensitivity degree of said fungus strain to an antifungal according to claims 1 to 3, wherein the possible variation in the chitin rate is determined by fluorescence, in particular by a fluorescence microscopy method, more particularly by automated microscopy with high content imaging (HCA) and in which the possible variation in the chitin rate is determined in particular using a fluorescent marker, in particular the Calcofluor White.

5. The use of the possible variation in the chitin rate in a population of cells of a fungus strain to determine the sensitivity degree of said fungus strain to an antifungal according to claims 1 to 4, wherein the antifungal is selected from the group of antifungals devoid of polyenes such as amphotericin B and nystatin, and devoid of antifungals with direct action on chitin, more particularly:
- among the group comprising or consisting of azole antifungals such as fluconazole, voriconazole, posaconazole, itraconazole and isavuconazole, or other molecules inhibiting the synthesis of ergosterol such as allylamines including terbinafine or morpholines;
- or among the echinocandins, echinocandin being more particularly selected from the group comprising or consisting of anidulafungin, caspofungin and micafungin.

6. The use of the possible variation in the chitin rate in a population of cells of a fungus strain to determine the sensitivity degree of said fungus strain to an antifungal according to claims 1, 4-5, wherein the fungus is a yeast, and in which the population of yeast cells is selected from the group comprising or consisting of the genera *Candida, Cryptococcus, Saccharomyces, Trichosporon, Rhodotorula, Malassezia,* and more particularly in which the yeast of the genus *Candida* is selected from the group comprising or consisting of the species: *Candida albicans, C. glabrata, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

7. A method for determining the sensitivity degree of a population of cells of a strain of unicellular fungus to an antifungal comprising a step of determining the possible variation in the chitin rate in the population of cells of said fungus strain in the presence of antifungal relative to the chitin rate of a population of cells of said fungus strain in the absence of antifungal, and a step of determining the possible variation in the number of cells in said population of cells of said fungus strain in the presence of antifungal, said variation in the number of cells being determined relative to the number of cells of a population of cells of said fungus strain in the absence of antifungal, in which said sensitivity degree corresponds to a sensitive, resistant or intermediate phenotype:
- in which said sensitivity degree corresponds to a resistant phenotype and said method comprises a step of determining the possible variation in the chitin rate in the population of cells of said fungus strain in the presence of antifungal, said possible variation being an increase in the chitin rate less than 10% or a decrease in the chitin rate or a stationary chitin rate, relative to the chitin rate of a population of cells of said fungus strain in the absence of antifungal, and in which the minimum cell threshold in said population of cells of said fungus strain presenting an increase in the chitin rate is at least 10%, in particular 20%, 25%, 30%, 35%, 40%, 45%, 50%, 55%, 60%, 65%, 70%, 75%, 80%, 85%, 90%, 95% and 100%; or
- in which said sensitivity degree corresponds to an intermediate phenotype and said method comprises
either a step of determining the variation in the chitin rate in the population of cells of said fungus strain in the presence of antifungal, said variation being an increase in the chitin rate of 10% to a value less than 20%, relative to the chitin rate of a population of cells of said fungus strain in the absence of antifungal,
or a step of determining the variation in the chitin rate in the population of cells of said fungus strain in the presence of antifungal, said variation being an increase in the chitin rate greater than or equal to 20%, compared to the chitin rate of a population of cells of said fungus strain in the absence antifungal, and a step of determining the possible variation in the number of cells of said fungus strain in the presence of antifungal, said variation being a decrease in the number of cells of less than 0.3 log or a stationary number of cells, relative to the number of cells in a population of cells of said fungus strain in absence of antifungal, and in which the minimum cell threshold in said cell population of said fungus strain presenting an increase in chitin rate is at least 10%, in particular 20%, 25%, 30%, 35%, 40%, 45%, 50%, 55%, 60%, 65%, 70 %, 75%, 80%, 85%, 90%, 95% and 100% and optionally in which the minimum threshold for reducing the number of cells in said population of cells of said fungus strain is at least 0.3 log, in particular 0.4; 0.5; 0.6; 0.7; 0.8; 0.9; 1; 1.1; 1.2; 1.3; 1.4; 1.5; 1.6; 1.7; 1.8; 1.9; 2; 2.1; 2.2; 2.3; 2.4; 2.5; 2.6; 2.7; 2.8; 2.9; 3; or
- in which said sensitivity degree corresponds to a sensitive phenotype and said method comprises a step of determining the variation in the chitin rate in the population of cells of said fungus strain in the presence of antifungal, said variation being an increase in the chitin rate greater than or equal to 20%, relative to the chitin rate of a population of cells of said fungus strain in the absence of antifungal and a step of determining the variation in the number of cells in said population of cells of said fungus strain in the presence of antifungal, said variation being a reduction in the number of cells of at least 0.3 log relative to the number of cells of a population of cells of said fungus strain in the absence of antifungal, and in which the minimum cell threshold in said population of cells of said fungus strain presenting an increase in the chitin rate is at least 10%, in particular 20%, 25%, 30%, 35%, 40%, 45%, 50%, 55%, 60%, 65%, 70%, 75%, 80%, 85%, 90%, 95% and 100% and in which the minimum threshold for reducing the number of cells in said population of cells of said fungus strain is at least 0.3 log, in particular 0.4; 0.5; 0.6; 0.7; 0.8; 0.9; 1; 1.1; 1.2; 1.3; 1.4; 1.5; 1.6; 1.7; 1.8; 1.9; 2; 2.1; 2.2; 2.3; 2.4; 2.5; 2.6; 2.7; 2.8; 2.9; 3.

8. The method for determining the sensitivity degree of a population of cells of a fungus strain to an antifungal according to claim 7 wherein the possible variation in the chitin rate in the population of cells of said fungus strain in the presence of antifungal relative to the chitin rate of a population of cells of said fungus strain in the absence of antifungal is determined by fluorescence method, in particular by a fluorescence microscopy method, more particularly by automated microscopy with high content imaging (HCA) and in particular comprising a step of fluorescent marking of the cells of said fungus strain allowing the determination of the possible variation in the chitin rate, said step of fluorescent marking the cells of said fungus strain being carried out in particular using Calcofluor White.

9. The method for determining the sensitivity degree of a population of cells of a fungus strain to an antifungal according to claims 7 to 8, wherein said antifungal is selected from the group of antifungals devoid of polyenes such as amphotericin B and nystatin, and devoid of antifungals with direct action on chitin, more particularly:
- among the group comprising or consisting of azole antifungals such as fluconazole, voriconazole, posaconazole, itraconazole and isavuconazole, or other molecules inhibiting the synthesis of ergosterol such as allylamines including terbinafine or morpholines;
- or among the echinocandins, echinocandin being more particularly selected from the group comprising or consisting of anidulafungin, caspofungin and micafungin.

10. The method for determining the sensitivity degree of a population of cells of a fungus strain to an antifungal according to claims 7 to 9, wherein said unicellular fungus is a yeast, and in which the population of yeast cells is selected from the group comprising or consisting of the genera *Candida, Cryptococcus, Saccharomyces, Trichosporon, Rhodotorula, Malassezia,* and more particularly in which the population of yeast cells of the genus *Candida* is selected from the group comprising or consisting of the species: *Candida albicans, C. glabrata, C. tropicalis, C. parapsilosis, C. krusei, C. dubliniensis, C. kefyr, C. lusitaniae, C. zeylanoides, C. rugosa, C. inconspicua, C. norvegensis, C. guilliermondii, C. utilis.*

11. The method for determining the sensitivity degree of a population of cells of a fungus strain to an antifungal according to claims 7 to 10 comprising a step of introducing said antifungal and the cells of said fungus strain, before the step of determining the possible variation in the chitin rate, said introducing step is less than or equal to 48 hours, in particular lasting from 6.5 hours to 24 hours, and in which said introducing step is carried out at a temperature of 30 to 35°C.

12. The method for determining the sensitivity degree of a population of cells of a fungus strain, said fungus being a unicellular fungus, with respect to an antifungal according to claims 7 to 11, comprising:
a. a step of introducing the cells of said fungus strain into contact with an antifungal concentration gradient varying from 0.0009 to 130 µg/mil, at a temperature of 30 to 35°C for a period less than or equal to 48 hours, in particular a period of 6.5 hours to 24 hours, to obtain a mixture of cells of said fungus strain with the antifungal;
b. a step of adding the fluorescent marker Calcofluor White to the mixture of cells of said fungus strain and antifungal obtained previously, to obtain a mixture of cells of said fungus strain marked with Calcofluor White and antifungal;
c. a step of quantifying the chitin rate by automated fluorescence microscopy with high content imaging, of the cells of said fungus strain marked in the mixture obtained in the previous step;
d. a step of determining the possible variation in the chitin rate in the mixture of cells of said fungus strain marked with Calcofluor White and antifungal, as a function of the antifungal concentrations in the population of cells of said fungus strain, relative to the chitin rate of a population of cells of said fungus strain in the absence of antifungal;
and in the case where said possible variation in the chitin rate is an increase in the chitin rate greater than or equal to 20%, the above-mentioned step d is followed by
e. a step of counting the cells in the mixture of cells of said fungus strain marked with Calcofluor White and antifungal; then by
f. a step of determining the possible variation in the number of cells as a function of the concentration of antifungal in the population of cells of said fungus strain, relative to the number of cells in a population of cells of said fungus strain in the absence of antifungal;
when said variation in the chitin rate of the above-mentioned step d. is an increase in the chitin rate of less than 10% or a decrease in the chitin rate in particular less than 20% or a stationary rate; it is deduced that said fungus strain is of a resistant phenotype;
when said variation in the chitin rate of the above-mentioned step d. is an increase from 10% to a value less than 20%, it is deduced that said fungus strain is of an intermediate phenotype;
when said variation in the chitin rate of the above-mentioned step d. is an increase of a value greater than or equal to 20%, and that said variation in the number of cells of the above-mentioned step f. is a decrease of less than 0.3 log, it is deduced that said fungus strain is of an intermediate phenotype;
when said variation in the chitin rate of the above-mentioned step d. is an increase of a value greater than or equal to 20%, and that said variation in the number of cells is a decrease of at least 0.3 log, it is deduced that said fungus strain is of a sensitive phenotype.

13. The method for determining the sensitivity degree of a population of cells of a fungus strain to an antifungal, in which said fungus is a multicellular fungus, comprising a step of determining the possible variation in the chitin rate in the population of cells of said fungus strain in the presence of said antifungal compared to the chitin rate in a population of cells of said fungus strain in the absence of antifungal, and a step of determining the possible variation in the length of the vegetative germination hypha in said population of fungus cells in the presence of said antifungal, said possible variation in the length of the vegetative germination hypha being determined in relation to the length of the vegetative germination hypha in a population of cells of said fungus strain in the absence of antifungal.

14. The method for determining the sensitivity degree of a population of cells of a fungus strain, said fungus being a multicellular fungus, to an antifungal according to claim 13, comprising:
a. a step of introducing the cells of said fungus strain with an antifungal concentration gradient varying from 0.0009 to 130 µg/ml, for a period of less than or equal to 48 hours, in particular for a period of 6.5 hours to 24 hours, at a temperature of 30 to 35°C, to obtain a mixture of cells of said fungus strain with the antifungal:
b. a step of adding the fluorescent marker Calcofluor White to the mixture of cells of said fungus strain and antifungal obtained previously, to obtain a mixture of cells of said fungus strain marked with Calcofluor White and antifungal;
c. a step of quantifying the chitin rate by automated fluorescence microscopy with high content imaging, of the cells of said fungus strain marked in the mixture obtained in the previous step;
d. a step of determining the possible variation in the chitin rate in the mixture of cells of said fungus strain marked with Calcofluor White and antifungal, as a function of the concentrations of antifungal in the population of cells of said fungus strain, relative to the chitin rate of a population of cells of said fungus strain in the absence of antifungal;
and in the case where said possible variation in the chitin rate is an increase in the chitin rate greater than or equal to 20%, the above-mentioned step d is followed by
e. a step of measuring the length of the vegetative germination hypha in the mixture of cells of said fungus strain marked with Calcofluor White and antifungal; then by
f. a step of determining the possible variation in the length of the vegetative germination hypha as a function of the concentration of antifungal in the population of cells of said fungus strain, relative to the length of the vegetative germination hypha of a population of cells of said fungus strain in the absence of antifungal;
when said possible variation in the chitin rate of the above-mentioned step d. is an increase of less than 10% or a decrease in particular less than 20% or a stationary rate, it is deduced that said fungus strain is of a resistant phenotype;
when said variation in the chitin rate of the above-mentioned step d. is an increase from 10% to a value less than 20%, it is deduced that said fungus strain is of an intermediate phenotype;
when said variation in the chitin rate of the above-mentioned step d. is an increase of a value greater than or equal to 20%, and that said possible variation in the length of the vegetative germination hypha of the above-mentioned step f. is a reduction in the length of said hypha or a stationary length of said hypha, it is deduced that said fungus strain is of a sensitive or an intermediate phenotype.
